# EUROPEAN PATENT APPLICATION

(11) **EP 2 752 487 A1**
(43) Date of publication of application: **09.07.2014**
(21) Application number: 13305003.9
(22) Date of filing: 03.01.2013
(51) Int. Cl.: C12N 15/10, G01N 33/68, G01N 33/50

(54) **Intracellular phenotypic screening**

(71) Applicant: SANOFI, 75008 Paris (FR); INSTITUT NATIONAL DE LA SANTE ET DE LA RECHERCHE MEDICALE, 75654 Paris Cedex 13 (FR); Université de Montpellier I, 34006 Montpellier Cedex 1 (FR); Université Montpellier 2 Sciences et Techniques, 34095 Montpellier Cedex 5 (FR); Centre Val D'Aurelle - Paul Lamarque, 34298 Montpellier Cedex 5 (FR)
(72) Inventor: Dariavach, Piona, 34090 Montpellier (FR); Hahn, Chang, Chestnut Hill, MA 02467 (US); Martineau, Pierre, 34980 Saint Gely Du Fesc (FR)
(74) Representative: Almond-Martin, Carol

(57) **Abstract**

The present invention relates to a method for identifying a cellular target involved in a cell phenotype comprising identifying an intrabody which can modify a cell phenotype and identifying a direct or indirect cellular target of the intrabody. The present invention also relates to intrabodies 3H2-1, 3H2-VH and 5H4 which are capable of inhibiting the degranulation reaction in mast cells triggered by an allergic stimulus, and especially to intrabodies 3H2-1 and 5H4 which are capable of directly or indirectly targeting a protein of the ABCF1 family and of the C12ORF4 family respectively. The present invention also relates to ABCF1 and C12ORF4 inhibitors for use in therapy, in particular for treating allergic and/or inflammatory conditions.

## Description

The present invention relates to a method for identifying a cellular target involved in a cell phenotype comprising identifying an intrabody which can modify a cell phenotype and identifying a direct or indirect cellular target of the intrabody. The present invention also relates to intrabodies which are capable of inhibiting the degranulation reaction triggered by an allergic stimulus on mast cells, particularly to intrabodies 3H2-1, 3H2-VH and 5H4, and especially to intrabodies 3H2-1 and 5H4 which are capable of directly or indirectly targeting a protein of the ABCF1 family and of the C12ORF4 family respectively. The present invention also relates to ABCF1 and C12ORF4 inhibitors for use in therapy, in particular for treating allergic and/or inflammatory conditions.

Identifying cellular targets associated with a given phenotype is an essential prerequisite to a better understanding of cellular mechanisms underlying that phenotype. In particular, identifying cellular targets associated with a medical condition is of great interest for the pharmaceutical industry as it allows the design of new therapeutics which have an effect on these targets and can be used to treat or diagnose the medical condition. Frequently, a cell phenotype may be associated with a given pathology. An assay which would allow the identification of molecules that modify a cell phenotype (phenotypic screening) could thus be of great help to drug design. The identification of new cellular targets which may be associated with a given phenotype is also important to other sectors, for example in cosmetics or in the plant and food industry.

Understanding the role played by a known or unknown cellular molecule in a given phenotype is not an easy task in particular because cellular pathways are highly complex and because many intracellular molecules play a part in a number of different cellular pathways. Thus inactivating a target protein, for example by RNA interference, can have a modulatory effect on several cellular pathways and generate as a result several concomitant phenotypic effects which may be difficult to distinguish from one another. Moreover, many cell proteins can assume different conformations and/or may comprise post-translational modifications such as phosphorylated amino acids, and as a consequence may interact or not with another molecule as a function of their conformation or of post-translational modifications. A technique which would allow screening for cellular targets responsible for a given phenotype, and even for targets in a specific conformation and/or modified or non-modified state, without completely inactivating these cellular targets would thus be a highly valuable tool towards a better understanding of a number of cellular pathways.

Antibodies can be seen as precision tools as they are highly specific for their target and can be raised against virtually any part of a protein and in particular against large planar zones where protein interactions often take place and which are more difficult to target with small organic molecules or peptides. However, natural antibodies are not adapted to the intracellular environment because of their large size and because the reducing environment of the cytoplasm prevents the formation of disulfur bridges and thus prevents proper folding of the antibodies. Recombinant antibodies, among which scFv, diabodies and sdAb, have been developed for expression in an intracellular environment. They are referred to as "Intracellular antibodies" or "intrabodies".

An application recently described by the inventors' team, is the use of intrabodies preselected for their modulating effect of a target antibody (or "Ab") to screen a library of small molecules (European Patent EP1743178B1). The goal is to isolate drug candidates capable of mimicking the intracellular effects of the scFv of interest and that could be used in vivo. The inventors applied this ELISA displacement of Ab in a model of allergy. Previous work of the same team had led to the isolation of a scFv directed against the SH2 domains of tyrosine kinase Syk, involved in the early stages of mast cell activation. It had been shown that intracellular expression of this scFv in a mast cell line inhibits the release of allergic mediators induced by activation of IgE receptors, without disrupting the kinase activity of Syk (Dauvillier et al., 2002). An Ab displacement assay was developed to isolate molecular mimics of the anti-Syk scFv. In that manner, the screening of a chemical library of 3000 molecules led to the identification of a molecule, designated C-13. This molecule has a high anti-allergic potential because further studies demonstrated its ability to inhibit mast cell activation *in vitro* as well as anaphylactic shock *in vivo* in mice (Mazuc et al., 2008). The site of interaction of C-13 with Syk was also identified by a structural analysis and directed mutagenesis. The identified cavity at the interface between the two SH2 domains of Syk was used for a second screen *in silico* of a library of 500,000 molecules. In this manner, 85 non-enzymatic inhibitors of Syk were selected for their ability to inhibit the release of allergic mediators (Villoutreix et al., 2011).

Inoue et al., 2011 describes a loss-of-function screening using a library of randomized intracellular camelid VHH. The authors consider that conventional V_{H} domains are insoluble in the intracellular environment. In the corresponding Japanese patent application JP2008-136455, the authors of Inoue et al., also consider scFvs as useless in the context of intracellular loss-of-function screening, because of a poor intracellular stability and functionality.

The inventors have developed a novel phenotypic screening technique based on the use of intracellular antibodies (or "intrabodies"), for the identification of cellular targets which can act on a cellular pathway and modulate a phenotype of interest. The present work describes the first phenotypic selection using intracellular human antibody fragments in mammalian cells.

The novel method developed by the inventors allows the identification of intracellular targets associated with a given phenotype, and in particular unknown intracellular targets or known targets not previously associated with a given phenotype. The inventors used this technique to identify two RBL-2H3 cellular clones named 3H2 and 5H4 which expressed respectively antibody fragments 3H2-1 and 3H2-VH, and 5H4. When expressed in mast cells, these antibody fragments inhibit the degranulation reaction which is normally triggered by an allergic challenge. The inventors have also identified two proteins, ABCF1 and LOC297607, which can be precipitated with 3H2-1 and 5H4 respectively. The inventors have demonstrated the involvement of LOC297607, for which no known function has been described in the art, in mast cell degranulation.

The choice of intracellular Ab for the realization of a phenotypic screening at the scale of the proteome, offers many advantages. Indeed, when expressed in cells, they are able to interfere with protein functions and thereby generate a given phenotype. Moreover, with their high affinity and specificity properties, they are potentially capable of targeting any molecule in a cell, and in particular proteins, and more precisely certain sub-domains or post-transcriptional modifications which may be involved in a particular signalling. In the context of disease models, another advantage offered by the intracellular antibodies, is that once their target is identified, they can be used to guide drug development.

More specifically, the use of a combinatory scFv library of high diversity and optimised for intracellular expression has allowed the inventors to perform a large scale phenotypic screen. Applying this screen to the model of mast cell activation, they have identified a new molecular player involved in this signalling pathway.

This approach was initially developed with a plasmid system allowing expression of the scFv library in a mast cell line. In order to retain as much as possible the initial diversity of the library, transfection conditions imposing a multicopy expression mode have been selected. Phenotypic selection has been performed and has led to enrichment in cells presenting the phenotype of interest, and expressing the scFv which inhibit the studied signalling pathway. The expression of two particular antibody (Ab) fragments, 3H2-1 and 5H4, in the mast cell line RBL-2H3, strongly inhibited the ability of cells to release allergic mediators. After having produced them, these antibodies have been used *in vitro* to identify their protein targets by immunoprecipitation experiments coupled to mass spectrometry. Proteins ABCF1 and LOC297607 have thereby been identified as the targets of 3H2-1 and 5H4 respectively.

LOC297607 (or "LOC") is a protein of previously unknown function which seems to be involved in mast cell activation via FcεRI. Indeed, preliminary results with shRNA experiments suggest it intervenes in regulation both of early events following receptor activation and of later events of allergic mediators' release. It could be interesting to identify more precisely the molecular partners of the protein LOC in the mast cells, by evaluating amongst other things the activity of proteins which intervene in transduction of the signal mediated by the FcεRI.

In addition, several genomic data suggest a link between protein LOC and inflammation. A study using the technique of chromosome mapping by hybrid radiation in pigs showed that the chromosomal locus of LOC may be associated with inflammation, and more specifically with a particular form of arthritis (Genini et al. 2006). On analyzing this chromosome locus in Genebank, the inventors found that the LOC gene was colocalized with the gene encoding the protein tyrosine phosphatase PTPN6. Furthermore, this chromosomal co-location is conserved between rat, mouse and man. This information suggests that these proteins may be involved in the same signalling network. Indeed, new approaches for identifying protein-protein interactions are based on the comparison, between different species, of the chromosomal distances between the sequences of associated proteins (Pazos and Valencia 2001).

To maximize this phenotypic screening approach and expand its scope, the inventors have adapted it using a retroviral system for the intracellular expression of the scFv library. Compared with the previous plasmid system, this mode of expression allows the transfection of a wider repertoire of cell types, and provides stable expression which allows the study of phenotypes over a longer term.

During the plasmid selection, 2000-2500 copies of scFv were expressed per cell, which allowed the exploration of a wide diversity of scFv, while handling a relatively small number of cells. However, the fact that so many scFv were expressed per cell, imposed a strong competition between each of them in terms of intracellular effects, and thus probably maintained a high background noise. In this regard, the expression of the retroviral library that did not exceed more than 3 copies per cell, had the advantage of lowering the selection pressure. These conditions also allow the screen to be carried out in more physiological conditions, where the integrity of the protein network is not disrupted by overexpression.

High throughput sequencing analyses revealed that the inclusion of recloning steps every two rounds, during the retroviral selection allowed a better enrichment of scFv. Cell clones from the same selection and analysed independently showed a significant inhibition of β-hexosaminidase degranulation, which suggests that this selection effectively allowed an enrichment in inhibitory scFv.

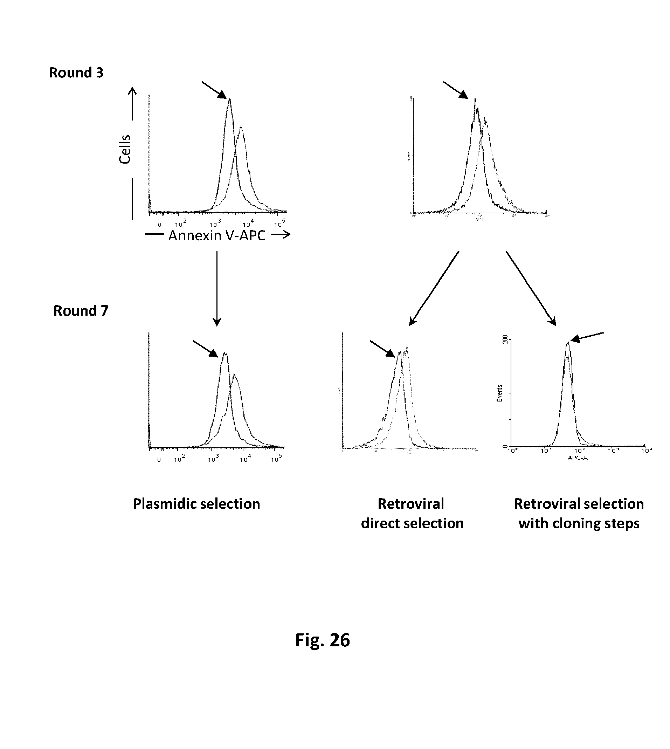

Finally, the main data that enables the comparison of the three selections to each other, is the evolution of the Annexin V marking phenotype after cell stimulation (Figure 26). The inventors found that the selection including the steps of retroviral recloning allowed convergence towards the most pronounced inhibitory phenotype as it was virtually abolished (92% inhibition). One hypothesis would be that with recloning, the selected cell population would be enriched in clones having both a good proliferation and an inhibitory phenotype. This hypothesis could explain the difference in convergence towards the inhibitory phenotype between the two retroviral selections.

The present inventors have proven that a scFv-based library could successfully be used in a phenotypic intracellular screening. By using a high diversity library of scFvs, based on a constant framework having intracellular stability, the inventors have succeeded in finding and identifying unknown intracellular targets to a given phenotype, for example mast cell degranulation.

The inventors have also discovered that truncated scFvs, including intrabodies 3H2-VH, 5H4-VH could be stable and functional in the intracellular environment. In particular, intrabody 5H4-VH, restricted to a full V_{H} domain was found stable in said intracellular environment, and successfully inhibited mast cell degranulation.

A first aspect of the present invention is a method for identifying a cellular target which is involved in a cell phenotype, comprising:
a) identifying an intrabody which can induce, modify or suppress said phenotype when present inside a cell;
b) identifying a cellular target which is a direct or indirect target of said intrabody in said cell; and optionally
c) isolating said cellular target.

Preferably, the intrabody comprises a full V_{H} and/or V_{L} domain of an immunoglobulin.

Antibodies are recognition proteins which are capable of binding specifically to a unique epitope on an antigen. The antigen binding site of an antibody is referred to as the paratope. Antibodies belong to the Immunoglobulin (Ig) class of proteins. Immunoglobulins are a family of proteins which have in common a specific structural domain referred to as the "Ig fold". Most naturally occurring antibodies (or conventional antibodies) are globular proteins of about 150 kDa, which comprise two light chains (L) and two heavy chains (H) joined together by disulfide bonds. Nonconventional antibodies characterized by the absence of light chains are found in camelids and in non-cartilaginous fishes (e.g. sharks). Light and Heavy chains comprise a constant region (C_{L} and C_{H} respectively) and a variable region (V_{L} and V_{H} respectively). In mammals, there are five types of Ig heavy chains (α, δ, ε, γ, µ), which have a length of about 450 to 550 amino acids, and two types of Ig light chains (κ ,λ), which have a length of about 210 to 220 amino acids. An Ig chain is composed of structural domains referred to as "Ig domains". α, δ, and γ heavy chains are composed of one variable domain, three constant domains and a hinge region which increases flexibility. ε and µ heavy chains are composed of one variable domain and four constant domains. κ and λ light chains are composed of one variable domain and one constant domain. Ig domains generally have a length of about 110 amino acids. Each variable domain comprises hypervariable regions or loops which are responsible for epitope binding and which are referred to as complementary determining regions (CDR), and less variable regions between the CDRs referred to as framework regions (FR). Full mammalian V_{H} and V_{L} domains comprise three CDRs, referred to as CDR1, CDR2 and CDR3 and four framework regions, referred to as FR1, FR2, FR3 and FR4. By contrast, a truncated V_{H} and V_{L} domain lacks at least one of the CDRs or at least one of the framework regions, for example via the occurrence of a stop codon within the nucleotide sequence encoding said truncated V_{H} and V_{L} domain. Similarly, a truncated scFv lacks at least one of the CDRs or at least one of the framework regions of the V_{H} and/or V_{L} domain. A truncated scFv can consist in a single full V_{H} or V_{L} domain. The heavy chain CDR3 is the main contributor to the interaction with the antigen. All Ig domains have a characteristic compact globular structure, referred to as the "Ig-Fold". The structure of the Ig-fold is well known to the skilled person. Briefly, it consists of a two-layer sandwich of 7 to 9 antiparallel β-strands arranged in two β-sheets with a Greek key topology. The backbone switches repeatedly between the two β-sheets. Typically, the pattern is (N-terminal β-hairpin in sheet 1)-(β-hairpin in sheet 2)-(β-strand in sheet 1)-(C-terminal β-hairpin in sheet 2). The two β-sheets are also connected to each other by a disulfide bridge. The CDR regions form loops which are held on the outside of the sandwich structure.

A conventional antibody digested by papain yields three fragments: two Fab fragments (fragment antigen binding) and one Fc fragment (Fragment crystalizable). A Fab fragment is a region of an antibody that binds to antigens. It is about 50 kDa and is composed of one constant and one variable domain of each of the heavy and the light chain. The two variable domains (V_{L} and V_{H}) shape the antigen binding site (paratope). The Fc region is the tail region of an antibody which interacts with cell surface receptors (Fc receptors). A conventional antibody digested by pepsin yields two fragments: a F(ab')₂ fragment (fragment antigen binding) and a pFc' fragment. The F(ab')₂ fragment comprises two antigen binding sites and can be split in two Fab' fragments by mild reduction. The variable regions of a heavy and a light chain can be fused together to form a single chain variable fragment (scFv), which retains the specificity of a Fab fragment while being only half its size (about 25 kDa). The V_{L} and V_{H} domains of an scFv are generally connected to each other by a short peptide linker of about 15 amino acids. The linker is usually rich in glycine for flexibility, as well as serine or threonine for solubility. Bi-valent or trivalent scFv can be engineered by connecting two or three scFvs together. Bi-valent scFvs (a form of diabodies) are of interest because they have a very high affinity for their target. Single-domain antibodies (sdAb) are antibodies of about 12-15 kDa which are composed of a single variable domain (a V_{L} or V_{H} domain). sdAbs can for example be composed of a camelid V_{HH} domain, of a V_{H} or a V_{L} domain of a conventional antibody, or of a recombinant V_{H} or a V_{L} domain. One advantage of scFvs, diabodies and sdAbs is that they can be expressed as a single peptide and can for example be produced in bacteria and displayed on phages.

According to the present invention, an antibody (or "Ab") is a protein which comprises at least one V_{L} or V_{H} domain of an immunoglobulin.

According to the present invention, an intrabody (or "intracellular antibody") is an antibody, for example an scFv, a diabody, an sdAb, which is suitable for use in an intracellular environment. In particular, the antibody is able to fold in the reducing conditions of the cell cytosol and/or nucleus and is stable in an intracellular environment. The intrabody can in particular comprise a full V_{H} domain, a full V_{L} domain, or both. The intrabody can in particular be an scFv, a truncated scFv comprising at least a full V_{H} or V_{L} domain, a diabody or a V_{H} or V_{L} domain. Preferably, the intrabody is an scFv or a truncated scFv comprising at least a full V_{L} or V_{H} domain, most preferably a full V_{H} domain.

According to the present invention and in accordance with the common meaning of the term (Muyldermans, 2001) a V_{H} or V_{L} domain refers to a conventional V_{H} or V_{L} domain, namely to a V_{H} or V_{L} domain of an antibody which is not a camelid antibody. In other terms, the term "V_{H} domain" excludes V_{HH} fragments, especially camelid V_{HH} fragments. Thus, in a specific embodiment of the present invention, the V_{H} domain and, preferably the V_{L} domain is a non-camelid antibody, or is derived from a non-camelid antibody, preferably is a mammalian non-camelid antibody or is derived from a mammalian non-camelid antibody. In another embodiment, the V_{H} and, preferably, the V_{L} domain is not a non-cartilagenous fish antibody or is not derived from a non-cartilagenous fish antibody.

The distinction between conventional V_{H} or V_{L} domains and camelid V_{HH} finds a basis in a number of sequence and structural differences (Muyldermans, 2001). For example, CDR1 and CDR2 loops of camelid V_{HH} adopt structures that fall outside the canonical structures described for V_{H} or V_{L} domains of conventional antibodies. Moreover, V_{H} domains comprise 4 canonical hydrophobic amino acids in their FR2 region, which take part in the interface with the V_{L} domain. These amino acids are mutated in VHHs. These mutations, Val37Phe (or Tyr), Gly44Glu (or Gln), Leu45Arg (or Cys), and Trp47Gly (or Ser, Leu, or Phe), (Kabat numbering, reference in Kabat et al., 1991 and in Kontermann and Dübel, 2010) are highly conserved within the camel antibodies and are a key feature distinguishing them from a conventional V_{H} domain. The CDR3 loop of V_{HH} antibodies is also longer (16 to 18 amino acids, commonly 17 for the camel V_{HH}) than the CDR3 loop of conventional antibodies (12 amino acids for human antibodies, 9 amino acids for mouse antibodies).
VHHs have been recognized as having an ability to bind recessed antigenic sites, which has been attributed to their smaller size and the ability of the extended CDR3 loop to penetrate into such sites. However, the single-domain nature of VHHs can be a disadvantage for binding to small antigens because these can bind in a groove or cavity at the V_{H}-V_{L} interface.

In a particular embodiment, a V_{H} domain according to the invention comprises one or more, preferably all of the amino acids V37, G44, L45 and W47, with reference to the Kabat numbering scheme.

Alternatively, a V_{H} domain according to the invention comprises the motif VNNNNNNGLNW, preferably in the FR2 region, wherein each N is an amino acid, independently selected from each other, preferably selected among the 20 canonical amino acids of the genetic code.

In addition, or alternatively, a V_{H} domain according to the invention can also comprise a variant of the motif VNNNNNNGLNW, preferably in the FR2 region, wherein each N is an amino acid, independently selected from each other, preferably selected among the 20 canonical amino acids of the genetic code, and wherein the variant comprises one or more, such as one to two or one to three, amino acid additions and/or one or more, such as one to two or one to three amino acid deletions of any "N" amino acid.

The CDR3 loop of the V_{H} domain and/or V_{L} domain preferably comprises from 1 to 15 amino acids, still preferably from 1 to 12 amino acids.

The use of a high diversity library of intrabodies, containing not only full scFvs but also truncated scFvs, gives access to a high diversity of epitopes. While full scFvs preferably interact with the surface of a target protein or with small antigens, truncated scFvs, such as sdAbs, because of their smaller size, are more adapted to interact with protein cavities. Full scFvs are thus more adapted to disrupt protein-protein interactions, whereas truncated scFvs, such as sdAbs are more adapted to interact with enzymatic sites or with small molecule binding sites on their target antigen. The presence of both types of intrabodies is therefore highly advantageous in a library used for screening an unknown target.

In a specific embodiment, the intrabody comprises a V_{L} and/or V_{H} domain derived from a human antibody. Still preferably, the intrabody is a scFv derived from a human antibody, a truncated scFv comprising at least a full V_{L} or V_{H} domain derived from a human antibody, a diabody derived from a human antibody or a V_{L} or a V_{H} domain derived from a human antibody. More generally, the intrabody can be derived from a human antibody.

As meant herein, a "V_{L} or V_{H} domain derived from a human antibody" signifies a V_{L} or V_{H} domain which is identical or essentially identical to a V_{L} or V_{H} domain of a human immunoglobulin. By "essentially identical", it is meant that the V_{L} or V_{H} domain can consist in a V_{L} or V_{H} domain of a human immunoglobulin in which modifications have been introduced into one or more CDR regions, preferably into the CDR3 region. The modifications can also be introduced into the framework regions, provided that they do not adversely affect the intracellular folding and/or the intracellular stability of the V_{L} or V_{H} domain.

The modifications can consist of point mutations, additions and/or deletions of one or more amino acids, for example of one to twenty amino acids, especially of one to fifteen amino acids, more particularly of one to twelve amino acids. When the modifications are introduced into one CDR region, from one to all amino acids of the CDR can be modified. Preferably, the modifications are introduced so as to have the same representation of the amino acids as that observed in natural human CDRs or framework regions corresponding to the modified CDR or framework region. The modifications are also introduced so that the "V_{L} or V_{H} domain derived from a human antibody" is still recognized as human when using the below-mentioned test for assessing the species of origin of an antibody. In other terms, when the sequence of a "V_{L} or V_{H} domain derived from a human antibody" is aligned with a library of immunoglobulins, or fragments thereof, of various species, including homo sapiens, the "best hit" corresponds to a human immunoglobulin or a fragment thereof.

As meant herein, a scFv derived from a human antibody comprises a V_{L} domain derived from a human antibody and a V_{H} domain derived from a human antibody. Similarly, a diabody derived from a human antibody comprises two sdAbs derived from a human antibody.

Mutatis mutandi, the term "derived from an antibody of a specific species or group of species" (for example "derived from a non-camelid antibody") should be understood in a similar way as "derived from a human antibody".

In another embodiment, the intrabody comprises a humanized V_{L} and/or V_{H} domain. Still preferably, the intrabody is a humanized scFv, a truncated scFv comprising at least a full humanized V_{L} or V_{H} domain, a humanized diabody or a humanized V_{L} or a V_{H} domain. More generally, the intrabody can be a humanized intrabody.

By "humanized" intrabody, it is meant that the intrabody has been obtained by engineering a non-human antibody so that it is less immunogenic for humans. Preferably, the intrabody has been obtained by "CDR grafting", i.e. it comprises human framework regions, and one or more CDR regions originating from a non-human species, preferably a non-camelid non-human species. The framework can be a "fixed framework", i.e. the same framework for all humanized antibodies, or can be chosen using the "best fit" approach, i.e. it is the human framework with matches at best the non-human framework to be replaced, when performing a sequence alignment.

The species of origin of an antibody (for example a human antibody) can be assessed by analyzing the sequence homology of said antibody with immunoglobulins of different species, and by determining the species of the immunoglobulin(s) with which said antibody shows the highest homology. This analysis can be carried out, for example, by consulting the database of the International Immunogenetics Information System (www.igmt.org).

By using a library of intrabodies derived from human antibodies, a screening method according to this embodiment is particularly well adapted for identifying targets in human cells. It can also identify intrabodies which are directly valuable for a therapeutic use in human, because of their non-immunogenicity.

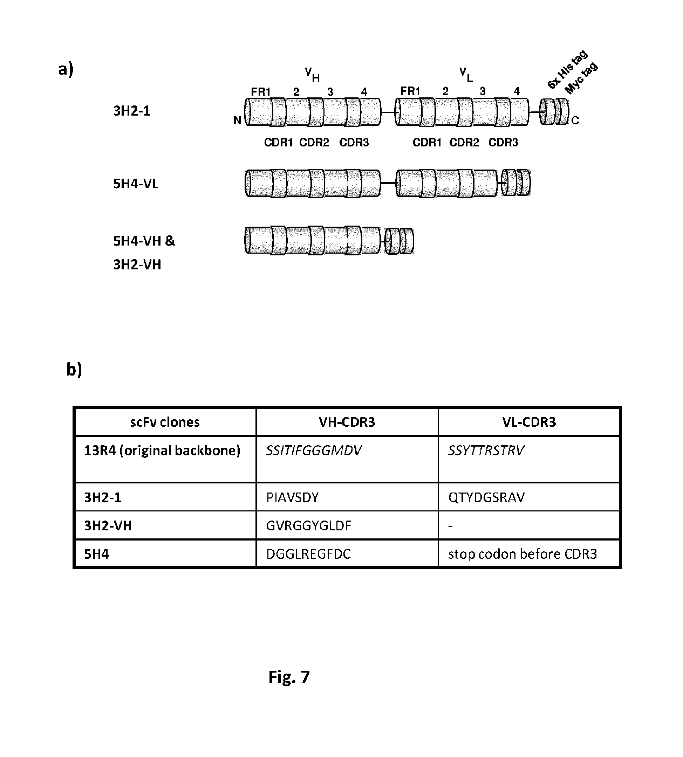

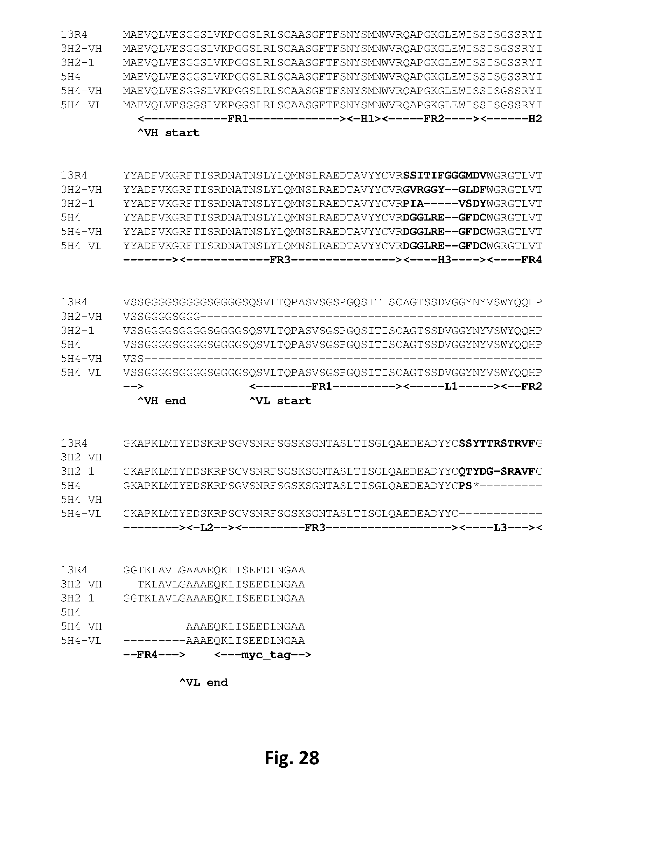

According to a specific embodiment the intrabody is a 3H2-1 intrabody, i.e. an intrabody comprising the CDR3 sequence of the V_{H} domain of scFv 3H2-1 as set forth in Figure 7, i.e PIAVSDY. Preferably, the 3H2-1 intrabody also comprises the CDR1 and CDR2 sequences of the V_{H} domain of scFv 3H2-1. Even more preferably, the 3H2-1 intrabody further comprises the CDR3 sequence of the V_{L} domain of 3H2-1, i.e. QTYDGSRAV, and optionally also its CDR1 and CDR2 sequences. According to a preferred embodiment, the 3H2-1 intrabody consists of or comprises the amino acid sequence 3H2-1 (SEQ ID NO:1) set forth in Figure 28. The intrabody may also be a variant of a 3H2-1 intrabody as defined above, in particular an intrabody whose amino acid sequence comprises one, two or three mutations in a CDR region, in particular a CDR3 region, more particularly the CDR3 region of the V_{H} domain, wherein said mutation(s) do not affect the ability of the intrabody to interact with the ABCF1 protein or another protein of the ABCF1 family. Alternatively, the intrabody is an intrabody which partly or totally suppresses the interaction between scFv 3H2-1 (SEQ ID NO: 1) and a protein of the ABCF1 family, preferably ABCF1.

According to another specific embodiment, the intrabody is a 3H2-VH intrabody, i.e. an intrabody comprising the CDR3 sequence of the V_{H} domain of 3H2-VH as set forth in Figure 7, i.e GVRGGYGLDF. Preferably, the 3H2-VH intrabody also comprises the CDR1 and CDR2 sequences of the V_{H} domain of 3H2-VH. According to a preferred embodiment, the 3H2-VH intrabody consists of or comprises the amino acid sequence 3H2-VH (SEQ ID NO: 2) set forth in Figure 28. The intrabody may also be a variant of a 3H2-VH intrabody as defined above, in particular an intrabody whose amino acid sequence comprises one, two or three mutations in a CDR region, in particular a CDR3 region, more particularly the CDR3 region of the V_{H} domain.

According to another specific embodiment the intrabody is a 5H4 intrabody, i.e. an intrabody comprising the CDR3 sequence of the V_{H} domain of scFv 5H4 as set forth in Figure 7, DGGLREGFDC. Preferably, the 5H4 intrabody also comprises the CDR1 and CDR2 sequences of the V_{H} domain of scFv 5H4. According to a preferred embodiment, the 5H4 intrabody consists of or comprises the amino acid sequence 5H4 (SEQ ID NO: 3), 5H4-V_{H} (SEQ ID NO: 4) or 5H4-V_{L} (SEQ ID NO: 5) set forth in Figure 28. The intrabody may also be a variant of a 5H4 intrabody as defined above, in particular an intrabody whose amino acid sequence comprises one, two or three mutations in a CDR region, in particular a CDR3 region, more particularly the CDR3 region of the V_{H} domain, wherein said mutation(s) do not affect the ability of the intrabody to interact with the LOC297607 protein or another protein of the "LOC" family. Alternatively, the intrabody is an intrabody which partly or totally suppresses the interaction between at least one of scFv 5H4, 5H4-V_{L} or 5H4-V_{H}, and a protein of the "LOC" family, preferably LOC297607.

According to another specific embodiment the intrabody comprises one of the V_{H} CDR3 sequences set forth in SEQ ID NO:7 to SEQ ID NO: 18. Preferably, the intrabody comprises one of the V_{L} CDR3 sequences set forth in SEQ ID NO:19 to SEQ ID NO: 29 or comprises a glutamine as V_{L} CDR3 sequence. Preferably still, the intrabody comprises one of the combinations of V_{H} and V_{L} CDR3 sequences (also referred to as H3 and L3 respectively) set forth in Table 1. Preferably still, the intrabody comprises one or more of the CDR1 and CDR2 sequences of the V_{H} and/or V_{L} domain of scFv 13R4 as set forth in figure 28 and the V_{H} and V_{L} of one cluster selected in those set forth in Table 1. Preferably still, the intrabody further comprises one or more of the framework sequences FR1, FR2, FR3 and FR4 of the V_{H} and/or V_{L} domain of scFv 13R4 as set forth in figure 28. Preferably still, the intrabody comprises the framework sequences and CDR1 and CDR2 sequences of the V_{H} and/or V_{L} domain of scFv 13R4, as set forth in Figure 28.

As used herein, the term "SEQ ID NO:7 to SEQ ID NO: 18" may refer to each single sequence of the group, namely SEQ ID NO:7, SEQ ID NO: 8, SEQ ID NO:9 etc..., each considered individually. The term "SEQ ID NO:7 to SEQ ID NO: 18" may also refer to any combination of sequences in the group, such as "SEQ ID NO:8 and SEQ ID NO: 13", or "SEQ ID NO:9 and SEQ ID NO:12 and SEQ ID NO:16", these exemples being purely illustrative and non limiting. The same applies to the terms SEQ ID NO:19 to SEQ ID NO:29.

**Table 1**

| **Cluster** | **VH CDR3** | **VL CDR3** |
|---|---|---|
| R_1 | MDCVIGSYGYGIFDT (SEQ ID NO : 7) | QSFVRNSTS (SEQ ID NO : 19) |
| R_2 | GKVLKKAEYSDWLDN (SEQ ID NO : 8) | QQCSKFPLT (SEQ ID NO : 20) |
| R_3 | RSASCEH (SEQ ID NO : 9) | EQYDTAPPYT (SEO ID NO : 21) |
| R_4 | GEVGFDY (SEQ ID NO : 10) | QQYFSQPFT (SEQ ID NO : 22) |
| R_5 | TLECSRCGDYGFDL (SEQ ID NO : 11) | HQSNTYPFT (SEQ ID NO : 23) |
| R_6 | DGLYARMYYNGSYY (SEQ ID NO : 12) | QQYFSQPFT (SEQ ID NO : 24) |
| R_7 | ERRDDDGMYAYSYQFDV (SEQ ID NO : 13) | Q |
| R_8 | DGGLREGFDC (SEO ID NO:44) | Stop codon |
| R_9 | NPASKCVYLEHDFEK (SEQ ID NO :14) | QTCNCLTLV (SEQ ID NO : 25) |
| R_10 | PERSAYDY (SEQ ID NO : 15) | QQYSSHPLT (SEQ ID NO : 26) |
| D_1 | GDSHIIDC (SEQ ID NO : 16) | QQSNILSVT (SEQ ID NO : 27) |
| D_2 | GSTAGFDY (SEO ID NO : 17) | QQDDSTPYT (SEQ ID NO : 28) |
| D_3 | EEGVDIEY (SEQ ID NO: 18) | PSLNNSLTYIV (SEQ ID NO : 29) |

In a particular embodiment of the method of the invention, step a) comprises the screening of an intracellularly expressed intrabody library, to identify an intrabody which can induce, modify or suppress the cell phenotype. Preferably, each intrabody in the library comprises a full V_{H} and/or V_{L} domain.

An Intrabody library can for example be obtained by selecting an intrabody which is functional inside a cell. An intrabody functional inside a cell is preferably an intrabody that, when expressed inside the cell, is able to bind an intracellular target, especially to modify a phenotype of the cell. On average, only about 0.1 to 1% intrabodies are suitable for intracellular use. This proportion can be significantly increased in particular by using the method described in WO2008/110914 and in Philibert et al., 2007. Briefly, this method first comprises the selection of an intrabody, for example a scFv such as 13R4 (SEQ ID NO: 6), which is stable *in vitro* and shows optimal folding proprieties in the cell cytosol, and then the introduction of modifications in one or more of its CDR regions, preferably in the CDR3 region of the V_{H} and/or V_{L} chain (also referred to as H3 and L3 respectively), more particularly in the heavy chain CDR3 (H3). The modifications can consist of point mutations, additions and/or deletions of one or more amino acids, for example of one to twelve amino acids. Mutations can also be introduced into the framework regions.

In a specific embodiment, said intrabody library and/or said library of molecules is obtained by selecting an intrabody which is functional inside a cell, and then introducing modifications in one or more of its CDR regions, especially the CDR3 region of the V_{H} and/or V_{L} domain.

In another embodiment, the intrabody library comprises scFvs and truncated scFvs comprising at least a full V_{H} or V_{L} domain.

Preferably, the intrabody library comprises at least 10⁵, more preferably at least 10⁶, 10⁷, 10⁸, 10⁹ or 10¹⁰ different intrabodies.

Using a high diversity library of intrabodies increases the number of possible targets. A high number of intrabodies can not only give access to the whole diversity of intracellular targets, including the various post-translational variants of a protein, but it can also give access to various epitopes on the same target. Different mechanisms of action of a single target can be targeted, thus increasing the success rate of the screening method.

In a preferred embodiment, step a) comprises:
i) obtaining a library of molecules, wherein each molecule from the library encodes a different intrabody comprising a full V_{H} and/or V_{L} domain of an immunoglobulin;
ii) transfecting a population of cells with the library of molecules of step i);
iii) culturing the transfected cells for a time and under conditions sufficient for detectable induction, modification or suppression of said phenotype;
iv) selecting the cells of step iii) which show an induction, modification or suppression of said phenotype;
v) optionally repeating steps iii) and iv) on the cells selected from step iv) or on cells recloned from the cells selected from step iv) for one or more additional selection rounds; and
vi) identifying the intrabody which is responsible for said phenotype induction, modification or suppression.

According to a specific embodiment, steps iii) to iv), forming a selection round, are repeated more than once. This means that the cells selected from step iv) are recultured according to step iii) for a further selection step. For example 2 to 10 selection rounds, preferably 5 to 8, may be performed prior to step vi). Further, the method may comprise a recloning step after one or more selection rounds, i.e. after step iv) or v). The recloning step may for example be performed after each selection round or after some of the rounds, for example each two rounds. Thereafter the recloned cells may be submitted to one or more selection rounds. The recloning procedure may comprise the extraction of the DNA from the selected cells after step iv), the amplification of the scFv sequences by PCR and their recloning into the expression vector and their transfection again into cells. This allows for a more marked phenotype at each round of selection and for enrichment in scFv sequences which generate the studied phenotype.

Preferably, the molecules encoding the intrabodies are vectors. Preferably the vectors are suitable for expression in eukaryotic cells, for example in mammalian cells, such as human, rodent and primate cells. The vectors may be non-integrative vectors which allow for transient expression, for example plasmids or adenoviral vectors, or integrative vectors such as retroviral vectors. The vector may also encode a signal which directs the intrabody to a specific cellular compartment or to the cell membrane.

In particular when using a non integrative vector, a high number of copies, for example between 100 and 100000 copies, preferably between 500 and 20000 copies, for example about 10000 copies may be transfected per cell. According to this embodiment, less cells are required. Additional subcloning steps are performed subsequently to identify the intrabody of interest. This embodiment is in particular suitable for screening for modifications in a dominant phenotype.

When using an integrative vector, it is preferred that less than five copies, more preferably one, two or three copies are transfected per cell.

The cell can be transfected *in vitro* or *in vivo* as part of a whole organism. If the cell is transfected in vivo, the cell phenotype can be a phenotype which may be determined in the whole organism.

The intrabody may be fused to another protein, for example a protein which causes a detectable signal such as the GFP protein.

The cellular target may be a fully intracellular or partially intracellular molecule. In the case of a partially intracellular molecule, it can be a membrane protein, which may comprise an intracellular and/or an extracellular domain. The cellular target may in particular be a protein, a peptide, a carbohydrate, a lipid or even a nucleic acid. If the target is a protein, it may be for example an enzyme such as a kinase or a protein phosphatase, a transmembrane receptor, a transcription factor, a scaffolding protein such as the tubulin, metabolic enzymes, proteins implicated in protein synthesis and turnover such as ribosomal proteins, chaperones and proteases... The cellular target may be endogenous or exogenous to the cell. Exogenous targets include in particular proteins, nucleic acids, particles of viral origin as well as whole viruses, bacterial antigens. The cellular target may also be an unknown protein or a known protein whose function is unknown.

The cellular target is a target which interacts with the intrabody either directly by binding to it (direct target), or indirectly in the sense that in does not bind to the intrabody but that the intrabody has an indirect effect on it. A direct target may be a target for which the dissociation constant (Kd) corresponding to its binding with the intrabody *in vitro* is at least 1nM. An indirect target can also be a molecule which interacts with another cellular molecule with which the intrabody interacts also, or can be a molecule which is part of a cellular pathway which is modulated by the intrabody. For example, an indirect target may be a molecule which can be precipitated with the intrabody because it binds to another cellular molecule to which the intrabody also binds.

The interaction, whether direct or indirect, between the intrabody and the target may have a modulatory effect on or more several cellular pathways. This interaction can for example change the conformation and/or the phosphorylation status of the target, and thereby induce, enhance or repress its ability to interact with one or more other molecules, in particular proteins, inside the cell. This interaction may completely or partially activate or inactivate the target and/or another protein.

The cell phenotype can be any observable or measurable trait of the cell, including a morphological feature, a developmental stage, a biochemical or physiological property. For example the phenotype may be cell death, the onset of senescence, a resistance to an antibiotic, a resistance to a viral or bacterial attack, the expression or loss of expression of a receptor, the release of a compound outside the cellular environment. The phenotype may also be any type of signal, for example a signal, such as fluorescence, triggered by the expression of a recombinant reporter gene. Preferably, the cell phenotype is a phenotype associated with an animal or plant disease, preferably a pathology which affects a human being. Such a phenotype is thus a phenotype of therapeutic or diagnostic interest. In a particular embodiment, the phenotype is a phenotype associated with allergy or inflammation, in particular with a type I allergic reaction. The phenotype may be a trait which can be observed or measured *in vitro* on a cell culture or *in vivo* on a whole being.

The modification in the cell phenotype may be a modulation, such as an increase or a decrease in intensity, the induction (or the occurrence) or the suppression (or repression) of the cell phenotype.

The cell may be a prokaryote cell or a eukaryote cell. Preferably, the cell is a eukaryotic cell, such as a yeast cell or a higher eukaryote cell, for example an animal cell or a plant cell. In a specific embodiment, the cell is a mammalian cell, such as a mouse, rat or human cell. In a preferred embodiment, the cell is a human cell. According to a specific embodiment, the cell is a eukaryotic cell involved in allergy, inflammation, or both, and the cell phenotype is a phenotype associated with an allergic reaction, an inflammatory reaction, or both. For example the cell is a mast cell and the cell phenotype is degranulation and release of pro-inflammatory mediators.

The intrabody which is responsible for the phenotype induction, modification or suppression may be identified by any suitable means. For example, the cells which show the phenotype induction, modification or suppression are cloned and the DNA from each clone is extracted. The genes encoding the scFv are amplified by PCR and sequenced.

The direct or indirect target of the intrabody may be isolated by any suitable means. For example, the direct or indirect target of the intrabody can be isolated by a binding assay wherein the intrabody is marked with a detectable label. Alternatively, the direct or indirect target is immunoprecipitated together with the labelled intrabody. Once the target has been isolated, Mass spectrometry (MS-MS) can for example be used to identify it.

According to a specific embodiment, the method further comprises a step of target validation using RNA interference technology or a known inhibitor of said target such as an antibody. The method may also comprise a step of identification of the epitope of the target, and/or a step of characterisation of the target. The identification of the epitope is of particular interest as it may guide towards the identification of a site of interest on the target. In yet another embodiment, the method may comprise a step of identification of a molecule which competes with the binding of the intrabody identified in step a) to the protein identified in step b), and which is capable of modifying said cell phenotype.

Another aspect of the invention is an intrabody comprising the CDR3 sequence of the V_{H} domain of scFv 3H2-1 as set forth in Figure 7, i.e. PIAVSDY (SEQ ID NO:42) ("3H2-1 intrabody"). Preferably, the 3H2-1 intrabody also comprises the CDR1 and CDR2 sequences of the V_{H} domain of scFv 3H2-1. Even more preferably, the 3H2-1 intrabody further comprises the CDR3 sequence of the V_{L} domain of 3H2-1, i.e. QTYDGSRAV, and optionally also its CDR1 and CDR2 sequences. According to a preferred embodiment, the 3H2-1 intrabody consists of or comprises the amino acid sequence 3H2-1 (SEQ ID NO:1) set forth in figure 28. The intrabody may also be a variant of a 3H2-1 intrabody as defined above, in particular an intrabody whose amino acid sequence comprises one, two or three mutations in a CDR region, in particular a CDR3 region, more particularly the CDR3 region of the V_{H} domain, wherein said mutation(s) do not affect the ability of the intrabody to interact with the ABCF1 protein or another protein of the ABCF1 family. In a particular embodiment, the intrabody is for use in therapy, especially in treating allergy and/or inflammation.

A further aspect of the invention is an intrabody comprising the CDR3 sequence of the V_{H} domain of 3H2-VH as set forth in Figure 7, i.e. GVRGGYGLDF (SEQ ID NO:43) ("3H2-VH intrabody"). Preferably, the 3H2-VH intrabody also comprises the CDR1 and CDR2 sequences of the V_{H} domain of 3H2-VH. According to a preferred embodiment, the 3H2-VH intrabody consists of or comprises the amino acid sequence 3H2-VH (SEQ ID NO:2) set forth in figure 28. The intrabody may also be a variant of a 3H2-VH intrabody as defined above, in particular an intrabody whose amino acid sequence comprises one, two or three mutations in a CDR region, in particular a CDR3 region, more particularly the CDR3 region of the V_{H} domain, wherein said mutation(s) do not affect the ability of the intrabody to interact with its unknown target so far. In a particular embodiment, the intrabody is for use in therapy, especially in treating allergy and/or inflammation.

A further aspect of the invention is an intrabody comprising the CDR3 sequence of the V_{H} domain of the intrabody 5H4 as set forth in Figure 7, i.e. GGLREGFDC (SEQ ID NO: 44) ("5H4 intrabody"). Preferably, the 5H4 intrabody also comprises the CDR1 and CDR2 sequences of the V_{H} domain of scFv 5H4. According to a preferred embodiment, the 5H4 intrabody consists of or comprises the amino acid sequence 5H4 (SEQ ID NO:3), 5H4-V_{H} (SEQ ID NO:4) or 5H4-V_{L} (SEQ ID NO:5) set forth in Figure 28. The intrabody may also be a variant of a 5H4 intrabody as defined above, in particular an intrabody whose amino acid sequence comprises one, two or three mutations in a CDR region, in particular a CDR3 region, more particularly the CDR3 region of the V_{H} domain, wherein said mutation(s) do not affect the ability of the intrabody to interact with the LOC297607 protein or another protein of the "LOC" family. In a particular embodiment, the intrabody is for use in therapy, especially in treating allergy and/or inflammation.

Another aspect of the invention is an intrabody comprising one of the V_{H} CDR3 sequences set forth in Table 1 for use in therapy, especially for use in treating allergy and/or inflammation. Preferably, the antibody further comprises one of the V_{L} CDR3 sequences set forth in Table 1. Still preferably, it comprises one of the combinations of V_{H} and V_{L} CDR3 sequences set forth in Table 1. Preferably, the intrabody comprises the framework sequences and CDR1 and CDR2 sequences of scFv 13R4 as set forth in figure 28 and one of the combinations of V_{H} and V_{L} CDR3 sequences set forth in Table 1.

Another aspect of the invention is a nucleic acid sequence comprising or consisting in a sequence encoding an intrabody according to the invention as defined above, or a vector containing said nucleic acid sequence, or an eukaryotic cell containing said nucleic acid sequence or said vector.

Preferably, the vector is suitable for introduction and expression of the nucleic acid in mammalian cells, in mammalian tissue, or in a mammalian organ, for example a retroviral vector, a lentiviral vector or a plasmid.

Another aspect of the invention consists in the use of a 3H2-1 intrabody as defined above for identifying a molecule which is capable of competing with the binding of said intrabody with a protein of the ABCF1 family, preferably the ABCF1 protein, and of modifying a phenotype associated with an allergic reaction, an inflammatory reaction or both, in a cell involved in allergy, inflammation, or both.

The ABCF1 (ATP-binding cassette sub-family F member 1) protein is a member of the ABC protein superfamily. It is part of the subfamily F or GCN20 of the superfamily. The ABC sub-family F comprises other members, such as the ABCF2 and ABCF3 proteins.

A protein of the ABCF1 family can consist in a protein having an amino acid sequence with at least 60% identity, preferably at least 70% identity, most preferably at least 80% or at least 90% or least 95% or least 99% identity with the amino acid sequence set forth in SEQ ID NO:41. It can also consist in a protein encoded by a polynucleotide with at least 60% identity, preferably at least 70% identity, most preferably at least 80% or at least 90% or at least 95% or least 99% identity with the nucleotide sequence set forth in SEQ ID NO:40. It can especially consist into the human protein ABCF1 or into any ortholog of the human ABCF1 protein. It can also consist into any homolog or paralog of the human ABCF1 protein. It is to be noted that the gene encoding the ABCF1 protein is highly conserved in many species (for example in chimpanzee, Rhesus monkey, dog, cow, mouse, rat, zebrafish, fruit fly, mosquito, C. elegans, S. pombe, A. Thaliana and rice).

A further aspect of the invention consists in the use of a 5H4 intrabody as defined above for identifying a molecule which is capable of competing with the binding of said intrabody with a protein of the C12ORF4 or "LOC" family, and of modifying a phenotype associated with an allergic reaction, an inflammatory reaction or both, in a cell involved in allergy, inflammation, or both.

A protein of the C12ORF4 or "LOC" family can consist in a protein having an amino acid sequence with at least 60% identity, preferably at least 70% identity, most preferably at least 80% or at least 90% or at least 95% or at least 99% identity with one of the amino acid sequences set forth in SEQ ID NO: 33, SEQ ID NO:35, SEQ ID NO:37 and SEQ ID NO:39 , preferably with the amino sequence set forth in SEQ ID NO:35. It can also consist in a protein encoded by a polynucleotide with at least 60% identity, preferably at least 70% identity, most preferably at least 80% or at least 90% or at least 95% or at least 99% with one of the nucleotide sequences of set forth in SEQ ID NO: 32, SEQ ID NO:34, SEQ ID NO:36, SEQ ID NO:38, preferably with SEQ ID NO: 34. It can especially consist into one of the proteins C12ORF4, LOC297607, LOC57102 or LOC28040, preferably into the human C12ORF4 protein or into any ortholog of the human C12ORF4 protein. It can also consist into any homolog or paralog of the human C12ORF4 protein. It is to be noted that the gene encoding the protein C12ORF4 is highly conserved in many species (for example in chimpanzee, dog, cow, mouse, rat, chicken, zebrafish, fruit fly, mosquito, and C.elegans).

The phenotype associated with an allergic reaction, an inflammatory reaction or both may be related to a disease or condition where allergic reaction or inflammatory reaction happen, especially Type I, II, III and IV hypersensitivity, rheumatoid arthritis, ankylosing spondylitis, and systemic lupus erythematosus (SLE).

The cells involved in allergy, inflammation, or both may be selected among mast cells and basophil granulocytes, phagocytic cells or phagocytes (granulocytes-mainly neutrophil- and scavenger cells), and lymphocytes.

According to a particular embodiment of one or the other of the uses above, the molecule which is capable of competing with the binding of the intrabody with the target protein is an organic molecule having a molecular weight of 100 to 2500 Da. In yet another aspect, the invention is directed to an inhibitor of a protein of the ABCF1 family, in particular of ABCF1, for use in a method for treating allergy, inflammation, or both. Such an inhibitor is herein referred to as an "ABCF1 inhibitor".

In a specific embodiment, the ABCF1 inhibitor according to the invention is for use in a method for treating allergy, inflammation, or both, especially for treating a disease or condition where allergic reaction and/or inflammatory reaction happen especially Type I, II, III and IV hypersensitivity, rheumatoid arthritis, ankylosing spondylitis, and systemic lupus erythematosus (SLE).

In yet another aspect, the invention relates to a method for manufacturing a medicament or a pharmaceutical composition for treating allergy, inflammation, or both, comprising:
- identifying an inhibitor of a protein of the ABCF1 family, especially of ABCF1;
- manufacturing a medicament or a pharmaceutical composition comprising said inhibitor.

In a particular embodiment, the ABCF1 inhibitor is
- an intrabody or an antigen-binding fragment thereof capable of binding to a protein of the ABCF1 family, in particular to protein ABCF1, preferably a 3H2-1 intrabody according to the invention;
- an RNA molecule capable of interfering with the expression of a protein of the ABCF1 family, in particular of ABCF1, in a cell; or
- an organic molecule having a molecular weight of 100 to 2500 Da which is capable of displacing a 3H2-1 intrabody according to the invention from its binding site with a protein of the ABCF1 family, in particular of ABCF1.

According to the invention, an antigen-binding fragment of an intrabody may comprise at least one CDR loop of a V_{H} or V_{L} domain, preferably the CDR3 loop of a V_{H} or V_{L} domain, still preferably of a V_{H} domain.

In yet another aspect, the invention is directed to an inhibitor of a protein of the C12ORF4 family, also referred as "LOC" family, in particular of C12ORF4, LOC297607, of LOC57102 or of LOC28040, most preferably of C12ORF4, for use in therapy. Such an inhibitor is herein referred to as a "LOC inhibitor". In a specific embodiment, the LOC inhibitor according to the invention is for use in a method for treating allergy, inflammation, or both, especially for treating a disease or condition where allergic reaction and/or inflammatory reaction happen especially Type I, II, III and IV hypersensitivity, rheumatoid arthritis, ankylosing spondylitis, and systemic lupus erythematosus (SLE).

In yet another aspect, the invention relates to a method for manufacturing a medicament or a pharmaceutical composition for treating allergy, inflammation, or both, comprising:
- identifying an inhibitor of a protein of the C12ORF4 family, especially of C12ORF4;
- manufacturing a medicament or a pharmaceutical composition comprising said inhibitor.

In a particular embodiment, the LOC inhibitor is
- an intrabody or an antigen-binding fragment thereof capable of binding to a protein of the C12ORF4 family, in particular to C12ORF4, LOC297607, LOC57102 or LOC29040, preferably to C12ORF4, preferably a 5H4, 5H4-VL or 5H4-VH intrabody according to the invention or an antigen-binding fragment thereof;
- an RNA molecule capable of interfering with the expression of a protein of the C12ORF4 family, in particular of C12ORF4, LOC297607, LOC57102 or LOC29040, preferably to C12ORF4 in a cell; or
- an organic molecule having a molecular weight of 100 to 2500 Da which is capable of displacing a 5H4 intrabody according to the invention from its binding site with a protein of the C12ORF4 family, in particular with C12ORF4, LOC297607, LOC57102 or LOC29040, preferably with C12ORF4.

### Figures

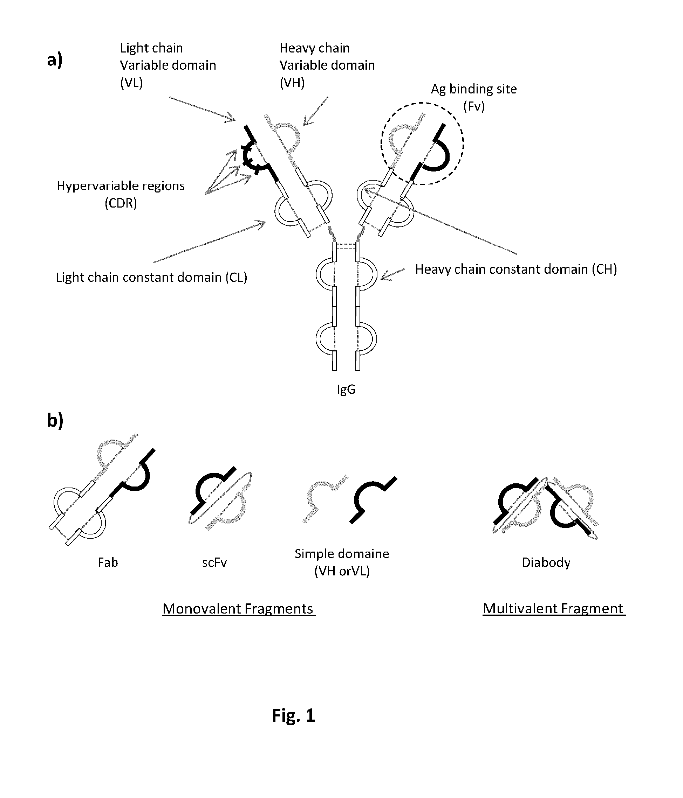

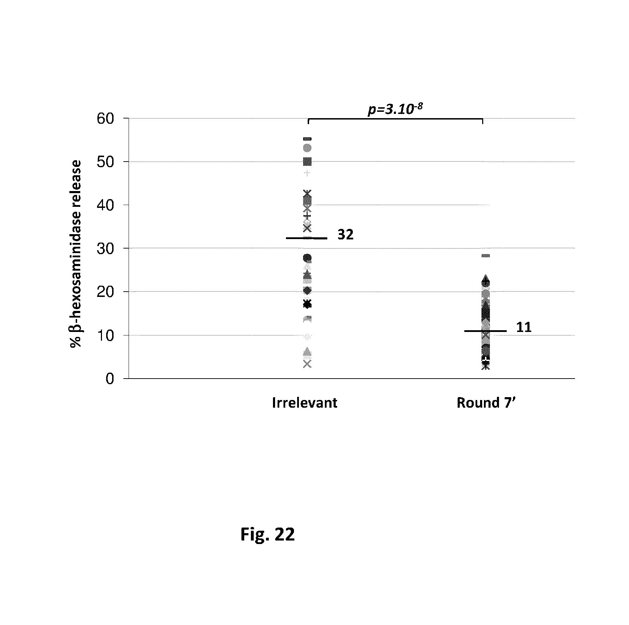

**Figure 1****: schematic view of an antibody molecule and of some possible recombinant fragments.**
   The heavy chain variable domain is shown in black. The light chain variable domain is shown in grey.
   a) is a schematic view of an IgG. Disulfide bonds are indicated with dotted lines
   b) is the representation of some monovalent and bivalent antibody fragments.
**Figure 2****: phenotypic selection principle.**
   The scFv library was cloned in an expression vector (Step 1) and was transfected in a suitable cell line (Step 2). The cells were sorted according to a given phenotype in Step 3. In the described case, sorting was performed by FACS after cell staining with fluorescent Annexin V. After a suitable number of selection rounds, the enriched scFv population was used to generate stable clones and the scFv genes were sequenced (Step 4). The best scFv were produced in E. coli and they were used to identify their target by capturing it from cellular extracts and analyzing the captured proteins by Mass spectrometry (Step 5). The implication of the target in the given phenotype was then confirmed using usual techniques (Step 6).

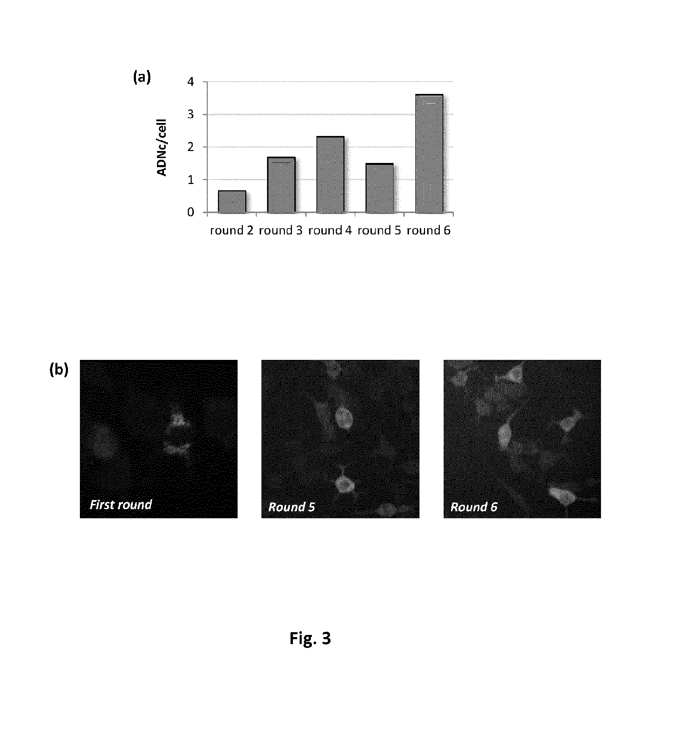

**Figure 3****: intracellular expression of the plasmidic intrabody library in the RBL-2H3 mast cell line.**
   In (a), the expression of the antibody fragments was measured by RT-PCR; and in (b) by immunofluorescence using anti-myc tag 9E10 monoclonal antibody, following the indicated selection rounds.

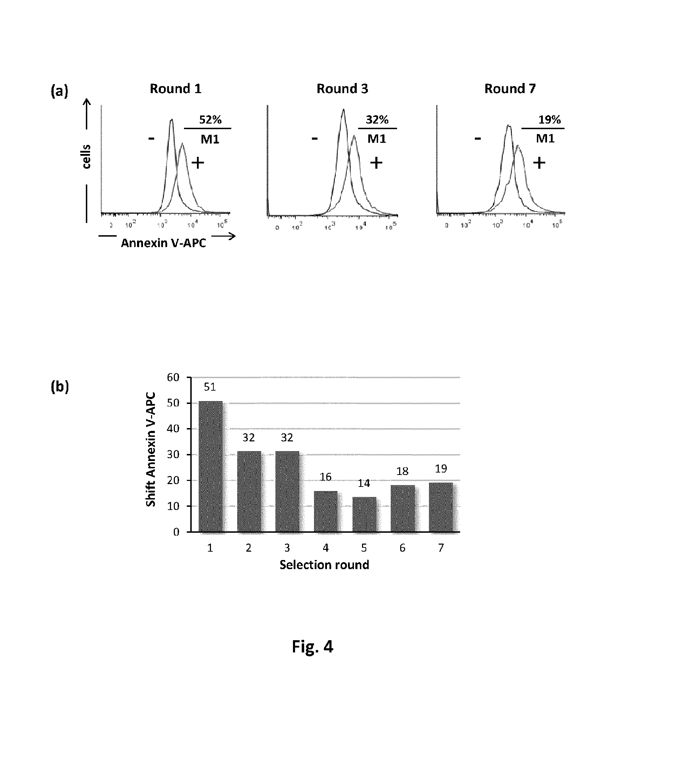

**Figure 4****: FACS enrichment of the transfected cell population throughout selection rounds.**
   For each round of selection, the cell population transfected with the plasmidic intrabody library was analyzed by FACS after staining with Annexin-V-APC. Cells were either stimulated with IgE/DNP (+) or non-stimulated by omitting the DNP (-). The value of the shift is represented in (a) by the horizontal marker labeled M1. (b) shows the value of the Annexin-V-APC shift for rounds 1 to 7 of the selection.

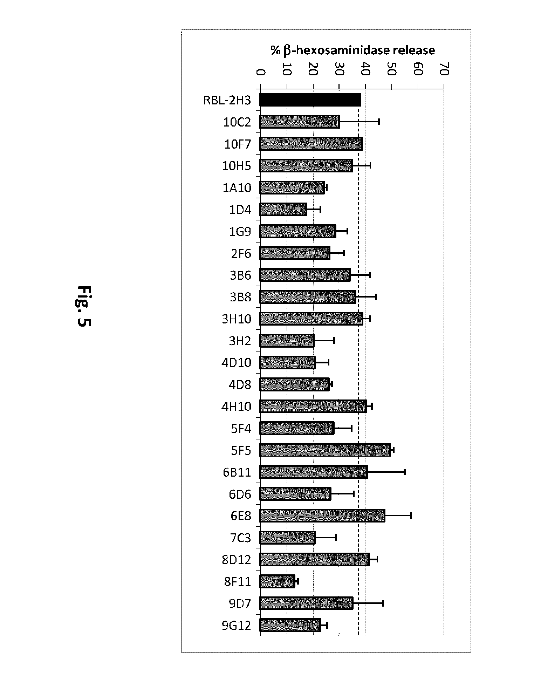

**Figure 5****: Percentage of FcεRI-mediated β-hexosaminidase release.**
   Analysis of 24 stables RBL-2H3 clones generated from the selection round 7 following plasmidic intrabody library transfection; The RBL-2H3 cell line was used as a positive control.

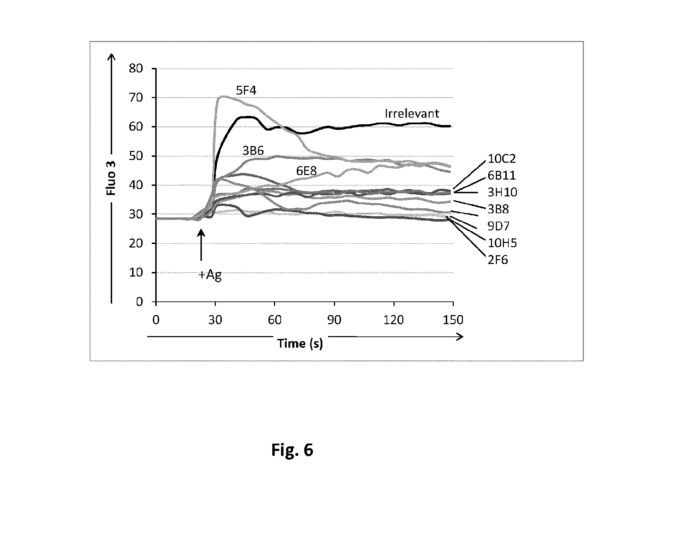

**Figure 6****: FcεRI-mediated increase of calcium flux.**
   Some stable RBL-2H3 clones generated from the selection round 7 were analyzed in parallel with an irrelevant non-inhibitory intrabody used as a positive control. IgE loaded cells were stimulated by adding the antigen DNP after 20 seconds and the rise of calcium was evaluated by FACS using Fluo 3 dye. The names of the tested clones are indicated in the figure.
**Figure 7****: Sequence of the scFv expressed in cellular clones 5H4 and 3H2.**
   a) Schematic view of 5H4-VH, 5H4-VL, 3H2-1 and 3H2-VH fragments.
   b) The sequence of the CDR3 loops are indicated in comparison with the original scFv 13R4 .

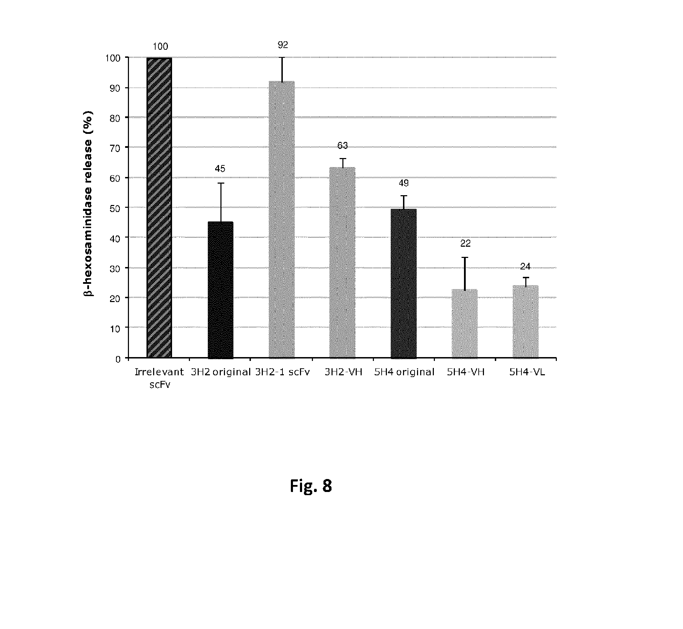

**Figure 8****: β-hexosaminidase release of the RBL-2H3 stable clones following FcεRI stimulation.**

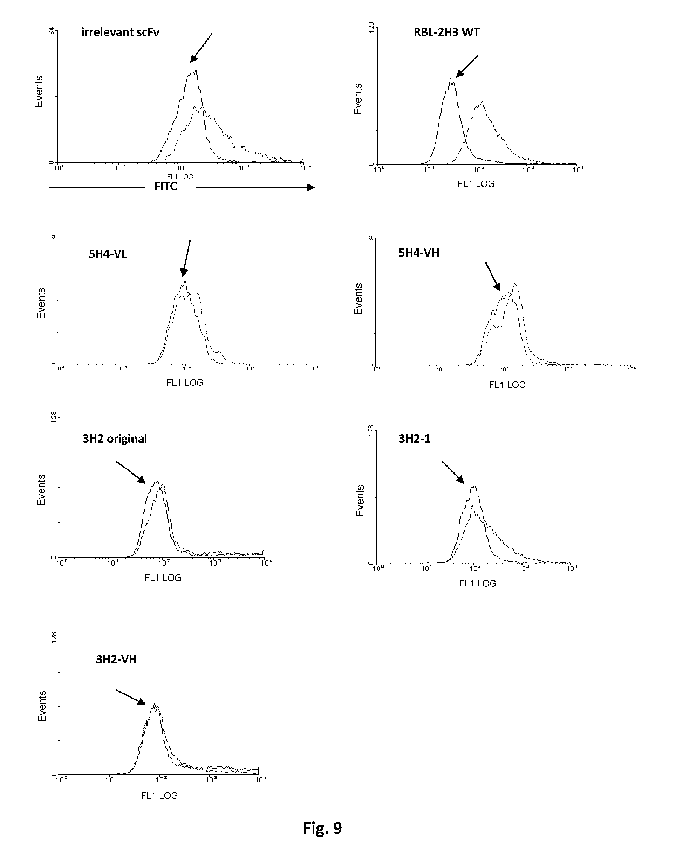

**Figure 9****: FcεRI -mediated Annexin-V-APC staining measured by FACS.**
   The arrow indicates the non-stimulated cell population (+IgE/-DNP); FcεRI stimulation-dependent Annexin V shift follows the incubation of cells with IgE and the antigen DNP (+IgE/+DNP).

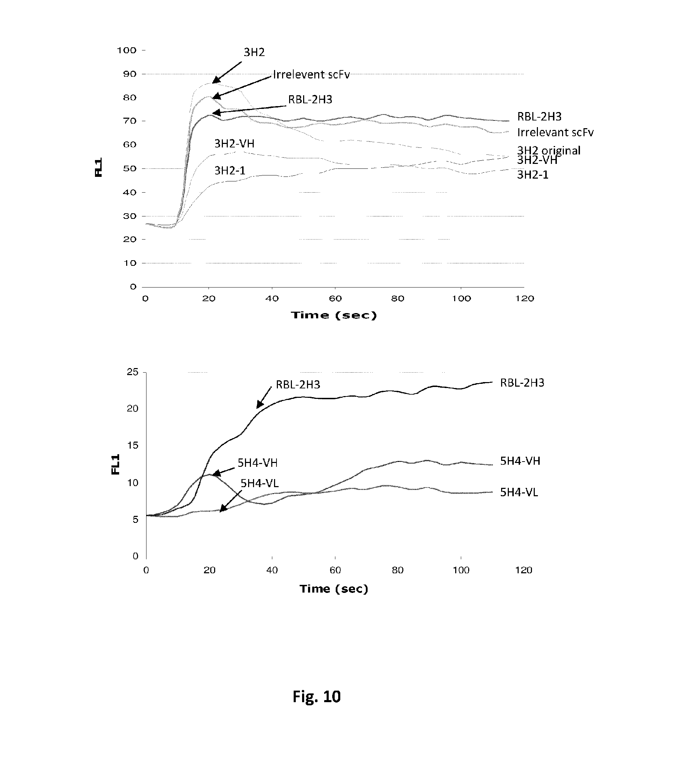

**Figure 10****: Calcium flux visualized by FACS.**
   To measure the calcium flux, the antigen DNP was added to IgE loaded cells and the rise of intracellular calcium was measured by FACS using the Fluo 3 dye.

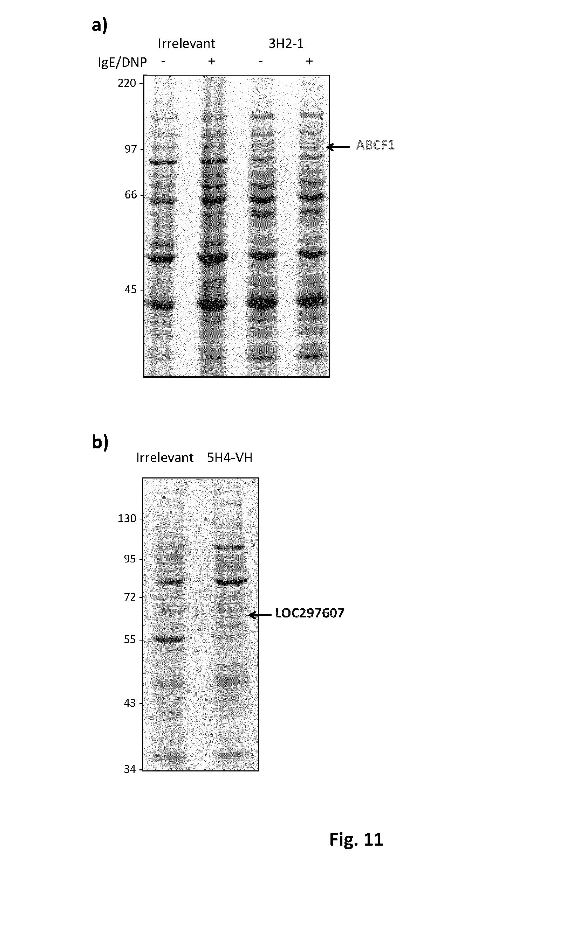

**Figure 11****: Target identification.**
   Mass spectrometry analysis of the proteins captured from a cellular extract using (a) the scFv 3H2-1 and (b) 5H4-VH fragment.
   The band corresponding to the protein with the highest Protscore is indicated with an arrow on the gels.

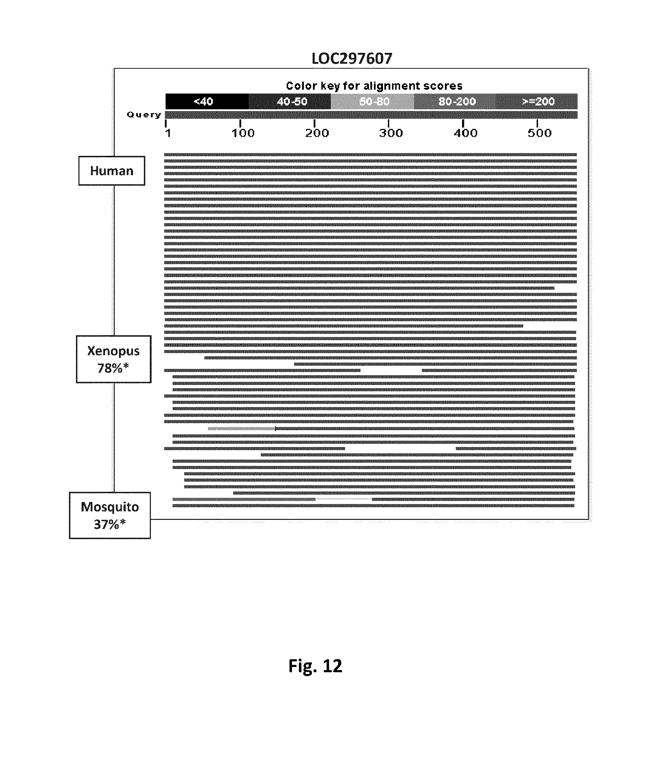

**Figure 12****: BLAST alignment of LOC297607.**
   The sign "*" indicates the percentage of sequence identity using the human protein as reference.

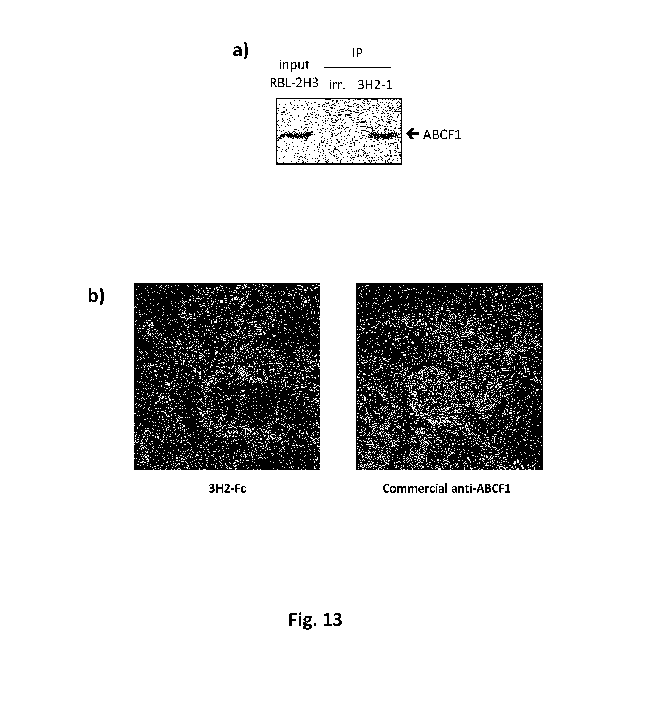

**Figure 13****: Validation of the scFv 3H2-1 target.**
   The identity of the protein identified in Figure 11 a was confirmed using a commercial antibody directed against ABCF1 protein. (a) Cellular extracts captured with the scFv 3H2-1 versus an irrelevant scFv (irr) were revealed using the commercial anti-ABCF1 antibody. (b) Immunofluorescence analysis of RBL-3H2 cells using the scFv 3H2-1-Fc fusion (left) and the commercial anti-ABCF1 antibody (right).

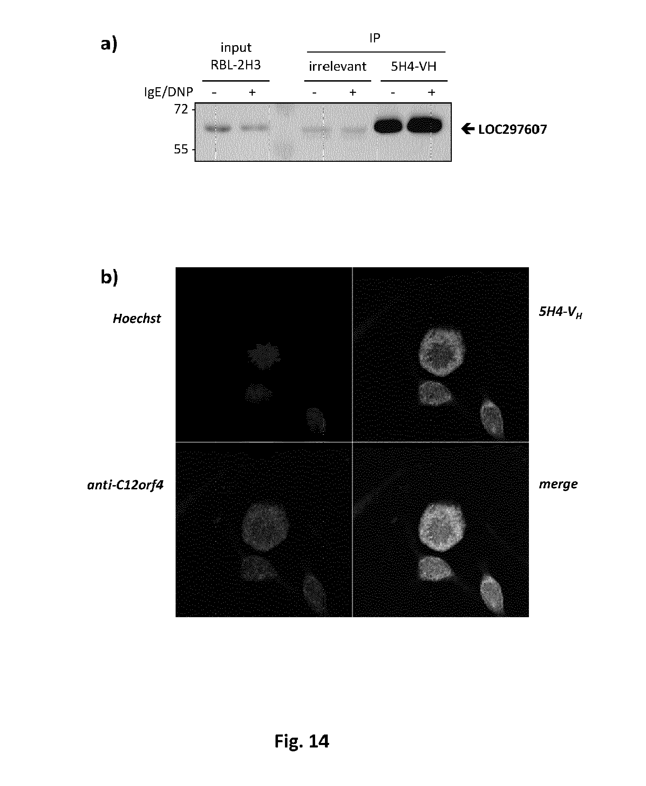

**Figure 14****: Validation of the 5H4-VH fragment target.**
   The identity of the protein identified in Figure 11 b was confirmed using a commercial antibody directed against C12orf4 protein. (a) Cellular extracts captured with 5H4-VH fragment versus an irrelevant VH were revealed using the commercial antiC12orf4 antibody. (b) Immunofluorescence confocal analysis of RBL-3H2 cells stained with 5H4-VH-Fc fusion (top right panel), the commercial anti-C12orf4 antibody (bottom left panel) and the merge (bottom right panel). The top left panel represents the Hoechst staining of the nucleus.

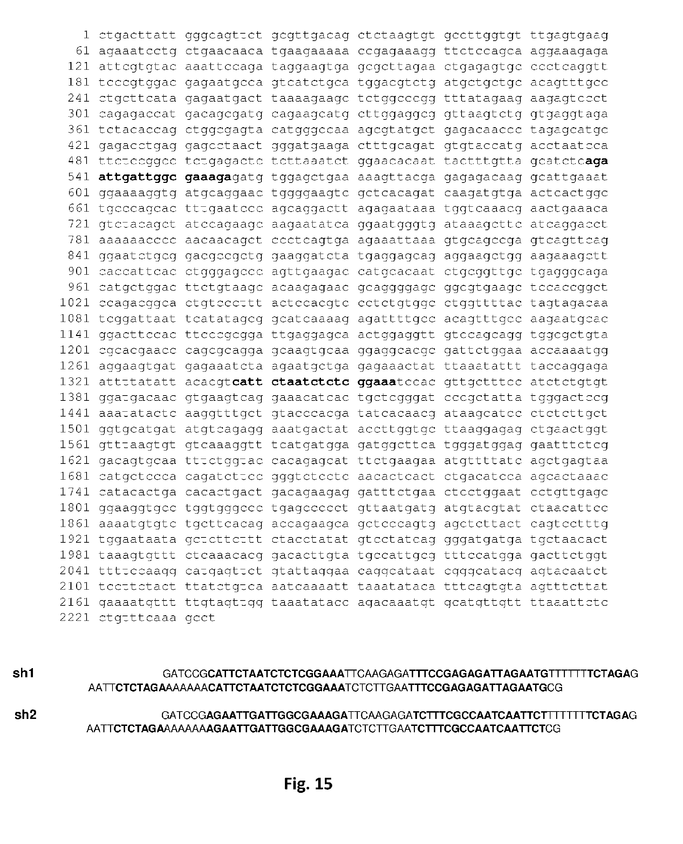

**Figure 15****: Nucleic acid sequence of LOC297607, the Rat C12Orf4 gene.**
   The localization (in bold in the sequence) and the sequences of the two shRNA are indicated.

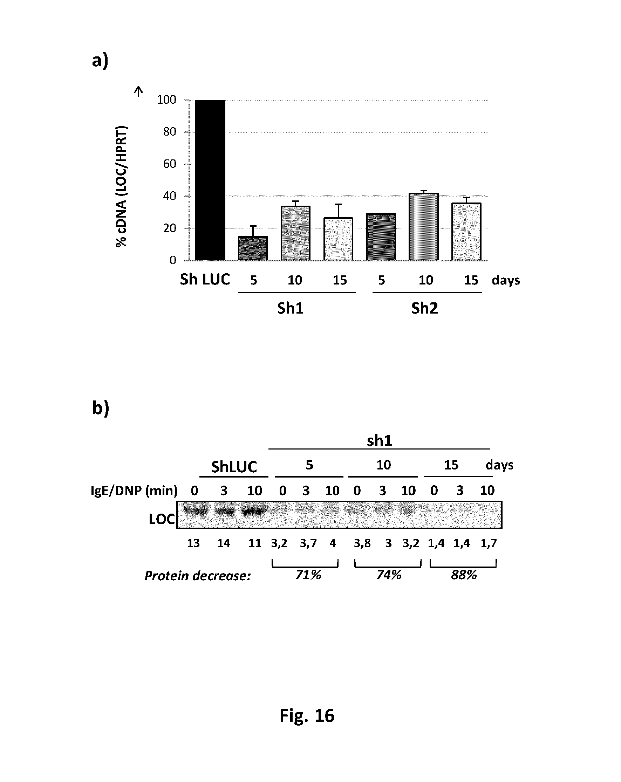

**Figure 16****: Invalidation of LOC297607 in the RBL-2H3 cell line using two shRNA (sh1 and sh2) evaluated by (a) qPCR and (b) by western blot.**
   An RBL-2H3 cell population transfected with an irrelevent shRNA specific to Luciferase (shLUC) was used as a control.

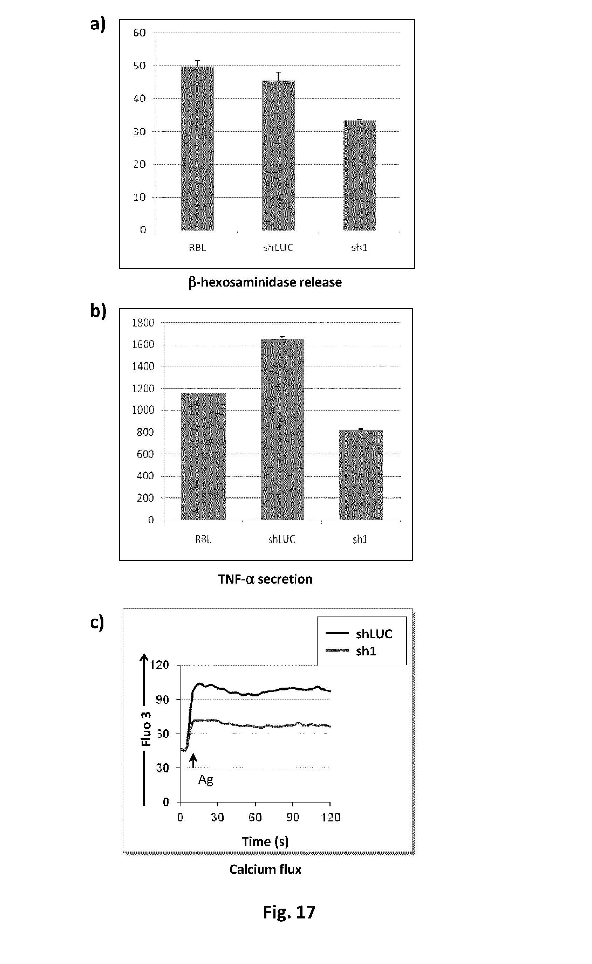

**Figure 17****: Cellular phenotypes associated with invalidation of LOC297607 using shRNA.**
   FcεRI-mediated activation of the transfected RBL-2H3 cell populations was evaluated by the measure of: (a) β-hexosaminidase release; b) TNF-α secretion; c) rise of intracellular calcium flux. The RBL-2H3 cell line was used in parallel with the cell populations transfected either with shRNA specific to the Luciferase (shLUC) as a control, or with shRNA specific to LOC297607 (sh1).

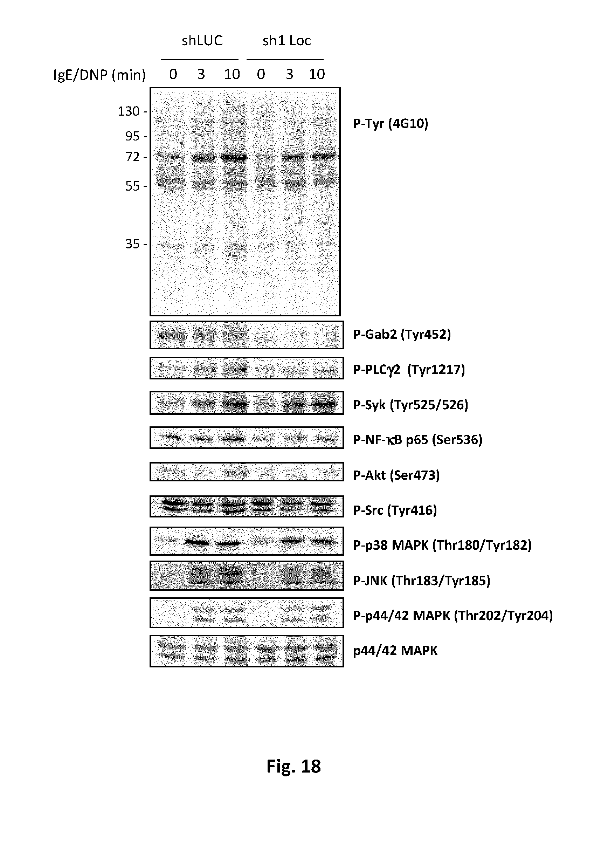

**Figure 18****: Western blot analysis of the RBL-2H3 populations transfected with sh1 Loc and shLUC (control), 5 days post-selection.**
   Cell extracts were prepared 0, 3 and 10 minutes after FcεRI stimulation (+IgE/+DNP). Antibodies specific to the indicated targets were used.

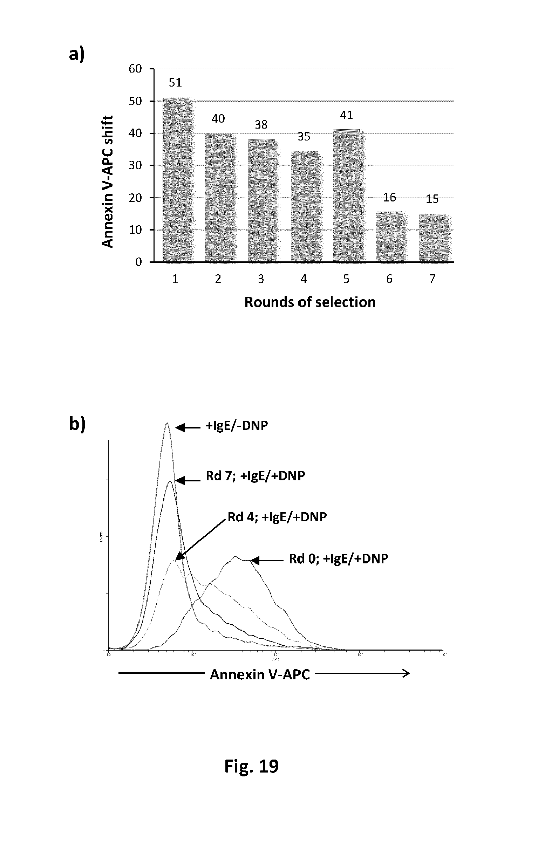

**Figure 19****: Annexin V-APC staining of RBL-2H3 cell populations issued from different rounds of selection following intrabody retroviral library transfection.**

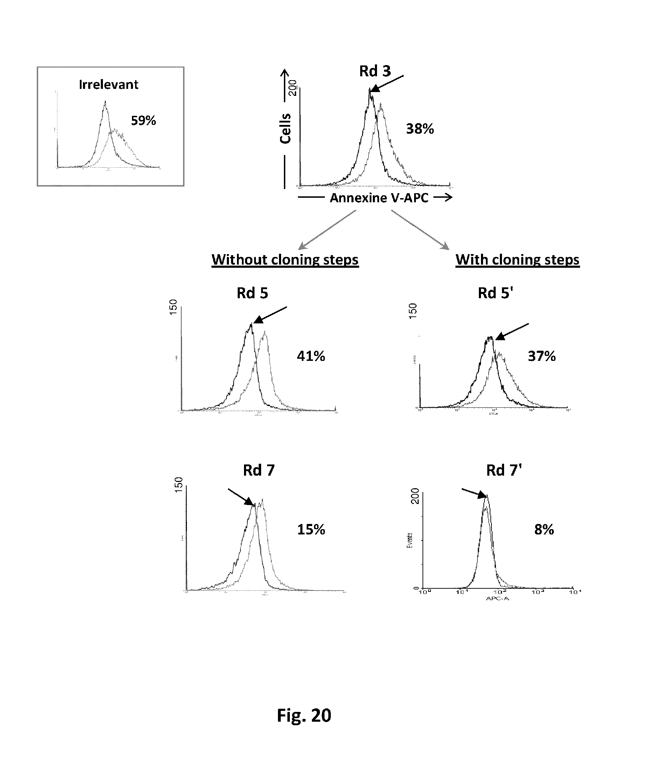

**Figure 20****: Comparison of Annexin V-APC staining throughout phenotypic selection steps of intrabody retroviral library.**
   The arrow indicates the non stimulated cell population (+IgE/-DNP); FcεRI stimulation-dependent Annexin V shift follows the incubation of cells with IgE and the antigen DNP (+IgE/+DNP). The percentages indicate the value of the shift.

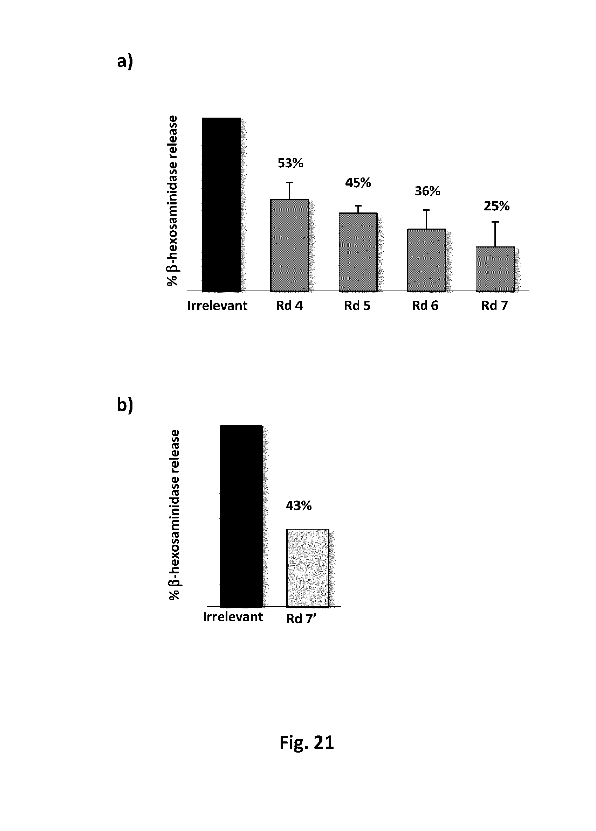

**Figure 21****: Measure of β-hexosaminidase release during phenotypic selection steps of intrabody retroviral library.**
   a) selection including cloning steps
   b) selection without cloning steps
**Figure 22****: Measure of β-hexosaminidase release of RBL-2H3 clones resulting from the intrabody retroviral library.**

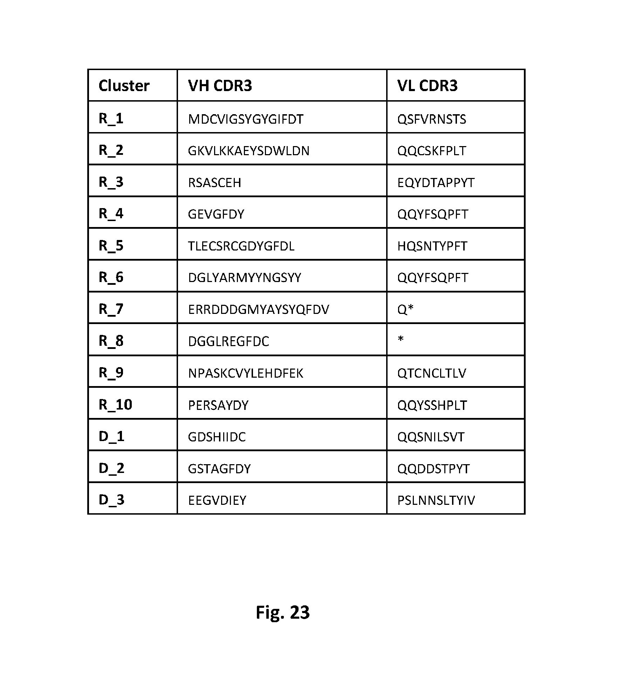

**Figure 23****: Aminoacid sequences of the CDR3 loops of one representative scFv gene from each cluster.**

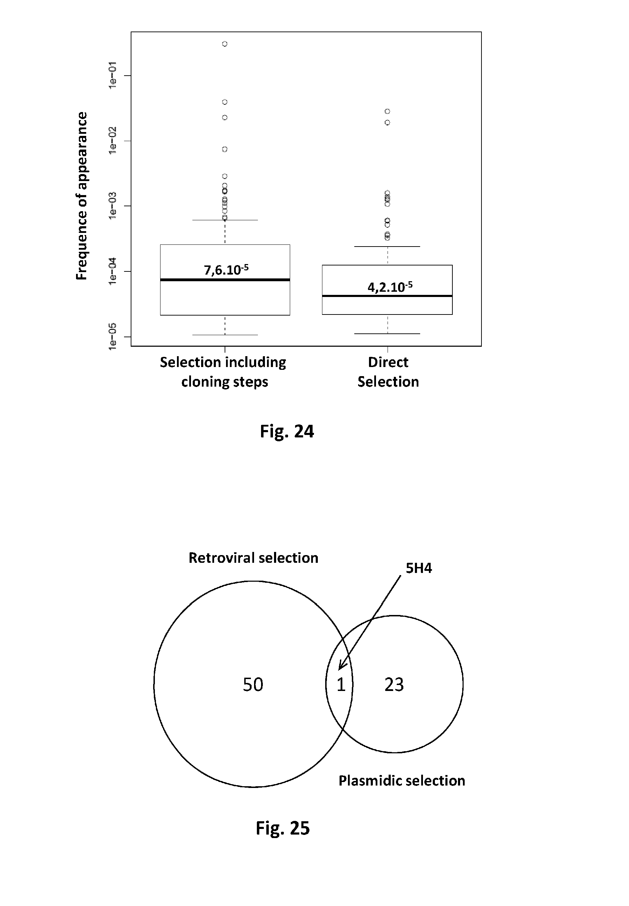

**Figure 24****: High-throughput sequencing and comparison of the sequences of enriched VH genes.**
**Figure 25****: Venn Diagram of VH sequences resulting from retroviral and plasmidic selections.**
**Figure 26****: Annexin V staining comparison of cell populations resulting from different phenotypic selections.**
   The arrow indicates the non stimulated cell population (+IgE/-DNP); FcεRI stimulation-dependent Annexin V shift follows the incubation of cells with IgE and the antigen DNP (+IgE/+DNP).

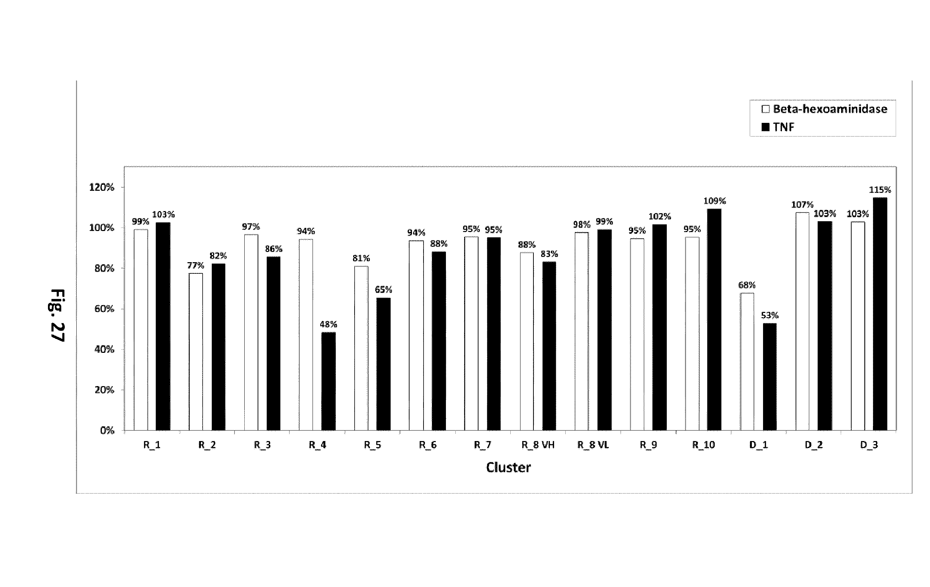

**Figure 27****: FcεRI-mediated mast cell degranulation was evaluated by the measure of the β-hexosaminidase release and by the measure of TNF-α secretion.**
   One representative scFv gene from each cluster was transfected in the RBL-2H3 cell line and the cellular phenotype was studied. The indicated percentage is calculated in comparison to the results obtained with an irrelevant scFv fragment.
**Figure 28****: Alignment of intrabody sequences 13R4, 3H2-1, 3H2-VH, 5H4, 5H4-VH, and 5H4-VL.**

### Examples

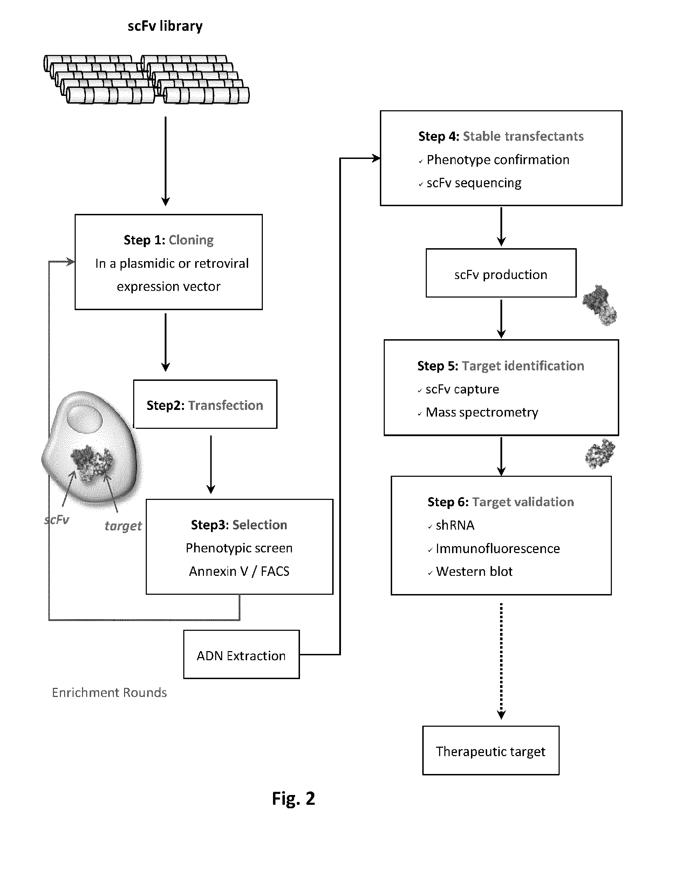

The phenotypic screening approach developed by the inventors is based on the selection, from a combinatorial library, of scFvs capable of inducing a phenotype of therapeutic interest. A phenotype associated with allergy was studied, using a cellular model of mast cell degranulation. The steps of the functional screening developed by the inventors is described in figure 2. First, a combinatory scFv library of high diversity and optimised for intracellular expression was cloned into a plasmid expression vector and into a retroviral expression vector. A mast cell line was transfected (step 2), followed by a phenotypic selection. Cells presenting an inhibition of degranulation and a *priori* expressing scFvs which interfere with proteins involved in the signalling pathway in question were sorted (step 3). The scFv genes expressed by these cells were used to transfect again the same mast cell line and perform new selection rounds. The sequences of the expressed scFvs in the cells sorted in the final selection round were used to generate stable transfectants (step 4). This allowed an individual analysis of the inhibitory potential of each scFv. The potential target of the scFvs of interest was identified by mass spectrometry (step 5). Finally, the last step consisted in the study of the involvement of these proteins in the signalling pathways associated with the activation of mast cells (step 6).
Two parallel approaches were developed, using either a plasmid expression system, or a retroviral system. This generic approach may be used for the study of multiple cellular phenotypes.

### Example 1. Phenotypic screen using a plasmid expression system

### Step 1: Cloning of the scFv library

The present strategy uses a combinatory library of human scFvs of high diversity (10⁹), allowing in theory the coverage of all the proteins expressed in cells (about 10⁶, when taking into account alternative splicing and posttranslational modifications). Moreover, this library was built using a constant framework allowing for a great stability in the cytosol, so as to optimise intracellular expression of the scFvs in the cytoplasm of eukaryote cells. The framework antibody is the 13R4 human scFv, having the sequence set forth in Figure 28. Epitopic diversity was introduced by PCR into the V_{H} and V_{L} CDR3s, and two "sub-libraries" were generated (Philibert et al., 2007). The introduction of epitopic diversity into the V_{H} and V_{L} CDR3s was made by mimicking the amino acid distribution in naturally-occurring human CDR3 loops, so as to generate human-derived scFvs, or in other terms scFvs as human as possible.

These two sub-libraries were assembled by PCR and cloned into the eukaryote expression vector pEF/myc/cyto (Persic et al., 1997). This vector comprises a strong promoter of the type EF1a, and is adapted to cytoplasmic expression of the scFvs. It also allows the inclusion of a C-terminal c-Myc tag (EQKLISEEDL), which is recognised by monoclonal antibody 9E10.

After bacterial transformation, a library having a diversity of 10⁹ was generated.

### Step 2: Transfection of the mast cell line

Mast cell line RBL-2H3 (a line derived from a subcloning of RBL, 'rat basophilic leukaemia') was used for this study. This line is commonly used in the field of allergy and its signalling pathways have been well studied.

These cells were transfected by electroporation with the previously cloned vector pEF/myc/cyto. The voltage, capacitance, and biochemical conditions relative to the electric shock were chosen such as to maintain as much as possible the initial diversity of the scFv library. The parameters to be considered therefor are:
- A manageable number of cells to be transfected,
- A survival rate post-transfection of 10 to 20%,
- A transfection efficiency evaluated at 80%,

In this manner, multicopy transfection conditions were attained, where each cell was transfected with 2000-2500 recombinant plasmids. Reverse transcription experiments, followed by qPCR (RT-qPCR) showed that in these conditions, the transcription of a single gene copy of one scFv among 2500 could be detected. Thus, in the first selection round, 3.10⁸ cells were electroporated with 3mg DNA. This allowed in theory for a representation of about 50 times the scFv library while taking into account the previously cited parameters.

Intracellular expression of the scFv library was controlled by performing an RT-qPCR analysis of the sequences expressed by the cells obtained after each selection round (see Fig. 2). This analysis showed that scFv expression, in terms of amplified cDNA per plasmid copy, increases with each selection round (figure 3a). These results are correlated by the observations on cell immunofluorescence (IF) of the transfected cells. Cell marking with the 9E10 antibody coupled to Alexa 488, revealed a rather diffused cytosolic expression, with the presence of a few scFv insoluble aggregates, the latter diminishing during the selection (figure 3b).

### Step 3: Phenotypic selection

For each selection round, transfected cells were stimulated via the FcεRI (high affinity IgE Receptor) by addition of IgE coupled to its Ag, dinitrophenyl (DNP). Immediately after the stimulus, the cells were labelled with Annexin V coupled to APC (allophycocyanin). This marker specifically binds to the phosphatidylserines exposed at the plasma membrane during exocytosis proportionally to the intensity of degranulation (Demo et al. 1999). The fluorescent Annexin V allowed a distinction to be made with a flow cytometer between cells which degranulate (strongly marked) and cells whose activation is inhibited (faintly marked). Marking with Propidium iodide (PI) allowed the exclusion of apoptotic cells. When sorting by FACS, a population of cells transfected with an empty vector was used as control.

A region corresponding to 0-10% of the least degranulating cells which a priori express the inhibiting scFvs was sorted. After each selection, the plasmid DNA of the sorted cells was extracted and the sequences of the scFvs were amplified by PCR and recloned into the same expression vector in order to transfect RBL-2H3 cells and carry out a new selection round. 8 enrichment rounds were thus performed. The analysis of the Annexin shift along selection revealed a marked decrease of cell degranulation (figure 4). Indeed, in the first round, stimulation with IgE/DNP induces a level of Annexin marking of about 52% of the total population; and this percentage decreases to 19% at the 7^{th} and 8^{th} rounds of selection.

### Step 4: Generation and analysis of the stable transfectants

After selection, the DNA extracted from the cells was used to generate stable transfectants of rat mast cell line RBL-2H3. The stable transfectants were obtained by electroporation of the extracted DNA, followed by a selection with the antibiotic G418 (Geneticine) at the final concentration of 2 mg/mL. The stable cell transfectant clones were obtained by limited dilutions. 132 stable clones were isolated and their phenotype associated with activation of the FcεRI was analysed by the measurement of β-hexosaminidase release, which is one of the preformed allergic mediators released by exocytosis.

### - Phenotype of the stable clones

The cellular test was performed in duplicate. Figure 5 presents a representative sample of all the clones tested. RBL-2H3 cells were used as a control of degranulation, to which the different clones were compared in terms of percentage of enzyme release. The results show that 53 clones (42%) present an inhibition of degranulation, among which 10 (8%) present a level of inhibition of at least 50%. After the first screen, 34 clones were selected and analysed using a second cellular test consisting in the measurement of the intracellular calcium flux. The calcium flux is triggered a few seconds after activation of the FcεRI by the release of intracellular stocks of Ca²⁺, followed by a calcium influx. Cell marking was performed with a calcium sensor, Fluo 3-AM, whose fluorescence detected by FACS, is proportional to the amount of intracellular Ca²⁺. Figure 6 shows the measurements of calcium flux corresponding to a selection of 10 clones. In correlation with the results obtained by the measurement of β-hexosaminidase, the majority of the analysed clones presented an inhibition of the calcium flux.

### - Sequence analysis

The genomic DNA of 34 clones presenting an inhibition of β-hexosaminidase was extracted, and the genes of the scFv expressed by these cells were amplified by PCR, and sequenced. This analysis revealed that 65% of the cellular clones presented a mixture of 2-3 sequences. In addition, 74% of the scFvs from the phenotypic selection presented complete sequences. However, 14% of the analysed were truncated at the CDR3 of the V_{L} (L3), 3% at the CDR3 of the V_{H} (H3), and 9% had neither H3 nor L3.

Two clones, 3H2 and 5H4, presenting pronounced and reproducible degranulation inhibition phenotypes, were retained for further study (Fig. 7a). Initially, a marking of the membrane FcεRI of clones 3H2 and 5H4 was performed in order to verify that the inhibitory phenotype which was obtained was not due to a defect in the expression of the IgE receptors. These analyses showed a marking similar to that of non transfected RBL-2H3 cells.

### - Clone 3H2

The three cellular tests allowing the evaluation of the degranulation of this clone were performed in parallel, and showed that β-hexosaminidase release was inhibited by 59% (figure 8), as well as the Annexin V-Fitc marking (figure 9). However, the intracellular flux of this clone was weakly affected (figure 10). The sequence analysis of the scFv expressed by clone 3H2 showed two sequences corresponding to a full scFv named 3H2-1 and to a truncated gene containing only a VH domain and named 3H2-VH (figures 7, 28). An alignment of the amino acid sequences of scFv 3H2-1, 3H2-VH and scFv 13R4 which served as the original backbone scFv for the building of the library, confirmed that only the V_{H} and V_{L} CDR3 regions presented variability.

New stable transfectants of the RBL-2H3 line were generated and clones expressing scFv 3H2-1 and 3H2-VH were analyzed. Three cellular tests allowing the evaluation of the degranulation of these clones were performed, and showed that β-hexosaminidase release was strongly inhibited in cells expressing 3H2-VH (figure 8), while Annexin V-Fitc marking (figure 9) and the intracellular flux were inhibited (figure 10) in stable RBL-2H3 transfectants expressing 3H2-1 and 3H2-VH.

### - Clone 5H4

The analysis of the sequence of the scFv expressed by this clone revealed the presence of a stop codon at the beginning of the L3 (figures 7, 28). The corresponding truncated gene was devoid of its C-terminal end, as well as of the Myc tag. The variability of the original library being only carried by the CDR3s, it was assumed that the V_{H} domain alone could be responsible for the specificity of 5H4 for its target and its inhibitory activity, especially since the literature reports the efficiency of such formats for targeting proteins (Stijlemans et al., 2004; Tanaka et al., 2007).

Therefore, two 5H4 formats were recloned into the pEF/myc/cyto vector: 5H4-VL corresponding to the sequence of the original clone; and 5H4-VH which comprises only the VH domain (figure 28). The C-terminal Myc tag was reintroduced in the sequences.

New stable transfectants of the RBL-2H3 line were generated. The cellular tests for these populations revealed that the stimulation of the FcεRI resulted in a strong reduction of calcium mobilisation (figure 10); an inhibition of β-hexosaminidase release (figure 8); and a low marking by Annexin V-APC (figure 9).These results show that the 5H4-VH format generates a phenotype similar to 5H4-VL.

### Step 5: Production of selected scFvs and identification of their target

### - Production of the scFvs

So as to use the selected Ab fragments for identifying their target, the three sequences 3H2-1, 5H4-VH and 5H4-VL, were cloned into two prokaryote expression vectors allowing their production from E. *Coli* cytoplasmic (pET23 vector) or periplasmic (pHEN2 vector) extracts.

They were also cloned into a eukaryote expression vector (ps1119) allowing their production fused with a mouse Fc, in the form of dimers (Moutel et al. 2009).

### - Target Identification

The three Ab fragments, as well as the appropriate irrelevant Abs, were purified and immobilised on magnetic nickel beads thanks to their 6xHis tag. They were used to immunoprecipitate protein lysates from RBL-2H3 cells stimulated or not beforehand via the FcεRI. After the immunoprecipitations, the beads were washed in low stringency conditions in order to preserve low affinity bindings.

After electrophoresis on acrylamide gel (SDS-PAGE), and staining with Coomassie blue, bands present when immunoprecipitated by Ab fragments 3H2-1 and 5H4 and whose intensity was either higher as compared to the irrelevant Ab, or who were absent with the irrelevant Ab, were cut. After digestion with trypsin, the bands were analysed by mass spectrometry MS/MS using the MALDI-TOF (matrix-assisted laser desorption/ionization time of flight) technique.

The Mass spectrometer which was used is composed of a laser which ionises previously digested peptides; an analyser which measures the mass/charge (m/z) ratios of the ionised peptides as a function of their flight time; and a detector which records the number of ions for each given m/z ratio. The obtained spectra of m/z ratios allow the identification of peptides and thus proteins present in the samples, by interrogating the *Genebank* rat database, as well as the mouse database for comparison (the mice *Genebank* data being more complete). A confidence index or *ProtScore* is attributed for each protein as a function of the number of identified peptides which are part of its sequence and of the nature of these peptides (composition, size). A *ProtScore* higher than 3 corresponds to 0,1% probability of identifying a false positive, in other words the probability that this protein is present is 99,9%. The proteins with *Protscores* the highest for the tested Ab fragments and absent or insignificant for the irrelevant Ab were kept.

In the case of scFv 3H2-1, 10 proteins were selected at the end of this analysis, with *Protscores* ranging from 4 to 51 against 0 to 2 for the control. 95kDa protein ABCF1 (*ATP binding cassette sub-family F member 1*) which had the highest *Protscore* was retained (figure 11a and Table 2).

**Table 2**

| Protscore IP | | Accession gi* | Name | Da |
|---|---|---|---|---|
| 3H2-1 | irrelevant | | | |
| 51 | 2 | 158081775 | ATP-binding cassette sub-family F member 1 | 95252 |
| 10 | 0 | 71043774 | guanine nucleotide binding protein-like 2 | 83869 |
| 10 | 0 | 194294493 | lysine (K)-specific demethylase 1 | 94520 |
| 10 | 2 | 55741637 | lysyl-tRNA synthetase | 71623 |
| 10 | 0 | 149024626 | rCG30986, isoform CRA_a | 100718 |
| 7 | 2 | 199560247 | CTP synthase | 66640 |
| 7 | 0 | 75516369 | Eif3c protein | 82545 |
| 4 | 0 | 189491616 | metastasis associated 1 family, member 2 | 74960 |
| 4 | 0 | 30794228 | fragile X mental retardation 1 | 65631 |
| 4 | 0 | 21693591 | CCCH-type zinc finger antiviral protein | 86770 |

In the Table, the columns are the following: Column 1, Protscore of the identified proteins using the intrabody. Column 2, Protscore of the identified proteins using an irrelevant intrabody. Column 3, Genbank gi number of the identified protein. Column 4, name of the protein in Genbank. Column 5, predicted molecular weight.

This protein is a particular member of the ABC transporters family because it is the only one which does not comprise a transmembrane domain. Several studies indicate that it could have a role in translation initiation by interacting with the elF2 factor (*eucaryotic initiation factor* 2) (Paytubi et al., 2009, 2008; Tyzack et al., 2000). Previous work reported that the gene encoding ABCF1 was regulated by TNF-α in synoviocytes. In this context, this protein may take part in the increase of protein synthesis and in the inflammatory process (Richard et al., 1998). In addition, the locus of the gene coding for ABCF1, located in the HLA region (*human leukocyte antigen*), may be associated with a predisposition for an autoimmune disease (Ota et al., 2007).

In the case of 5H4, the comparison of the results obtained for 5H4-VH and 5H4-VL showed that the proteins immunoprecipitated by the two formats were the same. Therefore, only 5H4-VH was used in the further experiments. The MS/MS results allowed the selection of 8 proteins with *Protscores* ranging from 4 to 22 versus 0 to 2 in the control. The 64kDa protein LOC297607 (hereafter "LOC"), with a *ProtScore* of 16 to 22 for three independent experiments was retained (figure 11 b and Table 3).

**Table 3**

| Protscore IP | | Accession gi* | Name | Da |
|---|---|---|---|---|
| 5H4-VH | irrelevant | | | |
| 22/18/16 | 0/0/2 | 157820037 | hypothetical protein LOC297607 | 63619 |
| 8/20 | 0/0 | 109500976 | PREDICTED : similar to myosin IG | 126567 |
| 15/6 | 0/2 | 157823677 | adaptor-related protein complex 2, alpha 1 subunit | 107675 |
| 8/10 | 0/0 | 157822743 | kinesin family member 20A | 100016 |
| 8/5 | 0/0 | 9507177 | USO1 homolog, vesicle docking protein | 107225 |
| 8/5 | 0/0 | 149033810 | vesicle docking protein | 107162 |
| 4/4 | 0/0 | 157823309 | ATP-binding cassette, sub-family E, member 1 | 67300 |
| 4/4 | 0/0 | 109492380 | similar to Actin, cytoplasmic 2 (Gamma-actin) | 58801 |

In the Table, the columns are the following: Column 1, Protscore of the identified proteins using the intrabody. When several experiments have been performed, the 3 values separated by a "/" are reported. Column 2, Protscore of the identified proteins using an irrelevant intrabody. Column 3, Genbank gi number of the identified protein. Column 4, name of the protein in Genbank. Column 5, predicted molecular weight.

This protein whose function was unknown until now is found in higher eukaryotes, and is ubiquitously expressed. Its sequence, identified using cDNA libraries, is highly conserved among species (figure 12).

### Step 6: Validation of the protein targets

### - 3H2-1 potential target: ABCF1

In order to confirm the protein identification, the IP (immunoprecipitation) experiment conducted for mass spectrometry was repeated and followed by an immunoblot revealed by a commercial Ab directed against the ABCF1 protein (figure 13a). This blot showed that the ABCF1 protein is specifically immunoprecipitated by scFv 3H2-1 because it was absent in the control IP.

An IF marking of RBL-2H3 cells with 3H2-1 in a dimerised form (3H2-Fc), as well as with the commercial Ab directed against ABCF1 was performed. These analyses revealed similar cytoplasmic markings (figure 13b).

### - 5H4 potential target: LOC297607

In the same manner as for the mass spectrometry analysis, an IP of the RBL-2H3 lysates was performed with 5H4-VH, and revealed by WB with a commercial Ab directed against the LOC protein (figure 14a). This blot revealed that 5H4-VH recognised the LOC protein in its native form.

This result was confirmed by a double marking by IF analysis with a confocal microscope. This experiment revealed a cytosolic marking of the LOC protein, and a colocalisation of signals corresponding to 5H4-VH and the commercial Ab directed against LOC (figure 14b).

### - 5H4 target: LOC297607, confirmation by shRNA

Inhibition of the LOC protein expression by shRNA (*short hairpin RNA),* followed by a study of the associated phenotype was performed in order to validate the implication of the LOC protein in mast cell activation by an independent approach. Therefore, two shRNA, sh1 (SEQ ID NO: 30) and sh2 (SEQ ID NO:31) directed against the LOC protein were constructed using the Dharmacon software (http://www.dharmacon.com/designcenter); as well as one control irrelevant shRNA: shLUC directed against luciferase. The corresponding sequences of the shRNA are presented in Figure 15.

The shRNA were cloned into the retroviral expression vector pSIREN to infect the RBL-2H3 line. A selection with puromycin allowed the recovery of only the infected cells expressing the different shRNA. Cellular tests detailed below were performed 5, 10 and 15 days from the beginning of the puromycin selection and on three independent series of shRNA infected cells.

In order to control the extinction of the expression of the protein, an RT-qPCR was first carried out on the RNA extracted from the different types of cells.

Figure 16a shows the results obtained with the infected cells, after normalisation with the housekeeping gene HPRT encoding hypoxanthine-guanine phosphoribosyltransferase. This experiment revealed that sh1 expression extinguishes 85% of the messenger of this protein at day 5, and from 66 to 73% at days 10 and 15 of selection. The extinction of the LOC messenger by sh2 showed similar results, ranging from 58 to 64% inhibition between days 5 and 15 of selection.

The extinction of the protein was also confirmed by WB. Protein extracts from cells expressing the different shRNA were revealed with the commercial Ab directed against LOC. After normalisation of charges between the tracks, the loss of intracellular LOC protein when sh1 was expressed was evaluated at 50-71% (depending upon the series) after 5 days, and up to 88% after 15 days of selection (figure 16b). The expression of sh2 was at the origin of a lower inhibition (about 30%).

Throughout the puromycin selection, cellular tests were performed to evaluate the impact of the shRNA on mast cell activation. The measurement of β-hexosaminidase revealed that after 5 days of selection, the cells expressing sh1 present a significant inhibition of 28% (figure 17a). The release of TNF-α, a neosynthesised mediator, was also inhibited by 41% at day 5 of selection (figure 17b). The intracellular calcium flux analyses showed also an important inhibition at day 5 (figure 17c).

Following mast cell activation, signal transduction is mediated by a balance between the activity of protein tyrosine kinases (PTK) and protein tyrosine phosphatases (PTP). This results from an increase in phosphorylation of a number of intracellular substrates. The protein extracts of cells non stimulated or stimulated via the FcεRI were analysed by WB with monoclonal Ab 4G10 directed against the P-Tyr. It was noted that a reduction in LOC expression affected phosphorylation of certain intracellular proteins migrating to around 130, 75, 50-60 kDa (figure 18). In order to have an indication as to the identity of these proteins, this blot was revealed with different antibodies directed against proteins of similar sizes and known to be involved in mast cell activation.
- The use of phospho-specific antibodies to key proteins involved in mast cell activation suggest that LOC intervenes in the signalling pathway of the Src family tyrosine kinase Fyn. Indeed, we have observed an inhibition of the phosphorylation of the protein Gab2 (GRB2-associated-binding protein 2), a substrate of Fyn and subsequently the phosphorylation of Akt (protein kinase B) and NF-κB (nuclear factor kappa-light-chain-enhancer of activated B cells) are affected.

These preliminary results confirm the involvement of the protein LOC in mast cell activation following FcεRI activation. Indeed, during the first week following infection by the shRNA, a partial extinction of the protein in the cell is at the origin of a phenotype of inhibition of the calcium flux and the release of preformed and neosynthesised allergic mediators (β-hexosaminidase and TNF-α).

The structure of the LOC protein being unknown, a search for conserved motifs was carried out, which revealed potential phosphorylation sites of tyrosine and serine residues. We also identified two adjacent «ITIM-like» motifs similar to the motifs that are present in inhibitory receptors of immune cells, and that are capable of recruiting potential partners via their SH2 domains (amino acids 319-342: SLYSTSLCGLVLLVDNRINSYSGI)

### Example 2. Phenotypic screen with a retroviral expression system

The phenotypic screen was adapted using a retroviral system for expression of the scFv library in the same mast cell line. The objective was to optimize this generic approach to apply it to the study of other phenotypes. The principle is the same as for the approach described in example 1, with the exception of selection: indeed, the viral approach a priori allows the avoidance of the prior recloning steps of the scFv library, and the use of the same population throughout the selection.

### Step 1: Cloning of the scFv library

The same scFv library as for the plasmid selection (Philibert et al., 2007), was cloned into the pMSCV-hygro-GFP vector. This vector confers on the one hand, hygromycin resistance when cells are infected, and on the other hand, allows the production of the scFv fused to the GFP protein. Infection efficiency can thus be evaluated, and intracellular scFv expression can also be monitored and controlled throughout the entire selection. After the cloning of the library, it was amplified by transformation of E. Coli. The putative initial diversity was 2.108 clones.

### Step 2: Infection of the RBL-2H3 line

The retroviral supernatants used were produced after co-transfection of 293T cells with genes coding for the scFv library, the capsid proteins, as well as for the amphotropic envelop proteins. This system allows the infection of the RBL-2H3 line with an efficiency of over 95%.

The number of copies per cell was checked by qPCR, and showed that each cell had between 1 and 3 scFv copies. In order to remain in manageable experimental conditions, 4.10⁷ RBL-2H3 cells were infected. Thus, under these conditions, the diversity of the putative initial scFv library was approximately 10⁸.

### Step 3: Phenotypic screen

4 days after infection, cells were activated with IgE/DNP, labelled with Annexin V-APC and Propidium Iodide and analyzed by FACS (Figure 19).

A population corresponding to 10% of the least fluorescent cells was then sorted and put directly into culture, so as to amplify the cells for the following selection a week later. Eight rounds of selection were performed in this manner.

Inhibition of the phenotype appeared in the 6^{th} round of selection. Indeed, the shift in Annexin V-APC between the non-stimulated and stimulated with IgE/DNP cell populations, went from 51 % in the first round to 16% in round 6, and 15% in round 7.

In order to control that the appearance of the phenotype came from the enrichment in inhibitory scFv and was not due to cell bias, such as a population drift, or an enrichment of scFv according to their genomic insertion site, a second selection of the retroviral library was performed including recloning steps.

Starting from the DNA of cells derived from selection round 3, the scFv sequences were amplified by PCR and cloned into the pMSCV new-hygro-GFP vector. After transformation into the virus, the infection was carried out on a new cell population. This step of recloning was repeated every 2 rounds of selection, in rounds 5' and 7'. The results of the marking with Annexin V-APC show that this method of selection also allowed the convergence towards an inhibitory phenotype, which demonstrates that it was not a phenotypic drift of the cell population used (Figure 20). Interestingly, the selection with recloning almost completely extinguished the phenotype, since the corresponding inhibition was over 90%.

The evolution of the degranulation phenotype of the cell populations derived from different rounds of selection was analysed. Aliquots of cells from different sorting steps were thawed and subcultured in order to achieve a measure of the release of β-hexosaminidase on entire populations. The results show a decrease of 75% of the degranulation of cells infected with the scFv library from round 7 (Figure 21 a), and 57% for cells from round 7' (Figure 21 b), as compared to control cells.

### Step 4: Generation and analysis of stable transfectants

### - Phenotype of the stable transfectants

An aliquot of cells from the retroviral selection including the recloning steps was seeded at limiting dilution on five 96-well plates. After a hygromycin selection, 50 cell clones were analysed in duplicate by measuring the release of β-hexosaminidase (Figure 22).

This analysis revealed that the degranulation of the 50 clones tested, was inhibited by 54% on average compared to 10 irrelevant clones.

### - High throughput sequencing

The evolution of diversity of the retroviral library during the two modes of phenotypic selection (with or without recloning steps) was analysed by high throughput sequencing (or NGS, "next generation sequencing") using Illumina technology.

The sequences of the analysed scFv were amplified from 10⁶ cells infected before the first screen (naive library), and from 5.10⁵ cells from each of rounds 3, 5 and 7, with and without recloning. Ultimately 33.10⁶ scFv were sequenced

From the 10⁶ cells infected with the naive library and analysed, approximately 10⁷ sequences of V_{H} were obtained, among which 4-5.10⁵ sequences were unique. This result suggests that the diversity of the library has not been affected significantly by retroviral infection. Indeed, the difference could be due to the fact that during the 3-4 days between the retroviral infection and the extraction of genomic DNA, cells would have had time to double, thus lowering the diversity by a factor of 2.

The sequencing revealed that over 99% of the read sequences all had H3 or L3 without frame shift or stop codon.

The translation of the unique nucleotide sequences generated 2-3.10⁵ unique V_{H} amino acid sequences and 3. 10⁵ V_{L} sequences. These results reflect the actual diversity of the library from the 10⁶ cells analyzed. This diversity potentially increases with the number of possible combinations between V_{H} and V_{L}.

These sequencing analyses also revealed that starting with the same number of analyzed cells, the number of unique sequences is comparable between the direct and recloned retroviral selections, which suggests that the cloning steps do not seem to affect the diversity of the library.

### - Statistic analysis

After the sequencing, statistical analysis of data was performed with the SAMSeq software (Significance analysis of microarrays) (Li and Tibshirani, 2011), which calculates a statistical score of enrichment for each sequence, and thus determines the subset of sequences significantly enriched during the selection. By setting a FDR (False Discovery Rate) of 0.05, 2500-10000 sequences appear to be enriched. The next step of the statistical analysis consisted in the comparison of the two selections in terms of enrichment of scFv sequences between different rounds, or Fold Change (FC) and frequency of occurrence, which corresponds to the presence of the same sequence in each library. The figures herein refer to the analysis of V_{H} sequences. Similar results were obtained with V_{L} libraries.

The sequences considered as enriched corresponded to sequences with an increase throughout the selections, and whose FC was greater than or equal to 100 between the naive library and round 7. In the end, 110 V_{H} sequences were enriched with the recloned selection, and 107 with the direct selection.

Comparison of these sequences with each other allowed their grouping into clusters or families, with at least 60% identity between them. 71 out of the 110 sequences from the recloned selection were regrouped into 14 clusters (each containing 2-25 sequences), and 54 out of the 107 sequences of the direct selection were grouped into 17 clusters (2-7 sequences per cluster). Among these 31 clusters, we conserved those present at least at 0,1% in the final enriched library after 7 rounds. Finally, 10 clusters from the recloned selection and 3 clusters from the direct selection were selected and further studied (Figure 23).

### - Comparison of retroviral selections

Comparison of the FC and frequency of the V_{H} sequences enriched after two selections revealed that:
- The recloned selection led to FC twice as high as with the direct selection: FC max = 1246 and 492 respectively;
- The median of the occurrence frequencies was also about twice as important in the recloned selection: frequency = 7,6.10⁻⁵ and 4,2.10⁻5 respectively (Figure 24);
- Finally, the 110 V_{H} from the recloned selection represent 40% of all sequences of the library after round 7, while the 107 V_{H} from the direct selection represent less than 10%.

In conclusion, in the light of this analysis, it appears that the recloned selection has a better enrichment than the direct selection, both in terms of frequency of occurrence and fold change. One hypothesis would be that without recloning, the selection could be biased by an enrichment in insertion sites favouring the appearance of the phenotype of interest. Thus the inclusion of recloning steps in the selection would optimise the enrichment in scFv inhibitors by limiting this bias.

### - Comparison of retroviral and plasmid selections

Finally, 178 scFv sequences corresponding to the plasmid selection (stable clones and sequences stitched at random) were included in a second analysis in order to compare the scFv sequences enriched using plasmid and retroviral selections, in clusters rather than individually.

It is interesting to note that there is only one sequence in common to both selections, and that it is the V_{H} domain of 5H4 (Figure 25). It is worth noting that the 5H4 cluster is part of the 10 best clusters selected in the recloned selection.

This sequence presented an enrichment of 150 times using the retroviral selection.

### - Validation of retroviral clones

The most frequent sequence of each of the 13 clusters was cloned in the pMSCV-hygro-GFP retroviral vector and the cellular phenotype of retrovirus-infected cell populations were analyzed. As shown in Figure 27, most of the clones showed an inhibition of degranulation following FcεRI stimulation. These results confirmed the presence of inhibitory antibody sequences in the last round of selection and that these antibody sequences were correctly identified by the statistical approach followed.

### Materials and Methods

### 1. Reagents and Antibodies

All antibodies used were obtained from Santa Cruz Biotechnology (Santa Cruz, CA, USA), with the exception of phospho-specific Antibodies which were obtained from Cell Signaling Technologies (Danvers, MA, USA). The anti-LOC (anti-C12orf4) antibody and all reagents were obtained from Sigma-Aldrich (St Louis, MO, USA).

### 2. Cell culture

Rat mast cell line RBL-2H3: RBL-2H3 cells (ATCC, Manassas, VA) were cultured in DMEM medium supplemented by 15% foetal calf serum (FCS) and antibiotics, at 37°C in a humid incubator with 5% CO2. Adherent cells were passaged 3 times a week. The cells were detached by trypsin treatment during 5 minutes at 37°C, and subsequently inactivated by addition of two volumes of cell culture medium.

Line 293T (or HEK-T): These cells were maintained in culture in DMEM medium supplemented with 10% FCS and antibiotics, at 37°C in a humid incubator with 5% CO2.

Hybridoma. The murine monoclonal antibody producing hybridoma were maintained in culture in DMEM medium supplemented with 10% FCS and antibiotics, at 37°C in a humid incubator with 5% CO2. The culture supernatants containing the antibodies were filtered and preserved at 20°C. The antibody concentration was determined by an ELISA technique.

### 3. Phenotypic selection

### 3.1. Cloning of the scFv library

The eukaryote cytoplasmic expression vector pEF/myc/cyto (Invitrogen) was used to express the scFv library in RBL-2H3 cells. This vector comprised a promoter EF1a, followed by a Ncol site containing the initiatory ATG. The cloning was carried out between the Ncol and Notl sites, the latter being followed a c-Myc epitope detected by the 9E10 monoclonal antibody. In order to clone the scFv library into this vector, *VHpool and VLpool* sub-libraries, which are the source of the diversity of the CDR3 loops (P. Philibert et al., 2007), were assembled by PCR using the *Pfu* ADN-polymerase (Promega, Madison, WI, USA) and the M13rev-49 and scFvCAT2.rev primers (35 cycles, 55°C). Following purification, the PCR was digested with Ncol (Fermentas, Thermo Fisher Scientific, Waltham, MA, USA) and *Not*l (New England Biolabs, Ipswich, MA, U.S.) enzymes, and then purified and quantified on gel. The pEF/myc/cyto vector was also digested with *Nco*l et *Not*l enzymes, and subsequently purified and quantified on gel. The linearised vector was used for the equimolar ligation with the *T4 DNA ligase* insert. The ligation was inactivated 20 minutes at 65°C and purified. It was used to electroporate C-Max5αF' competent bacteria (Sidhu et al., 2000), which were subsequently plated on 600cm² square dishes of LB containing 100µg/ml ampicillin. After 16h incubation at 37°C, the bacteria were recovered with a rake in LB containing 10% glycerol and preserved at -80°C in aliquots. An aliquot corresponding to 40 times the diversity of the library was used to prepare DNA with the Nucleobond Xtra Maxi kit (Macherey Nagel). This DNA corresponds to the library used subsequently for the transfection of the RBL-2H3 line. Ampicillin resistant bacteria were counted in order to estimate the initial diversity of the scFv library. In these conditions, the estimated size of the library cloned in the pEF vector was of 10⁹ clones. For expression of the scFv library by retroviral infection, the pMSCV-hygro-GFP vector (Clontech) comprising the packaging sequence ψ was used. The cloning was performed between the Sfi et Notl sites. For this cloning, the estimated size of the library cloned into the pMSCV vector was of 2.10⁸ clones. For the recloning steps during the phenotypic selection, the scFv sequences were amplified from genomic DNA of the sorted cells, by PCR with pMSCV.for and EGFP-N.back primers, using Taq Phusion. The insert corresponding to the library was digested by Sfi and Notl enzymes, purified on gel, and cloned again into the pMSCV-hygro-GFP vector by ligation. As previously, the plasmid DNA was amplified by CMax5αF' bacterial culture, and purified by kit.

### 3.2. Plasmid transfection of the scFv library

The trypsinised RBL-2H3 cells were washed once by centrifugation with culture medium, and subsequently resuspended at 10⁷ cells/ml in culture medium supplemented with 1mM sodium pyruvate (Invitrogen). 500µl of this cell suspension was mixed with 50µg plasmid and transferred in a 4mm electroporation cuvette (BioRad, Hercules, CA, U.S.). The cuvette was placed on ice for 5 min and subsequently the electroporation was performed with Gene Pulser I (BioRad) at 310V and 960µF capacitance. After the *pulse,* the cell suspension was placed in a culture flask containing culture medium pre-heated in an incubator with 5% CO2. After 24h, the culture medium was changed. In the first selection round, 3.10⁸ cells were electroporated, and then only 10⁷ cells for each subsequent round. The selection medium of the stable des transfectants contained culture medium complemented by the addition of geneticin (G418, Gibco) at a final concentration of 1 mg/ml the first week following transfection, and at 2mg/ml subsequently.

### 3.3. Retroviral infection of the scFv library

Initially, the retroviral supernatants were produced by 293T cells. One day after plating, a transient double transfection was performed with the cationic agent JetPRIME^{®} (Polyplus, Illkirch, France), with the DNA of interest, as well as the DNA coding for the amphotropic envelop gene (vsv-g) and the gag/pol genes. 48h later, the culture supernatant were collected, filtered, and used for the infection of RBL-2H3 cells. Therefore, the cells were plated in the morning and infected with 1/3 volume of retroviral supernatant, 2/3 volume culture medium and 8µg/ml polybrene. 48h post-infection, the culture medium was replaced by fresh medium supplemented with selection agent. The expression of the transgene can be detected from days 4-5 post-infection. The stable transfectants were selected by addition of 1mg/ml hygromycin B (Invitrogen) to the culture medium.

### 3.4. Cell activation, Annexin labelling and Cell sorting

The cells were incubated one night at 37°C with anti-DNP (dinitrophenyl) IgE hybridome supernatant at a final IgE concentration of 0,5µg/ml. On the activation day, the cells were washed once with RPMI and once with Tyrode buffer (10mM HEPES pH 7.4, 130mM NaCl, 5mM KCI, 1mM CaCl2, 1mM MgCl2, 5.6mM glucose, et 0.01% BSA). The cells were subsequently activated in the Tyrode buffer with 100ng/ml DNP-KLH *(keyhole limpet hemocyanin conjugated dinitrophenyl,* Sigma-Aldrich, St Louis, MO, USA) at 37°C, in the dark, for 45 minutes. The activation was stopped by placing the cells on ice or by centrifugation at 4°C. Once the cells were stimulated, they were immediately marked with Annexin V-APC (Becton Dickinson Biosciences, San Jose, CA, USA) as follow: 100µl Annexin V-APC were added to 2.10⁶ cells (contained in 500µl), placed 30 min on ice in the dark glace. The cells were then marked with 20µg/ml Propidium Iodide 3 minutes prior to FACS analysis. The analysis and the sorting by flow cytometry were performed immediately after the marking, by means of a FACS Aria cell sorter (BD) on the Montpellier Rio Imaging platform.

### 3.5. DNA extraction

All the oligonucleotides were synthesised and purified by HPLC by MWG (Ebersberg, Allemagne). The plasmid DNA extraction and purification, PCR and ligation kits, were from Macherey Nagel (Duren, Germany). The genomic DNA and RNA extraction were performed with kits made by Qiagen (Germantown, MD, USA).

### 4. Analysis of the stable transfectants

### 4.1. Generation of stable clones

RBL-2H3 cells were transfected as described above and seeded in limiting dilution on 96 wells culture plates at 1 cell every 5 wells. The transfectants were selected 2 days after transfection by adding the antibiotic corresponding to the resistance provided by the plasmid which was used.

### 4.2. Measurement of β-hexosaminidase release

The day before the experiment, 10⁵ cells per well were seeded on 96-well culture plates. After 24 hours, adherent cells were activated by the addition of anti-DNP IgE overnight, and then by 50 to 200ng/ml DNP-KLH for 45 minutes at 37°C, as previously described. After collecting the supernatant of each well (S1), cells were lysed with lysis buffer (Tyrode buffer, 0.5% Triton, 50µg/ml aprotinin, 50µg/ml leupeptin, 50µg/ml pepstatin, 2 mM PMSF) for 20 minutes on ice. The plate was then centrifuged for 5 minutes at 2000 rpm and the supernatants corresponding to the cell lysates (S2) were harvested. The β-hexosaminidase dosage was carried out on 20µl of each S1 and S2 supernatants, by addition of 50µl of the β-hexosaminidase substrate (p-dinitrophenyl-N-acetyl-β-D-glucosaminidase, SIGMA) at a final concentration of 1.3 mg/ml for 1h30 at 37°C. The β-hexosaminidase substrate is freshly prepared or stored at -20°C in a solution of 0.1 M citric acid, pH4.5. The reaction was stopped by addition of 75µl per well of 0.4 M glycine, pH10.7, and the intensity of staining was evaluated by measuring the optical density at 405 nm. The percentage of β-hexosaminidase release was calculated according to the ratio: S1/(S1 + S2) x100.

### 4.3. Annexin V-Fitc marking

Annexin V coupled to Fitc (25µg/ml stock, Assay Designs, Ann Arbor, MI) was used for analyses of degranulation. 10µl of Annexin V-Fitc were added in 10⁶ cells (contained in 1 ml), placed 30 min on ice away from light. Then, in order to distinguish degranulating cell populations from cells undergoing apoptosis, Propidium Iodide at a final concentration of 20µg/ml was added to cells 3 minutes before analysis by flow cytometry.

### 4.4. Measurement of Calcium flux

To measure the intracellular calcium flux, 10⁶ cells were stimulated in suspension by the addition of anti-DNP IgE for 2-3 hours at 37°C with stirring (60-70rpm). After washing in RPMI (centrifugation for 5 minutes at 1000rpm), cells were labelled with Fluo 3-AM (Invitrogen) at a final concentration of 5µM for 30 minutes at room temperature and away from light. After marking, washing in RPMI was realized, and then the cells were resuspended at 10⁶ cells/ml, and kept on ice. The marking is stable 1h. Prior to their activation by addition of DNP and their sorting by flow cytometer, the cells were warmed 10 minutes at 37°C. Two aliquots of cells were used for the analysis of calcium flux in FL1 in function of time. A first tube of cells allowed setting the cytometer to exclude debris and establish the basal fluorescence of the cells. Then a second batch of cells was activated by the addition of DNP-KLH at a final concentration of 200ng/ml. The intracellular Ca²⁺ flow increases within seconds, and the analysis is performed over 2-3 minutes. Subsequently, analysis of the mean fluorescence of the population is carried out on Excel.

### 4.5. Measurement of TNF-α release

The day before the experiment, 8.10⁵ cells per well were seeded on 12-well culture plates. The next day, after washing with RPMI and washing with Tyrode, cells are activated as described earlier, with 200µl per well of 50ng/ml DNP-KLH for 2h at 37°C. The supernatants were harvested and used for the quantification of released TNF-α, using an ELISA kit (BD). The cell monolayer was washed once with cold PBS and lysed in PBS supplemented with 0.1% Triton and protease inhibitors (Complete Mini EDTA free tablet, Roche) for 15 minutes at 4°C. The amount of cellular protein was measured using BC Assay kit (Uptima) to normalize the results.

### 4.6. High throughput sequencing

The genomic DNA of 10⁶ cells for the naive library, or 5.10⁶ cells for the following libraries, was extracted and amplified by PCR with primers bordering the V_{H} and V_{L} scFv domains. Then the DNA to be analysed were prepared according to sequencing procedures.

The analysis was performed on the MGX-Montpellier sequencing platform.

The data were analyzed using the SAM software.

### 5. Target identification and validation

### 5.1. Production and purification of Ab fragments

### - Production of scFv fused to a mouse Fc

The antibody fragments were previously cloned into the vector ps1119 allowing the expression of scFv fused to a mouse Fc (mufc), of the type IgG1, by insertion at the BgIII and EcoRI sites. 293T cells were transiently transfected with JetPEI^{®} (Polyplus), with DNA encoding the Ac fragments, and the vector ps1119. 6 h after transfection, the culture medium was replaced by OPTIMEM (Gibco). Culture supernatants enriched in scFv in the muFc format, were harvested after 6 days, filtered on 0.2□m and stored at -80°C.

### - Production of antibody fragments from bacteria cytoplasmic extracts

The antibody fragments were cloned into the vector pET23NN (modified vector from Novagen, allowing the expression of a Myc tag and a 6xHis tag at the C-terminus of the scFv product) between the Ncol and Notl sites. The protocol followed for the expression of scFv in the cytoplasm of E. Coli has been described by the inventor's team (Guglielmi and Pierre Martineau, 2009).

### - Production of antibody fragments from bacteria periplasmic extracts

The antibody fragments were inserted into the vector pHEN2 at sites Ncol and Notl. HB2151 bacteria were thus transformed. After 16 hours of pre-culture at 16°C in 2xTY containing 100µg/ml ampicillin and 1% glucose, the growth of bacteria was relaunched at 37°C. At an OD 600nm of 0.8, induction was triggered by the addition of IPTG at a final concentration of 1 mM for 3-4h at 30°C. The bacteria were then centrifuged for 20 minutes at 3500rpm and the pellets were lysed 15 minutes on ice in buffer: 30 mM Tris, pH 8, 20% sucrose, 1 mM EDTA, 1 mM PMSF. After centrifugation, 5 mM MgCl₂ and MgSO₄ were added to the supernatant periplasm. The extracts were then stored at -20°C.

### - Purification on Nickel resin

The antibody fragments were purified on nickel NTA resin from Qiagen. The procedure of the corresponding Ni-NTA spin kit was followed.

### - Purification on Nickel magnetic beads

For each immunoprecipitation the scFv were purified from bacterial cytoplasmic extracts corresponding to 50 ml of culture, with 20µl of magnetic nickel beads (Ademtech, Pessac, France), according to the kit specification.

### 5.2. Production and immunoprecipitation of cell protein lysates

The cell layer was washed twice in RPMI. The activated cells were stimulated with 50-100ng/ml DNP-KLH in RPMI for 3-10 minutes at 37°C in the dark. After washing with cold PBS containing phosphatase inhibitors (100 mM NaF, 5 mM orthovanadate), cells were lysed for 15 minutes on ice with lysis buffer containing PBS supplemented with: 0.5% sodium deoxycholate, 1% NP-40, 0.1% SDS. After scratching the dishes with a rake, the cell lysates were clarified by centrifugation for 15 minutes at 4°C at 13000rpm. An aliquot of the supernatant containing the protein extracts was assayed using a BC Assay kit (Uptima), the remaining lysate was supplemented with loading buffer (2% SDS, 10% glycerol, 2.5% β-mercaptoethanol, 0.01% bromophenol blue, 30 mM Tris pH6.8).

For immunoprecipitation, 3mg of protein lysates were used for immunoprecipitation with scFv previously purified from bacteria cytoplasmic extracts on magnetic nickel beads for 2h at 4°C. Before elution, three 10 minutes washes were performed with the previously described lysis buffer supplemented with 10mM imidazole. The elution was performed by adding 500 mM imidazole, the eluate was then complemented with loading buffer and loaded on gel for electrophoresis by SDS-PAGE.

### 5.3. SDS-PAGE and MS/MS analysis

The immunoprecipitated proteins were separated by electrophoresis on 10% acrylamide gel. After staining with Coomassie Blue Brilliant Blue, bands of interest were excised, discoloured, reduced, alkylated and digested with trypsin treatment as described by previous work (Shevchenko et al., 2007). The method used for the mass spectrometry analysis was the nano reversed-phase LC-MALDI MS/MS. The data were then analyzed using the software ProteinPilot. The MS/MS analysis was performed on the Proteomics Imaging and Molecular Interaction platform of Montpellier, France.

### 5.4. Western blot

For these analyses, proteins were transferred on a 0.2µm nitrocellulose membrane. Before each hybridization, the membrane was blocked in 5% BSA in TBS-T buffer (10 mM Tris pH 7.4, 150 mM NaCl, and 0.1% Tween) for 1h at room temperature. After hybridization with specific and secondary antibodies coupled to peroxidase (HRP), the proteins were visualized thanks to the ECL-Plus chemiluminescent substrate (PerkinElmer, Waltham, MA, United States) and using the chemiluminescence camera G-Box (Syngene, Cambridge, UK). Quantification of the intensities of the signals detected was evaluated using the software supplied by the same manufacturer.

### 5.5. Immunofluorescence

The cells were seeded on glass slides in Labtek chambers. All stages of these experiments were performed at room temperature. After two washes in PBS, cells were fixed with 3.7% formaldehyde (Sigma) for 10 minutes, and then permeabilised with PBS containing 0.05% saponin and 0.2% BSA for 10 minutes. The antibodies were then incubated 1-2h. After washing, the slides were mounted in Mowiol and observed after 3-4h.

### 5.6. shRNA

The sequences of the shRNA used (list in Figure 15) were cloned into the vector pSIREN (Clontech) by ligation. The DNA was produced after transformation of thermocompetent bacteria and purified by using a kit.

Retroviral supernatants were produced as described previously after double transfection of 293T cells with pSIREN vector containing the sequences of the different shRNA and viral DNA. The infected RBL-2H3 cells were selected by addition of 2,5g/ml of puromycin (HyClone, ThermoScientific) to the culture medium two days after retroviral infection.

### 5.7. RT-qPCR

Total RNA was extracted and purified from 10⁵-10⁶ cells using the Qiagen kit. 1µg of RNA was reverse transcribed with 100ng of random primer, with the M-MuLV Reverse Transcriptase (Invitrogen). Subsequently the qPCR was performed on the cDNA thus obtained by using the SYBR Green I Master mix, and detected on a Light Cycler 480 (Roche). The data were analysed using the software supplied by the same manufacturer.

### References

**○** Dauvillier S, Merida P, Visintin M, Cattaneo A, Bonnerot C, Dariavach P (2002). Intracellular Single-Chain Variable Fragments Directed to the Src Homology 2 Domains of Syk Partially Inhibit FcεRI Signaling in the RBL-2H3 Cell Line. The Journal of Immunology, 169, 2274 -2283.
**○** Demo SD, Masuda E, Rossi AB, Throndset BT, Gerard AL, Chan EH, Armstrong RJ, Fox BP, Lorens JB, Payan DG, Scheller RH, Fisher JM (1999). Quantitative measurement of mast cell degranulation using a novel flow cytometric annexin-V binding assay. Cytometry, 36(4):340-8.
**○** Genini S, Nguyen TT, Malek M, Talbot R, Gebert S, Rohrer G, Nonneman D, Stranzinger G, Vögeli P (2006) Radiation hybrid mapping of 18 positional and physiological candidate genes for arthrogryposis multiplex congenita on porcine chromosome 5. Anim Genet., 37(3):239-44.
**○** Guglielmi L and Martineau P (2009). Expression of Single-Chain Fv Fragments in E. coli Cytoplasm. Antibody Phage Display. Humana Press, Totowa, NJ, pp. 215-224.
**○** Inoue A, Sawata SY, Taira K, Wadhwa R, (2007). Loss-of-function screening by randomized intracellular antibodies : Identification of hnRNP-K as a potential target for metastasis, PNAS, 104(21), 8983-8988.
**○** Kabat E.A., Wu,T.T., Perry,H., Gottesman,K. and Foeller,C. (1991) Sequences of Proteins of Immunological Interest, Fifth Edition. NIH Publication No. 91-3242.
**○** Kontermann R.E., Dübel S., Antibody Engineering Volume 2 (Springer Protocols) (2010).
**○** Li J, Tibshirani R (2011). Finding consistent patterns: A nonparametric approach for identifying differential expression in RNA-Seq data. Stat Methods Med Res. [Epub ahead of print].
**○** Mazuc E, Villoutreix BO, Malbec O, Roumier T, Fleury S, Leonetti JP, Dombrowicz D, Daeron M, Martineau P, Dariavach P (2008). A novel druglike spleen tyrosine kinase binder prevents anaphylactic shock when administered orally. The Journal of Allergy and Clinical Immunology, 122, 188-194.
**○** Moutel S, El Marjou A, Vielemeyer O, Nizak C, Benaroch P, Dubel S, Perez F (2009). A multi-Fc-species system for recombinant antibody production. BMC Biotechnol, 9:14.
**○** Muyldermans, S. (2001) Single domain camel antibodies: current status. Reviews in Molecular Biotechnology 74 (2001) 277-302.
**○** Ota M, Katsuyama Y, Hamano H, Umemura T, Kimura A, Yoshizawa K, Kiyosawa K, Fukushima H, Bahram S, Inoko H, Kawa S (2007). Two critical genes (HLA-DRB1 and ABCF1) in the HLA region are associated with the susceptibility to autoimmune pancreatitis. Immunogenetics, 59(1):45-52. Epub 2006 Nov 21.
**○** Paytubi S, Wang X, Lam YW, Izquierdo L, Hunter J, Hundal, Proud C (2009). ABC50 Promotes Translation Initiation in Mammalian Cells. Journal of Biological Chemistry, 284(36):24061-73.
**○** Paytubi S, Morrice N, Boudeau J, Proud C (2008). The N-terminal region of ABC50 interacts with eukaryotic initiation factoreIF2 and is a target for regulatory phosphorylation by CK2. J.Biochem, 409: 223-231.
**○** Pazos F, Valencia A (2001). Similarity of phylogenetic trees as indicator of protein-protein interaction. Protein Eng, 14(9):609-14.
**○** Persic L, Righi M, Roberts A, Hoogenboom HR, Cattaneo A, Bradbury A (1997). Targeting vectors for intracellular immunisation. Gene, 187, 1-8.
**○** Philibert P, Stoessel A, Wang W, Sibler AP, Bec N, Larroque C, Saven JG, Courtete J, Weiss E, Martineau P (2007). A focused antibody library for selecting scFvs expressed at high levels in the cytoplasm. BMC biotechnology, 7,81.
**○** Shevchenko A, Tomas H, Havlis J, Olsen JV, Mann M (2007). In-gel digestion for mass spectrometric characterization of proteins and proteomes. Nat. Protocols, 1, 2856-2860.
**○** Stijlemans B, Conrath K, Cortez-Retamozo V, Van Xong H, Wyns L, Senter P, Revets H, De Baetselier P, Muyldermans S, Magez S (2004). Efficient Targeting of Conserved Cryptic Epitopes of Infectious Agents by Single Domain Antibodies. Journal of Biological Chemistry, 279, 1256 -1261.
**○** Tyzack JK, Wang X, Belsham GJ, Proud CG (2000). ABC50 interacts with eukaryotic initiation factor 2 and associates with the ribosome in an ATP-dependent manner. J. Biol. Chem., 275, 34131-9.
**○** Villoutreix BO, Laconde G, Lagorce D, Martineau P, Miteva MA, Dariavach P (2011). Tyrosine Kinase Syk Non-Enzymatic Inhibitors and Potential Anti-Allergic Drug-Like Compounds Discovered by Virtual and In Vitro Screening. PLoS ONE, 6, e21117.

### Sequences

SEQ ID NO: 1 : Intracellular antibody 3H2-1
SEQ ID NO : 2 : Intracellular antibody 3H2-VH
SEQ ID NO : 3 : Intracellular antibody 5H4
SEQ ID NO : 4 : Intracellular antibody 5H4-VH
SEQ ID NO : 5 : Intracellular antibody 5H4-VL
SEQ ID NO : 6 : Intracellular antibody 13R4
SEQ ID NO : 7 : CDR3 of the VH chain of an intracellular antibody of cluster R_1
SEQ ID NO : 8 : CDR3 of the VH chain of an intracellular antibody of cluster R_2
SEQ ID NO : 9 : CDR3 of the VH chain of an intracellular antibody of cluster R_3
SEQ ID NO : 10 : CDR3 of the VH chain of an intracellular antibody of cluster R_4
SEQ ID NO : 11 : CDR3 of the VH chain of an intracellular antibody of cluster R_5
SEQ ID NO : 12 : CDR3 of the VH chain of an intracellular antibody of cluster R_6
SEQ ID NO : 13 : CDR3 of the VH chain of an intracellular antibody of cluster R_7
SEQ ID NO : 14 : CDR3 of the VH chain of an intracellular antibody of cluster R_9
SEQ ID NO : 15 : CDR3 of the VH chain of an intracellular antibody of cluster R_10
SEQ ID NO : 16 : CDR3 of the VH chain of an intracellular antibody of cluster D_1
SEQ ID NO : 17 : CDR3 of the VH chain of an intracellular antibody of cluster D_2
SEQ ID NO : 18 : CDR3 of the VH chain of an intracellular antibody of cluster D_3
SEQ ID NO : 19 : CDR3 of the VL chain of an intracellular antibody of cluster R_1
SEQ ID NO : 20 : CDR3 of the VL chain of an intracellular antibody of cluster R_2
SEQ ID NO : 21 : CDR3 of the VL chain of an intracellular antibody of cluster R_3
SEQ ID NO : 22 : CDR3 of the VL chain of an intracellular antibody of cluster R_4
SEQ ID NO : 23 : CDR3 of the VL chain of an intracellular antibody of cluster R_5
SEQ ID NO : 24 : CDR3 of the VL chain of an intracellular antibody of cluster R_6
SEQ ID NO : 25 : CDR3 of the VL chain of an intracellular antibody of cluster R_9
SEQ ID NO : 26 : CDR3 of the VL chain of an intracellular antibody of cluster R_10
SEQ ID NO : 27 : CDR3 of the VL chain of an intracellular antibody of cluster D_1
SEQ ID NO : 28 : CDR3 of the VL chain of an intracellular antibody of cluster D_2
SEQ ID NO : 29 : CDR3 of the VL chain of an intracellular antibody of cluster D_3
SEQ ID NO : 30 : sense strand of sh1 shRNA to LOC297607
SEQ ID NO : 31 : sense strand of sh2 shRNA to LOC297607
SEQ ID NO : 32 : LOC297607 CDS (Accession : NM_0011 06623.1 GI:157820036)
SEQ ID NO : 33 : amino acid sequence of LOC297607 (NP 001100093.1 GI:157820037)
SEQ ID NO : 34 : C12ORF4 CDS (Accession : NM_020374.2 GI:22095357)
SEQ ID NO : 35 : amino acid sequence of C12ORF4 (Accession : NP_065107.1 GI:9966847)
SEQ ID NO : 36 LOC57102 CDS (Accession: JV047725.1 ; GI: 384948381)
SEQ ID NO : 37 : amino acid sequence of LOC57102 (Accession : AFI37796.1 GI:384948382)
SEQ ID NO : 38 : LOC28040 CDS (Accession : NM_138594.3 GI:142372851)
SEQ ID NO : 39 : amino acid sequence of LOC28040 (NP_613060.1 GI:20070406)
SEQ ID NO : 40 : human ABCF1 CDS (Accession : NM_001025091.1 GI:69354670)
SEQ ID NO : 41 : amino acid sequence of human ABCF1 (Accession : NP_001020262.1 GI:69354671)
SEQ ID NO: 42 : CDR3 sequence of the VH domain of intrabody 3H2-1
SEQ ID NO : 43 : CDR3 sequence of the VH domain of intrabody 3H2-VH
SEQ ID NO : 44 : CDR3 sequence of the VH domain of intrabody 5H4
SEQ ID NO : 45 : antisense strand of sh1 shRNA to LOC297607
SEQ ID NO : 46 : antisense strand of sh2 shRNA to LOC297607

## Claims

1. Method for identifying a cellular target which is involved in a cell phenotype, comprising:
a) identifying an intrabody comprising a full V_{H} and/or V_{L} domain of an immunoglobulin, which can induce, modify or suppress said phenotype when present inside a cell;
b) identifying a cellular target which is a direct or indirect target of said intrabody in said cell; and optionally
c) isolating said cellular target.

2. Method according to claim 1, wherein step a) comprises the screening of an intracellularly expressed intrabody library, wherein each intrabody comprises a full V_{H} and/or V_{L} domain of an immunoglobulin.

3. Method according to claim 1 or 2, wherein step a) comprises:
i) obtaining a library of molecules, wherein each molecule from the library encodes a different intrabody comprising a full V_{H} and/or V_{L} domain of an immunoglobulin;
ii) transfecting a population of cells with the library of molecules of step i);
iii) culturing the transfected cells for a time and under conditions sufficient for detectable induction, modification or suppression of said phenotype;
iv) selecting the cells of step iii) which show an induction, modification or suppression of said phenotype;
v) optionally repeating steps iii) and iv) on the cells selected from step iv) or on cells recloned from the cells selected from step iv) for one or more additional selection rounds; and
vi) identifying the intrabody which is responsible for said phenotype induction, modification or suppression.

4. Method according to claim 3, wherein the method comprises a recloning step after at least one selection round, and submitting the recloned cells to one or more selection rounds.

5. Method according to claim 3 or 4, wherein said molecule encoding an intrabody is a vector.

6. Method according to claim 5, wherein said vector is an integrative vector such as a retroviral vector.

7. Method according to claim 2 to 6, wherein said intrabody library and/or said library of molecules is obtained by selecting an intrabody which is functional inside a cell, and then introducing modifications in one or more of its CDR regions.

8. Method according to claim 1 to 7, wherein said direct or indirect target of said intrabody binds to said intrabody or can be immunoprecipitated together with said intrabody.

9. Method according to claim 1 to 8, wherein the intrabody is an scFv, a truncated scFv comprising at least a full V_{H} or V_{L} domain, a diabody, a full V_{H} domain or a full V_{L} domain.

10. Method according to claim 1 to 9, wherein the full V_{H} domain and/or the full V_{L} domain is derived from a human antibody.

11. Method according to any one of claims 1 to 10, further comprising
- a step of target validation using RNA interference technology or a known inhibitor of said cellular target; and/or
- a step of identification of the epitope or the active site of the cellular target; and/or,
- a step of identification of a molecule which competes with the binding of the intrabody identified in step a) to the target identified in step b), and which is capable of modifying said cell phenotype.

12. Method according to any one of claims 1 to 11, wherein said cell is a eukaryotic cell, preferably a mammalian cell such as a human cell.

13. Method according to claim 12, wherein said eukaryotic cell is a cell involved in allergy, inflammation, or both and wherein said phenotype is a phenotype associated with an allergic reaction, an inflammatory reaction, or both.

14. Intrabody comprising the CDR3 sequence "DGGLREGFDC" of the V_{H} domain of scFv 5H4.

15. Intrabody according to claim 14, further comprising the CDR1 sequence and CDR2 sequence of the V_{H} domain of scFv 13R4.

16. Intrabody according to claim 14, wherein said intrabody is intrabody 5H4 (SEQ ID NO:3), 5H4-V_{H} (SEQ ID NO:4) or 5H4-V_{L} (SEQ ID NO:5).

17. Intrabody comprising the CDR3 sequence "PIAVSDY" of the V_{H} domain of scFv 3H2-1.

18. Intrabody according to claim 17, further comprising the CDR3 sequence of the V_{L} domain of scFv 3H2-1, and preferably the CDR1 sequence and CDR2 sequence of the V_{H} domain of scFv 13R4, and/or the CDR1 sequence and CDR2 sequence of the V_{L} domain of scFv 13R4.

19. Intrabody according to claim 18, wherein said intrabody is intrabody 3H2-1 (SEQ ID NO:1).

20. Intrabody comprising the CDR3 sequence "GVRGGYGLDF" of the V_{H} domain of scFv 3H2-VH.

21. Intrabody according to claim 20, further comprising the CDR1 sequence and CDR2 sequence of the V_{H} domain of scFv 13R4.

22. Intrabody according to claim 21, wherein said intrabody is intrabody 3H2-VH (SEQ ID NO:2).

23. Intrabody comprising one of the V_{H} CDR3 sequences set forth in SEQ ID NO: 7 to SEQ ID NO: 18.

24. Intrabody according to claim 23, wherein said intrabody further comprises one the V_{L} CDR3 sequences set forth in SEQ ID NO:19 to SEQ ID NO:29 or further comprises a glutamine as V_{L} CDR3 sequence.

25. Intrabody according to claim 24, wherein said intrabody further comprises the CDR1 sequence and CDR2 sequence of the V_{H} domain of scFv 13R4, and/or the CDR1 sequence and CDR2 sequence of the V_{L} domain of scFv 13R4.

26. Intrabody according to claim 14 to 25 for use in therapy.

27. Intrabody according to claim 26 for use in treating allergy and/or inflammation.

28. Use of an intrabody according to any one of claims 14 to 16 for identifying a molecule which is capable of competing with the binding of said intrabody with a protein from the C120RF4 family, and of modifying a phenotype associated with an allergic and/or inflammatory reaction in a cell involved in allergy and/or inflammation.

29. Use according to claim 28 wherein said protein from the C12ORF4 family is C12ORF4, LOC57102, LOC297607 or LOC28040, preferably is C12ORF4.

30. Use of an intrabody according to any one of claims 17 to 19 for identifying a molecule which is capable of competing with the binding of said intrabody with a protein of the ABCF1 family, and of modifying a phenotype associated with an allergic and/or inflammatory reaction in a cell involved in allergy and/or inflammation.

31. Use according to claim 28 to 30, wherein said molecule is an organic molecule having a molecular weight of 100 to 2500 Da.

32. Inhibitor of a protein from the C12ORF4 family for use in therapy.

33. Inhibitor according to claim 32 for use in treating allergy and/or inflammation.

34. Inhibitor according to claim 32 or 33, wherein said inhibitor is
- an intrabody or an antigen-binding fragment thereof capable of binding to a protein of the C12ORF4 family, preferably an intrabody according to any one of claims 14 to 16 or an antigen-binding fragment thereof;
- an RNA molecule capable of interfering with the expression of a protein of the C12ORF4 family in a cell; or
- an organic molecule having a molecular weight of 100 to 2500 Da which is capable of displacing an intrabody according to any one of claims 8 to 10 from its binding site with a protein of the C12ORF4 family.

35. Inhibitor according to claim 32 to 34, wherein said protein of the C12ORF4 family is C12ORF4, LOC57102, LOC297607 or LOC28040, preferably is C12ORF4.

36. Inhibitor of a protein of the ABCF1 family for use in therapy.

37. Inhibitor according to claim 36 for use in treating allergy and/or inflammation.

38. Inhibitor according to claim 36 or 37, wherein said inhibitor is
- an intrabody or an antigen-binding fragment thereof capable of binding to a protein of the ABCF1 family, preferably an intrabody according to any one of claims 17 to 19 or an antigen-binding fragment thereof;
- an RNA molecule capable of interfering with the expression of a protein of the ABCF1 family in a cell; or
- an organic molecule having a molecular weight of 100 to 2500 Da which is capable of displacing an intrabody according to any one of claims 11 to 13 from its binding site with a protein of the ABCF1 family.

39. Inhibitor according to claim 36 to 38, wherein said protein of the ABCF1 family is ABCF1.


SEQUENCE LISTING

<110>  SANOFI
       INSERM
       UNIVERSITE DE MONTPELLIER 1
       CENTRE VAL D'AURELLE- PAUL LAMARQUE
       UNIVERSITE DE MONTPELLIER 2 SCIENCES ET TECHNIQUES

<120>  Intracellular phenotypic screening

<130>  B09196

<160>  46

<170>  PatentIn version 3.5

<210>  1
<211>  260
<212>  PRT
<213>  Artificial Sequence

<220>
<223>  Intracellular antibody 3H2-1


<220>
<221>  DOMAIN
<222>  (3)..(118)
<223>  VH

<220>
<221>  DOMAIN
<222>  (101)..(107)
<223>  VH_CDR3

<220>
<221>  DOMAIN
<222>  (134)..(243)
<223>  VL

<220>
<221>  DOMAIN
<222>  (224)..(233)
<223>  VL_CDR3

<400>  1

Met Ala Glu Val Gln Leu Val Glu Ser Gly Gly Ser Leu Val Lys Pro
1               5                   10                  15


Gly Gly Ser Leu Arg Leu Ser Cys Ala Ala Ser Gly Phe Thr Phe Ser
            20                  25                  30


Asn Tyr Ser Met Asn Trp Val Arg Gln Ala Pro Gly Lys Gly Leu Glu
        35                  40                  45


Trp Ile Ser Ser Ile Ser Gly Ser Ser Arg Tyr Ile Tyr Tyr Ala Asp
    50                  55                  60


Phe Val Lys Gly Arg Phe Thr Ile Ser Arg Asp Asn Ala Thr Asn Ser
65                  70                  75                  80

Leu Tyr Leu Gln Met Asn Ser Leu Arg Ala Glu Asp Thr Ala Val Tyr
                85                  90                  95

Tyr Cys Val Arg Pro Ile Ala Val Ser Asp Tyr Trp Gly Arg Gly Thr
            100                 105                 110

Leu Val Thr Val Ser Ser Gly Gly Gly Gly Ser Gly Gly Gly Gly Ser
        115                 120                 125

Gly Gly Gly Gly Ser Gln Ser Val Leu Thr Gln Pro Ala Ser Val Ser
    130                 135                 140

Gly Ser Pro Gly Gln Ser Ile Thr Ile Ser Cys Ala Gly Thr Ser Ser
145                 150                 155                 160

Asp Val Gly Gly Tyr Asn Tyr Val Ser Trp Tyr Gln Gln His Pro Gly
                165                 170                 175

Lys Ala Pro Lys Leu Met Ile Tyr Glu Asp Ser Lys Arg Pro Ser Gly
            180                 185                 190

Val Ser Asn Arg Phe Ser Gly Ser Lys Ser Gly Asn Thr Ala Ser Leu
        195                 200                 205

Thr Ile Ser Gly Leu Gln Ala Glu Asp Glu Ala Asp Tyr Tyr Cys Gln
    210                 215                 220

Thr Tyr Asp Gly Ser Arg Ala Val Phe Gly Gly Gly Thr Lys Leu Ala
225                 230                 235                 240

Val Leu Gly Ala Ala Ala Glu Gln Lys Leu Ile Ser Glu Glu Asp Leu
                245                 250                 255

Asn Gly Ala Ala
            260

<210>  2
<211>  153
<212>  PRT
<213>  Artificial Sequence

<220>
<223>  Intracellular antibody 3H2-VH

<220>
<221>  DOMAIN
<222>  (3)..(121)
<223>  VH

<220>

<221>  DOMAIN
<222>  (101)..(110)
<223>  VH_CDR3

<400>  2

Met Ala Glu Val Gln Leu Val Glu Ser Gly Gly Ser Leu Val Lys Pro
1               5                   10                  15

Gly Gly Ser Leu Arg Leu Ser Cys Ala Ala Ser Gly Phe Thr Phe Ser
            20                  25                  30

Asn Tyr Ser Met Asn Trp Val Arg Gln Ala Pro Gly Lys Gly Leu Glu
        35                  40                  45

Trp Ile Ser Ser Ile Ser Gly Ser Ser Arg Tyr Ile Tyr Tyr Ala Asp
    50                  55                  60

Phe Val Lys Gly Arg Phe Thr Ile Ser Arg Asp Asn Ala Thr Asn Ser
65                  70                  75                  80

Leu Tyr Leu Gln Met Asn Ser Leu Arg Ala Glu Asp Thr Ala Val Tyr
                85                  90                  95

Tyr Cys Val Arg Gly Val Arg Gly Gly Tyr Gly Leu Asp Phe Trp Gly
            100                 105                 110

Arg Gly Thr Leu Val Thr Val Ser Ser Gly Gly Gly Gly Ser Gly Gly
        115                 120                 125

Gly Thr Lys Leu Ala Val Leu Gly Ala Ala Ala Glu Gln Lys Leu Ile
    130                 135                 140

Ser Glu Glu Asp Leu Asn Gly Ala Ala
145                 150


<210>  3
<211>  228
<212>  PRT
<213>  Artificial Sequence

<220>
<223>  Intracellular antibody 5H4


<220>
<221>  DOMAIN
<222>  (3)..(121)
<223>  VH

<220>
<221>  DOMAIN
<222>  (101)..(110)
<223>  VH_CDR3

<400>  3

Met Ala Glu Val Gln Leu Val Glu Ser Gly Gly Ser Leu Val Lys Pro
1               5                   10                  15

Gly Gly Ser Leu Arg Leu Ser Cys Ala Ala Ser Gly Phe Thr Phe Ser
            20                  25                  30

Asn Tyr Ser Met Asn Trp Val Arg Gln Ala Pro Gly Lys Gly Leu Glu
        35                  40                  45

Trp Ile Ser Ser Ile Ser Gly Ser Ser Arg Tyr Ile Tyr Tyr Ala Asp
    50                  55                  60

Phe Val Lys Gly Arg Phe Thr Ile Ser Arg Asp Asn Ala Thr Asn Ser
65                  70                  75                  80

Leu Tyr Leu Gln Met Asn Ser Leu Arg Ala Glu Asp Thr Ala Val Tyr
                85                  90                  95

Tyr Cys Val Arg Asp Gly Gly Leu Arg Glu Gly Phe Asp Cys Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Gln Ser Val Leu Thr Gln Pro Ala
    130                 135                 140

Ser Val Ser Gly Ser Pro Gly Gln Ser Ile Thr Ile Ser Cys Ala Gly
145                 150                 155                 160

Thr Ser Ser Asp Val Gly Gly Tyr Asn Tyr Val Ser Trp Tyr Gln Gln
                165                 170                 175

His Pro Gly Lys Ala Pro Lys Leu Met Ile Tyr Glu Asp Ser Lys Arg
            180                 185                 190

Pro Ser Gly Val Ser Asn Arg Phe Ser Gly Ser Lys Ser Gly Asn Thr
        195                 200                 205

Ala Ser Leu Thr Ile Ser Gly Leu Gln Ala Glu Asp Glu Ala Asp Tyr
    210                 215                 220

Tyr Cys Pro Ser
225

<210>  4
<211>  138
<212>  PRT

<213>  Artificial Sequence

<220>
<223>  Intracellular antibody 5H4-VH


<220>
<221>  DOMAIN
<222>  (3)..(121)
<223>  VH

<220>
<221>  DOMAIN
<222>  (101)..(110)
<223>  VH_CDR3

<400>  4

Met Ala Glu Val Gln Leu Val Glu Ser Gly Gly Ser Leu Val Lys Pro
1               5                   10                  15


Gly Gly Ser Leu Arg Leu Ser Cys Ala Ala Ser Gly Phe Thr Phe Ser
            20                  25                  30


Asn Tyr Ser Met Asn Trp Val Arg Gln Ala Pro Gly Lys Gly Leu Glu
        35                  40                  45


Trp Ile Ser Ser Ile Ser Gly Ser Ser Arg Tyr Ile Tyr Tyr Ala Asp
    50                  55                  60


Phe Val Lys Gly Arg Phe Thr Ile Ser Arg Asp Asn Ala Thr Asn Ser
65                  70                  75                  80


Leu Tyr Leu Gln Met Asn Ser Leu Arg Ala Glu Asp Thr Ala Val Tyr
                85                  90                  95


Tyr Cys Val Arg Asp Gly Gly Leu Arg Glu Gly Phe Asp Cys Trp Gly
            100                 105                 110


Arg Gly Thr Leu Val Thr Val Ser Ser Ala Ala Ala Glu Gln Lys Leu
        115                 120                 125


Ile Ser Glu Glu Asp Leu Asn Gly Ala Ala
    130                 135


<210>  5
<211>  243
<212>  PRT
<213>  Artificial Sequence

<220>
<223>  Intracellular antibody 5H4-VL


<220>
<221>  DOMAIN

<222>  (3)..(121)
<223>  VH

<220>
<221>  DOMAIN
<222>  (101)..(110)
<223>  VH_CDR3

<400>  5

Met Ala Glu Val Gln Leu Val Glu Ser Gly Gly Ser Leu Val Lys Pro
1               5                   10                  15

Gly Gly Ser Leu Arg Leu Ser Cys Ala Ala Ser Gly Phe Thr Phe Ser
            20                  25                  30

Asn Tyr Ser Met Asn Trp Val Arg Gln Ala Pro Gly Lys Gly Leu Glu
        35                  40                  45

Trp Ile Ser Ser Ile Ser Gly Ser Ser Arg Tyr Ile Tyr Tyr Ala Asp
    50                  55                  60

Phe Val Lys Gly Arg Phe Thr Ile Ser Arg Asp Asn Ala Thr Asn Ser
65                  70                  75                  80

Leu Tyr Leu Gln Met Asn Ser Leu Arg Ala Glu Asp Thr Ala Val Tyr
                85                  90                  95

Tyr Cys Val Arg Asp Gly Gly Leu Arg Glu Gly Phe Asp Cys Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Gln Ser Val Leu Thr Gln Pro Ala
    130                 135                 140

Ser Val Ser Gly Ser Pro Gly Gln Ser Ile Thr Ile Ser Cys Ala Gly
145                 150                 155                 160

Thr Ser Ser Asp Val Gly Gly Tyr Asn Tyr Val Ser Trp Tyr Gln Gln
                165                 170                 175

His Pro Gly Lys Ala Pro Lys Leu Met Ile Tyr Glu Asp Ser Lys Arg
            180                 185                 190

Pro Ser Gly Val Ser Asn Arg Phe Ser Gly Ser Lys Ser Gly Asn Thr
        195                 200                 205

Ala Ser Leu Thr Ile Ser Gly Leu Gln Ala Glu Asp Glu Ala Asp Tyr
    210                 215                 220

Tyr Cys Ala Ala Ala Glu Gln Lys Leu Ile Ser Glu Glu Asp Leu Asn
225                 230                 235                 240

Gly Ala Ala

<210>  6
<211>  266
<212>  PRT
<213>  Artificial Sequence

<220>
<223>  Intracellular antibody 13R4

<220>
<221>  DOMAIN
<222>  (3)..(123)
<223>  VH

<220>
<221>  DOMAIN
<222>  (3)..(32)
<223>  VH_FR1

<220>
<221>  DOMAIN
<222>  (33)..(37)
<223>  VH_CDR1

<220>
<221>  DOMAIN
<222>  (38)..(51)
<223>  VH_FR2

<220>
<221>  DOMAIN
<222>  (52)..(68)
<223>  VH_CDR2

<220>
<221>  DOMAIN
<222>  (69)..(100)
<223>  VH_FR3

<220>
<221>  DOMAIN
<222>  (101)..(112)
<223>  VH_CDR3

<220>
<221>  DOMAIN
<222>  (113)..(123)
<223>  VH_FR4

<220>
<221>  DOMAIN
<222>  (139)..(249)
<223>  VL

<220>
<221>  DOMAIN

<222>  (139)..(160)
<223>  VL_FR1

<220>
<221>  DOMAIN
<222>  (161)..(174)
<223>  VL_CDR1

<220>
<221>  DOMAIN
<222>  (175)..(189)
<223>  VL_FR2

<220>
<221>  DOMAIN
<222>  (190)..(196)
<223>  VL_CDR2

<220>
<221>  DOMAIN
<222>  (197)..(228)
<223>  VL_FR3

<220>
<221>  DOMAIN
<222>  (229)..(239)
<223>  VL_CDR3

<220>
<221>  DOMAIN
<222>  (240)..(249)
<223>  VL_FR4

<400>  6

Met Ala Glu Val Gln Leu Val Glu Ser Gly Gly Ser Leu Val Lys Pro
1               5                   10                  15

Gly Gly Ser Leu Arg Leu Ser Cys Ala Ala Ser Gly Phe Thr Phe Ser
            20                  25                  30

Asn Tyr Ser Met Asn Trp Val Arg Gln Ala Pro Gly Lys Gly Leu Glu
        35                  40                  45

Trp Ile Ser Ser Ile Ser Gly Ser Ser Arg Tyr Ile Tyr Tyr Ala Asp
    50                  55                  60

Phe Val Lys Gly Arg Phe Thr Ile Ser Arg Asp Asn Ala Thr Asn Ser
65                  70                  75                  80

Leu Tyr Leu Gln Met Asn Ser Leu Arg Ala Glu Asp Thr Ala Val Tyr
                85                  90                  95

Tyr Cys Val Arg Ser Ser Ile Thr Ile Phe Gly Gly Gly Met Asp Val
            100                 105                 110

Trp Gly Arg Gly Thr Leu Val Thr Val Ser Ser Gly Gly Gly Gly Ser
        115                 120                 125

Gly Gly Gly Gly Ser Gly Gly Gly Gly Ser Gln Ser Val Leu Thr Gln
    130                 135                 140

Pro Ala Ser Val Ser Gly Ser Pro Gly Gln Ser Ile Thr Ile Ser Cys
145                 150                 155                 160

Ala Gly Thr Ser Ser Asp Val Gly Gly Tyr Asn Tyr Val Ser Trp Tyr
                165                 170                 175

Gln Gln His Pro Gly Lys Ala Pro Lys Leu Met Ile Tyr Glu Asp Ser
            180                 185                 190

Lys Arg Pro Ser Gly Val Ser Asn Arg Phe Ser Gly Ser Lys Ser Gly
        195                 200                 205

Asn Thr Ala Ser Leu Thr Ile Ser Gly Leu Gln Ala Glu Asp Glu Ala
    210                 215                 220

Asp Tyr Tyr Cys Ser Ser Tyr Thr Thr Arg Ser Thr Arg Val Phe Gly
225                 230                 235                 240

Gly Gly Thr Lys Leu Ala Val Leu Gly Ala Ala Ala Glu Gln Lys Leu
                245                 250                 255

Ile Ser Glu Glu Asp Leu Asn Gly Ala Ala
            260                 265

<210>  7
<211>  15
<212>  PRT
<213>  Artificial Sequence

<220>
<223>  CDR3 of the VH chain of an intracellular antibody of cluster R_1

<400>  7

Met Asp Cys Val Ile Gly Ser Tyr Gly Tyr Gly Ile Phe Asp Thr
1               5                   10                  15

<210>  8
<211>  15
<212>  PRT
<213>  Artificial Sequence

<220>
<223>  CDR3 of the VH chain of an intracellular antibody of cluster R_2

<400>  8

Gly Lys Val Leu Lys Lys Ala Glu Tyr Ser Asp Trp Leu Asp Asn
1               5                   10                  15

<210>  9
<211>  7
<212>  PRT
<213>  Artificial Sequence

<220>
<223>  CDR3 of the VH chain of an intracellular antibody of cluster R_3

<400>  9

Arg Ser Ala Ser Cys Glu His
1               5


<210>  10
<211>  7
<212>  PRT
<213>  Artificial Sequence

<220>
<223>  CDR3 of the VH chain of an intracellular antibody of cluster  R_4

<400>  10

Gly Glu Val Gly Phe Asp Tyr
1               5


<210>  11
<211>  14
<212>  PRT
<213>  Artificial Sequence

<220>
<223>  CDR3 of the VH chain of an intracellular antibody of cluster R_5

<400>  11

Thr Leu Glu Cys Ser Arg Cys Gly Asp Tyr Gly Phe Asp Leu
1               5                   10


<210>  12
<211>  14
<212>  PRT
<213>  Artificial Sequence

<220>
<223>  CDR3 of the VH chain of an intracellular antibody of cluster R_6

<400>  12

Asp Gly Leu Tyr Ala Arg Met Tyr Tyr Asn Gly Ser Tyr Tyr
1               5                   10


<210>  13
<211>  17
<212>  PRT
<213>  Artificial Sequence

<220>
<223>  CDR3 of the VH chain of an intracellular antibody of cluster R_7

<400>  13

Glu Arg Arg Asp Asp Asp Gly Met Tyr Ala Tyr Ser Tyr Gln Phe Asp
1               5                   10                  15

Val

<210>  14
<211>  15
<212>  PRT
<213>  Artificial Sequence

<220>
<223>  CDR3 of the VH chain of an intracellular antibody of cluster R_9

<400>  14

Asn Pro Ala Ser Lys Cys Val Tyr Leu Glu His Asp Phe Glu Lys
1               5                   10                  15

<210>  15
<211>  8
<212>  PRT
<213>  Artificial Sequence

<220>
<223>  CDR3 of the VH chain of an intracellular antibody of cluster R_10

<400>  15

Pro Glu Arg Ser Ala Tyr Asp Tyr
1               5

<210>  16
<211>  8
<212>  PRT
<213>  Artificial Sequence

<220>
<223>  CDR3 of the VH chain of an intracellular antibody of cluster D_1

<400>  16

Gly Asp Ser His Ile Ile Asp Cys
1               5

<210>  17
<211>  8
<212>  PRT
<213>  Artificial Sequence

<220>
<223>  CDR3 of the VH chain of an intracellular antibody of cluster D_2

<400>  17

Gly Ser Thr Ala Gly Phe Asp Tyr
1               5

<210>  18
<211>  8
<212>  PRT
<213>  Artificial Sequence

<220>
<223>  CDR3 of the VH chain of an intracellular antibody of cluster D_3

<400>  18

Glu Glu Gly Val Asp Ile Glu Tyr
1               5


<210>  19
<211>  9
<212>  PRT
<213>  Artificial Sequence

<220>
<223>  CDR3 of the VL chain of an intracellular antibody of cluster R_1

<400>  19

Gln Ser Phe Val Arg Asn Ser Thr Ser
1               5


<210>  20
<211>  9
<212>  PRT
<213>  Artificial Sequence

<220>
<223>  CDR3 of the VL chain of an intracellular antibody of cluster R_2

<400>  20

Gln Gln Cys Ser Lys Phe Pro Leu Thr
1               5


<210>  21
<211>  10
<212>  PRT
<213>  Artificial Sequence

<220>
<223>  CDR3 of the VL chain of an intracellular antibody of cluster R_3

<400>  21

Glu Gln Tyr Asp Thr Ala Pro Pro Tyr Thr
1               5                   10


<210>  22
<211>  9
<212>  PRT
<213>  Artificial Sequence

<220>
<223>  CDR3 of the VL chain of an intracellular antibody of cluster R_4

<400>  22

Gln Gln Tyr Phe Ser Gln Pro Phe Thr
1               5


<210>  23
<211>  9
<212>  PRT
<213>  Artificial Sequence

<220>
<223>  CDR3 of the VL chain of an intracellular antibody of cluster R_5

<400>  23

His Gln Ser Asn Thr Tyr Pro Phe Thr
1               5


<210>  24
<211>  9
<212>  PRT
<213>  Artificial Sequence

<220>
<223>  CDR3 of the VL chain of an intracellular antibody of cluster R_6

<400>  24

Gln Gln Tyr Phe Ser Gln Pro Phe Thr
1               5


<210>  25
<211>  9
<212>  PRT
<213>  Artificial Sequence

<220>
<223>  CDR3 of the VL chain of an intracellular antibody of cluster R_9

<400>  25

Gln Thr Cys Asn Cys Leu Thr Leu Val
1               5


<210>  26
<211>  9
<212>  PRT
<213>  Artificial Sequence

<220>
<223>  CDR3 of the VL chain of an intracellular antibody of cluster R_10

<400>  26

Gln Gln Tyr Ser Ser His Pro Leu Thr
1               5


<210>  27
<211>  9
<212>  PRT

<213>  Artificial Sequence

<220>
<223>  CDR3 of the VL chain of an intracellular antibody of cluster D_1

<400>  27

Gln Gln Ser Asn Ile Leu Ser Val Thr
1               5


<210>  28
<211>  9
<212>  PRT
<213>  Artificial Sequence

<220>
<223>  CDR3 of the VL chain of an intracellular antibody of cluster D_2

<400>  28

Gln Gln Asp Asp Ser Thr Pro Tyr Thr
1               5


<210>  29
<211>  11
<212>  PRT
<213>  Artificial Sequence

<220>
<223>  CDR3 of the VL chain of an intracellular antibody of cluster D_3

<400>  29

Pro Ser Leu Asn Asn Ser Leu Thr Tyr Ile Val
1               5                   10


<210>  30
<211>  66
<212>  DNA
<213>  Artificial Sequence

<220>
<223>  sense strand of sh1 shRNA to LOC297607

<400>  30
gatccgcatt ctaatctctc ggaaattcaa gagatttccg agagattaga atgttttttt      60

ctagag                                                                  66


<210>  31
<211>  66
<212>  DNA
<213>  Artificial Sequence

<220>
<223>  sense strand of sh2 shRNA to LOC297607

<400>  31
gatccgagaa ttgattggcg aaagattcaa gagatctttc gccaatcaat tctttttttt      60

ctagag                                                                  66

<210>  32
<211>  1659
<212>  DNA
<213>  Rattus norvegicus

<220>
<221>  CDS
<222>  (1)..(1659)
<223>  Protein LOC297607

<400>  32
atg aag aaa aac cga gaa agg ttc tcc agc aag gaa aga gaa ttc gtg      48
Met Lys Lys Asn Arg Glu Arg Phe Ser Ser Lys Glu Arg Glu Phe Val
1               5                   10                  15

tac aaa ttc cag ata gga agt gag cgc tta gaa ctg aga gtg ccc ctc      96
Tyr Lys Phe Gln Ile Gly Ser Glu Arg Leu Glu Leu Arg Val Pro Leu
            20                  25                  30

agg ttt ccc gtg gac gag aat gcc agt cat ctg cat gga cgt ctg atg      144
Arg Phe Pro Val Asp Glu Asn Ala Ser His Leu His Gly Arg Leu Met
        35                  40                  45

ctg ctg cac agt ttg ccc tgc ttc ata gag aat gac tta aaa gaa gct      192
Leu Leu His Ser Leu Pro Cys Phe Ile Glu Asn Asp Leu Lys Glu Ala
    50                  55                  60

ctg gcc cgg ttt ata gaa gaa gag tcc ctc aga gac cat gac agc gat      240
Leu Ala Arg Phe Ile Glu Glu Glu Ser Leu Arg Asp His Asp Ser Asp
65                  70                  75                  80

gca gaa gca tgc ttg gag gcg gtt aag tct ggt gag gta gat cta cac      288
Ala Glu Ala Cys Leu Glu Ala Val Lys Ser Gly Glu Val Asp Leu His
                85                  90                  95

cag ctg gcg agt aca tgg gcc aaa gcg tat gct gag aca acc cta gag      336
Gln Leu Ala Ser Thr Trp Ala Lys Ala Tyr Ala Glu Thr Thr Leu Glu
            100                 105                 110

cat gcg aga cct gag gag cct aac tgg gat gaa gac ttt gca gat gtg      384
His Ala Arg Pro Glu Glu Pro Asn Trp Asp Glu Asp Phe Ala Asp Val
        115                 120                 125

tac cat gac cta atc cat tct ccg gcc tct gag act ctc tta aat ctg      432
Tyr His Asp Leu Ile His Ser Pro Ala Ser Glu Thr Leu Leu Asn Leu
    130                 135                 140

gaa cac aat tac ttt gtt agc atc tca gaa ttg att ggc gaa aga gat      480
Glu His Asn Tyr Phe Val Ser Ile Ser Glu Leu Ile Gly Glu Arg Asp
145                 150                 155                 160

gtg gag ctg aaa aag tta cga gag aga caa ggc att gaa atg gaa aag      528
Val Glu Leu Lys Lys Leu Arg Glu Arg Gln Gly Ile Glu Met Glu Lys
                165                 170                 175

gtg atg cag gaa ctg ggg aag tcg ctc aca gat caa gat gtg aac tca      576
Val Met Gln Glu Leu Gly Lys Ser Leu Thr Asp Gln Asp Val Asn Ser
            180                 185                 190

ctg gct gcc cag cac ttt gaa tcc cag cag gac tta gag aat aaa tgg      624
Leu Ala Ala Gln His Phe Glu Ser Gln Gln Asp Leu Glu Asn Lys Trp
        195                 200                 205

tca aac gaa ctg aaa cag tct aca gct atc cag aag caa gaa tat cag 672
Ser Asn Glu Leu Lys Gln Ser Thr Ala Ile Gln Lys Gln Glu Tyr Gln
    210                 215                 220

gaa tgg gtg ata aag ctt cat cag gac cta aaa aac ccc aac aac agc 720
Glu Trp Val Ile Lys Leu His Gln Asp Leu Lys Asn Pro Asn Asn Ser
225                 230                 235                 240

tcc ctc agt gaa gaa att aaa gtg cag ccg agt cag ttc agg gaa tct 768
Ser Leu Ser Glu Glu Ile Lys Val Gln Pro Ser Gln Phe Arg Glu Ser
                245                 250                 255

gcg gac gcc gct gga agg atc tat gag gag cag agg aag ctg gaa gaa 816
Ala Asp Ala Ala Gly Arg Ile Tyr Glu Glu Gln Arg Lys Leu Glu Glu
            260                 265                 270

agc ttc acc att cac ctg gga gcc cag ttg aag acc atg cac aat ctg 864
Ser Phe Thr Ile His Leu Gly Ala Gln Leu Lys Thr Met His Asn Leu
        275                 280                 285

cgg ttg ctg agg gca gac atg ctg gac ttc tgt aag cac aag aga acg 912
Arg Leu Leu Arg Ala Asp Met Leu Asp Phe Cys Lys His Lys Arg Thr
    290                 295                 300

cag ggg agc ggc gtg aag ctc cac cgg ctc cag acg gca ctg tcc ctt 960
Gln Gly Ser Gly Val Lys Leu His Arg Leu Gln Thr Ala Leu Ser Leu
305                 310                 315                 320

tac tcc acg tcc ctc tgt ggc ctg gtt tta cta gta gac aat cgg att 1008
Tyr Ser Thr Ser Leu Cys Gly Leu Val Leu Leu Val Asp Asn Arg Ile
                325                 330                 335

aat tca tat agc ggc atc aaa aga gat ttt gcc aca gtt tgc caa gaa 1056
Asn Ser Tyr Ser Gly Ile Lys Arg Asp Phe Ala Thr Val Cys Gln Glu
            340                 345                 350

tgc acg gac ttc cac ttc ccg cgg att gag gag caa ctg gag gtt gtc 1104
Cys Thr Asp Phe His Phe Pro Arg Ile Glu Glu Gln Leu Glu Val Val
        355                 360                 365

cag cag gtg gcg ctg tac gca cga acc cag cgc agg agc aag tgc aag 1152
Gln Gln Val Ala Leu Tyr Ala Arg Thr Gln Arg Arg Ser Lys Cys Lys
    370                 375                 380

gag gca cgc gat tct gga aac caa aat gga gga agt gat gag aaa tct 1200
Glu Ala Arg Asp Ser Gly Asn Gln Asn Gly Gly Ser Asp Glu Lys Ser
385                 390                 395                 400

aag aat gct gag aga aac tat tta aat att tta cca gga gaa ttt tat 1248
Lys Asn Ala Glu Arg Asn Tyr Leu Asn Ile Leu Pro Gly Glu Phe Tyr
                405                 410                 415

att aca cgt cat tct aat ctc tcg gaa atc cac gtt gct ttc cat ctc 1296
Ile Thr Arg His Ser Asn Leu Ser Glu Ile His Val Ala Phe His Leu
            420                 425                 430

tgt gtg gat gac aac gtg aag tca gga aac atc act gct cgg gat ccc 1344
Cys Val Asp Asp Asn Val Lys Ser Gly Asn Ile Thr Ala Arg Asp Pro
        435                 440                 445

gct att atg gga ctc cga aat ata ctc aag gtt tgc tgt acc cac gat 1392
Ala Ile Met Gly Leu Arg Asn Ile Leu Lys Val Cys Cys Thr His Asp
    450                 455                 460

atc aca acg ata agc atc cct ctc ttg ctg gtg cat gat atg tca gag 1440
Ile Thr Thr Ile Ser Ile Pro Leu Leu Leu Val His Asp Met Ser Glu
465                 470                 475                 480

gaa atg act ata cct tgg tgc tta agg aga gct gaa ctg gtg ttt aag 1488
Glu Met Thr Ile Pro Trp Cys Leu Arg Arg Ala Glu Leu Val Phe Lys
                485                 490                 495

tgt gtc aaa ggt ttc atg atg gag atg gct tca tgg gat gga gga att 1536
Cys Val Lys Gly Phe Met Met Glu Met Ala Ser Trp Asp Gly Gly Ile
            500                 505                 510

tct cgg aca gtg caa ttt ctg gta cca cag agc att tct gaa gaa atg 1584
Ser Arg Thr Val Gln Phe Leu Val Pro Gln Ser Ile Ser Glu Glu Met
        515                 520                 525

ttt tat cag ctg agt aac atg ctc cca cag atc ttc cgg gtc tcc tca 1632
Phe Tyr Gln Leu Ser Asn Met Leu Pro Gln Ile Phe Arg Val Ser Ser
    530                 535                 540

aca ctc act ctg aca tcc aag cac taa 1659
Thr Leu Thr Leu Thr Ser Lys His
545                 550

<210> 33
<211> 552
<212> PRT
<213> Rattus norvegicus

<400> 33

Met Lys Lys Asn Arg Glu Arg Phe Ser Ser Lys Glu Arg Glu Phe Val
1               5                   10                  15

Tyr Lys Phe Gln Ile Gly Ser Glu Arg Leu Glu Leu Arg Val Pro Leu
            20                  25                  30

Arg Phe Pro Val Asp Glu Asn Ala Ser His Leu His Gly Arg Leu Met
        35                  40                  45

Leu Leu His Ser Leu Pro Cys Phe Ile Glu Asn Asp Leu Lys Glu Ala
    50                  55                  60

Leu Ala Arg Phe Ile Glu Glu Glu Ser Leu Arg Asp His Asp Ser Asp
65                  70                  75                  80

Ala Glu Ala Cys Leu Glu Ala Val Lys Ser Gly Glu Val Asp Leu His
                85                  90                  95

Gln Leu Ala Ser Thr Trp Ala Lys Ala Tyr Ala Glu Thr Thr Leu Glu
            100                 105                 110

His Ala Arg Pro Glu Glu Pro Asn Trp Asp Glu Asp Phe Ala Asp Val
        115                 120                 125

Tyr His Asp Leu Ile His Ser Pro Ala Ser Glu Thr Leu Leu Asn Leu

130                 135                 140

Glu His Asn Tyr Phe Val Ser Ile Ser Glu Leu Ile Gly Glu Arg Asp
145                 150                 155                 160

Val Glu Leu Lys Lys Leu Arg Glu Arg Gln Gly Ile Glu Met Glu Lys
                165                 170                 175

Val Met Gln Glu Leu Gly Lys Ser Leu Thr Asp Gln Asp Val Asn Ser
            180                 185                 190

Leu Ala Ala Gln His Phe Glu Ser Gln Gln Asp Leu Glu Asn Lys Trp
        195                 200                 205

Ser Asn Glu Leu Lys Gln Ser Thr Ala Ile Gln Lys Gln Glu Tyr Gln
    210                 215                 220

Glu Trp Val Ile Lys Leu His Gln Asp Leu Lys Asn Pro Asn Asn Ser
225                 230                 235                 240

Ser Leu Ser Glu Glu Ile Lys Val Gln Pro Ser Gln Phe Arg Glu Ser
                245                 250                 255

Ala Asp Ala Ala Gly Arg Ile Tyr Glu Glu Gln Arg Lys Leu Glu Glu
            260                 265                 270

Ser Phe Thr Ile His Leu Gly Ala Gln Leu Lys Thr Met His Asn Leu
        275                 280                 285

Arg Leu Leu Arg Ala Asp Met Leu Asp Phe Cys Lys His Lys Arg Thr
    290                 295                 300

Gln Gly Ser Gly Val Lys Leu His Arg Leu Gln Thr Ala Leu Ser Leu
305                 310                 315                 320

Tyr Ser Thr Ser Leu Cys Gly Leu Val Leu Leu Val Asp Asn Arg Ile
                325                 330                 335

Asn Ser Tyr Ser Gly Ile Lys Arg Asp Phe Ala Thr Val Cys Gln Glu
            340                 345                 350

Cys Thr Asp Phe His Phe Pro Arg Ile Glu Glu Gln Leu Glu Val Val
        355                 360                 365

Gln Gln Val Ala Leu Tyr Ala Arg Thr Gln Arg Arg Ser Lys Cys Lys
    370                 375                 380

Glu Ala Arg Asp Ser Gly Asn Gln Asn Gly Gly Ser Asp Glu Lys Ser
385                 390                 395                 400

Lys Asn Ala Glu Arg Asn Tyr Leu Asn Ile Leu Pro Gly Glu Phe Tyr
                405                 410                 415

Ile Thr Arg His Ser Asn Leu Ser Glu Ile His Val Ala Phe His Leu
            420                 425                 430

Cys Val Asp Asp Asn Val Lys Ser Gly Asn Ile Thr Ala Arg Asp Pro
        435                 440                 445

Ala Ile Met Gly Leu Arg Asn Ile Leu Lys Val Cys Cys Thr His Asp
    450                 455                 460

Ile Thr Thr Ile Ser Ile Pro Leu Leu Leu Val His Asp Met Ser Glu
465                 470                 475                 480

Glu Met Thr Ile Pro Trp Cys Leu Arg Arg Ala Glu Leu Val Phe Lys
                485                 490                 495

Cys Val Lys Gly Phe Met Met Glu Met Ala Ser Trp Asp Gly Gly Ile
            500                 505                 510

Ser Arg Thr Val Gln Phe Leu Val Pro Gln Ser Ile Ser Glu Glu Met
        515                 520                 525

Phe Tyr Gln Leu Ser Asn Met Leu Pro Gln Ile Phe Arg Val Ser Ser
    530                 535                 540

Thr Leu Thr Leu Thr Ser Lys His
545                 550

<210>  34
<211>  1659
<212>  DNA
<213>  Homo sapiens

<220>
<221>  CDS
<222>  (1)..(1659)
<223>  Protein C12ORF4

<400>  34
atg aag aaa aac aga gaa aga ttc tgc aat aga gag aga gaa ttt gta      48
tat aaa ttt aaa gta gga agt cag tgc tta gaa ctg aga gtg cca ctc      96
aaa ttt cct gtt caa gag aat gcc agt cat ttg cat gga cgt ctg atg      144
ctg ctg cac agt tta ccg tgc ttt ata gaa aaa gac tta aaa gaa gct 192
ctg act cag ttt ata gaa gaa gaa tcc ctc agc gat tat gat aga gat 240
gct gaa gca tcc ctg gca gct gtg aaa tca ggt gaa gta gat tta cat 288
cag ctg gcg agt aca tgg gcc aaa gct tat gct gag acc acg tta gag 336
cat gca agg cct gaa gaa ccc agc tgg gat gaa gat ttt gca gat gtg 384
tac cat gac tta att cat tct cct gcc tct gaa act ctc tta aat ttg 432
gaa cat aat tac ttt gtt agt atc tca gaa ctg att ggt gaa aga gat 480
gtg gag ctg aaa aaa tta cga gag aga caa ggt att gaa atg gaa aaa 528
gtc atg cag gaa ttg gga aaa tca ctg aca gat caa gat gta aat tca 576
ctg gct gct cag cat ttt gaa tcc cag caa gac cta gaa aat aaa tgg 624
tcg aat gaa tta aaa caa tca act gcc atc caa aaa caa gag tat caa 672
gaa tgg gta ata aaa ctt cac caa gac cta aaa aac ccc aac aac agc 720
tcc ctt agt gag gaa att aaa gtt cag cca agt cag ttc aga gaa tct 768
gta gaa gca att gga agg att tat gag gaa cag aga aag tta gaa gaa 816
agt ttt acc att cac tta gga gcc cag ttg aag acc atg cat aat ttg 864
aga ttg ctg aga gca gat atg ctg gac ttc tgt aag cat aaa aga aat 912
cat cga agt ggt gtg aaa ctt cat cgg ctc caa aca gct ctg tca ctt 960
tat tct aca tct ctc tgt ggc ctg gtt tta cta gta gat aat cga att 1008
aat tca tat agt ggt att aaa aga gat ttt gcc aca gtt tgc caa gaa 1056
tgc act gac ttc cat ttc ccc cga att gaa gag caa tta gaa gtt gtc 1104
caa cag gtg gta ctt tat gct aga acc cag cgc agg agt aaa ttg aaa 1152
gaa tca ctt gat tct gga aac caa aat gga gga aat gat gat aag act 1200
aag aat gct gag agg aac tat tta aat gtt tta cct ggg gaa ttt tat 1248
att aca cgg cat tct aat ctc tca gaa atc cat gtt gct ttc cat ctc 1296
tgt gtg gat gac cat gtg aaa tcg gga aac atc act gct cgt gat cct 1344
gcc att atg gga ctc cga aat ata ctc aaa gtt tgc tgt acc cat gac 1392
atc aca aca ata agc att cct ctc ttg ctg gta cat gat atg tca gag 1440
gaa atg act ata ccc tgg tgc tta agg aga gcg gaa ctt gtg ttc aag 1488
tgt gtc aaa ggt ttc atg atg gaa atg gct tca tgg gat gga gga att 1536
tct agg aca gtg caa ttt cta gta cca cag agt att tct gaa gaa atg 1584
ttt tat caa ctt agt aac atg ctt ccc cag atc ttc cga gta tca tca 1632
aca ctc act ctg aca tcc aag cac taa 1659

<210>  35
<211>  552
<212>  PRT
<213>  Homo sapiens

<400>  35

<210>  36
<211>  1659
<212>  DNA
<213>  Macaca mulatta

<220>
<221>  CDS
<222>  (1)..(1659)
<223>  Protein LOC57102

<400>  36
atg aag aaa aac aga gaa aga ttc tgc aat aga gag aga gaa ttt gta        48
Met Lys Lys Asn Arg Glu Arg Phe Cys Asn Arg Glu Arg Glu Phe Val
1               5                   10                  15

tat aaa ttt aaa gta gga agt cag tgc tta gaa ctg aga gtg cca ctc        96
Tyr Lys Phe Lys Val Gly Ser Gln Cys Leu Glu Leu Arg Val Pro Leu
            20                  25                  30

aga ttt cct gtt caa gag aat gcc agt cat tta cat gga cgt ctg atg       144
Arg Phe Pro Val Gln Glu Asn Ala Ser His Leu His Gly Arg Leu Met
        35                  40                  45

ctg ctg cac agt tta ccg tgc ttt ata gaa aaa gac tta aaa gaa gct       192
Leu Leu His Ser Leu Pro Cys Phe Ile Glu Lys Asp Leu Lys Glu Ala
    50                  55                  60

ctg act caa ttt ata gaa gaa gaa tcc ctc aga gat tat gat aga gat       240
Leu Thr Gln Phe Ile Glu Glu Glu Ser Leu Arg Asp Tyr Asp Arg Asp
65                  70                  75                  80

gct gaa gca tcc ctg gaa gct gtg aaa tca ggt gag gtg gat tta cat       288
Ala Glu Ala Ser Leu Glu Ala Val Lys Ser Gly Glu Val Asp Leu His
                85                  90                  95

cag ctg gcg agt acg tgg gcc aaa gct tat gcg gag acc aca tta gag       336
Gln Leu Ala Ser Thr Trp Ala Lys Ala Tyr Ala Glu Thr Thr Leu Glu
            100                 105                 110

cat gcg agg cct gaa gaa ccc agc tgg gat gaa gat ttt gca gat gtg       384
His Ala Arg Pro Glu Glu Pro Ser Trp Asp Glu Asp Phe Ala Asp Val
        115                 120                 125

tac cat gac cta att cat tct cct gcc tct gaa acg ctc tta aat ttg       432
Tyr His Asp Leu Ile His Ser Pro Ala Ser Glu Thr Leu Leu Asn Leu
    130                 135                 140

gaa cat aat tac ttt gtt agt atc tca gaa ctg att ggt gag aga gat 480
Glu His Asn Tyr Phe Val Ser Ile Ser Glu Leu Ile Gly Glu Arg Asp
145                 150                 155                 160

gtg gaa ctg aaa aaa tta cga gag aga caa ggt att gaa atg gaa aaa 528
Val Glu Leu Lys Lys Leu Arg Glu Arg Gln Gly Ile Glu Met Glu Lys
                165                 170                 175

gta atg cag gag ttg gga aaa tca ctg aca gat caa gat gta aat tca 576
Val Met Gln Glu Leu Gly Lys Ser Leu Thr Asp Gln Asp Val Asn Ser
            180                 185                 190

ctg gct gct cag cat ttt gaa tcc cag caa gac cta gaa aat aaa tgg 624
Leu Ala Ala Gln His Phe Glu Ser Gln Gln Asp Leu Glu Asn Lys Trp
        195                 200                 205

tcg aat gaa tta aaa caa tca act gcc atc caa aaa caa gag tat cag 672
Ser Asn Glu Leu Lys Gln Ser Thr Ala Ile Gln Lys Gln Glu Tyr Gln
    210                 215                 220

gaa tgg gta ata aaa ctt cac caa gac cta aaa aac ccc aac aac agc 720
Glu Trp Val Ile Lys Leu His Gln Asp Leu Lys Asn Pro Asn Asn Ser
225                 230                 235                 240

tcc ctt agt gag gaa att aaa gtt cag cca agt cag ttc aga gaa tct 768
Ser Leu Ser Glu Glu Ile Lys Val Gln Pro Ser Gln Phe Arg Glu Ser
                245                 250                 255

gta gaa gca att gga agg att tat gag gaa cag aga aag tta gaa gaa 816
Val Glu Ala Ile Gly Arg Ile Tyr Glu Glu Gln Arg Lys Leu Glu Glu
            260                 265                 270

agt ttt acc att cac tta gga gcc cag tta aag acc atg cat aat ttg 864
Ser Phe Thr Ile His Leu Gly Ala Gln Leu Lys Thr Met His Asn Leu
        275                 280                 285

aga ttg ctg aga gca gat atg ctg gac ttc tgt aag cat aaa aga aat 912
Arg Leu Leu Arg Ala Asp Met Leu Asp Phe Cys Lys His Lys Arg Asn
    290                 295                 300

cat cga agt ggt gtg aaa ctt cat cgg ctc caa aca gct ctg tca ctt 960
His Arg Ser Gly Val Lys Leu His Arg Leu Gln Thr Ala Leu Ser Leu
305                 310                 315                 320

tat tct aca tct ctc tgt ggc ctg gtt tta cta gta gat aat cga att 1008
Tyr Ser Thr Ser Leu Cys Gly Leu Val Leu Leu Val Asp Asn Arg Ile
                325                 330                 335

aat tca tat agt ggt att aaa aga gat ttt gcc aca gtt tgc caa gaa 1056
Asn Ser Tyr Ser Gly Ile Lys Arg Asp Phe Ala Thr Val Cys Gln Glu
            340                 345                 350

tgc act gac ttc cat ttt ccc cga att gaa gag caa tta gaa gtt gtc 1104
Cys Thr Asp Phe His Phe Pro Arg Ile Glu Glu Gln Leu Glu Val Val
        355                 360                 365

caa cag gtg gta ctt tat gcc aga acc cag cgc agg agt aag ttg aaa 1152
Gln Gln Val Val Leu Tyr Ala Arg Thr Gln Arg Arg Ser Lys Leu Lys
    370                 375                 380

gaa tca ctt gat tct ggg aac caa aat gga gga aat gat gat aag act 1200
Glu Ser Leu Asp Ser Gly Asn Gln Asn Gly Gly Asn Asp Asp Lys Thr
385                 390                 395                 400

aag aat gct gag agg aac tat tta aat gtt tta cct ggg gaa ttt tat 1248

Lys Asn Ala Glu Arg Asn Tyr Leu Asn Val Leu Pro Gly Glu Phe Tyr
                405                 410                 415

att aca cgg cat tcg aat ctc tca gaa atc cat gtt gct ttc cat ctt 1296
Ile Thr Arg His Ser Asn Leu Ser Glu Ile His Val Ala Phe His Leu
            420                 425                 430

tgt gtg gat gac cat gtg aaa tcg gga aac atc act gct cgt gat cct 1344
Cys Val Asp Asp His Val Lys Ser Gly Asn Ile Thr Ala Arg Asp Pro
        435                 440                 445

gcc att atg gga ctc cga aat ata ctc aaa gtt tgc tgt acc cat gac 1392
Ala Ile Met Gly Leu Arg Asn Ile Leu Lys Val Cys Cys Thr His Asp
    450                 455                 460

atc aca aca ata agc att cct ctc ttg ctg gta cat gat atg tca gag 1440
Ile Thr Thr Ile Ser Ile Pro Leu Leu Leu Val His Asp Met Ser Glu
465                 470                 475                 480

gaa atg act ata ccc tgg tgc tta agg aga gca gaa cta gtg ttc aag 1488
Glu Met Thr Ile Pro Trp Cys Leu Arg Arg Ala Glu Leu Val Phe Lys
                485                 490                 495

tgt gtc aaa ggt ttc atg atg gaa atg gct tca tgg gat gga gga att 1536
Cys Val Lys Gly Phe Met Met Glu Met Ala Ser Trp Asp Gly Gly Ile
            500                 505                 510

tct agg aca gtg cag ttt cta gta cca cag agt att tct gaa gaa atg 1584
Ser Arg Thr Val Gln Phe Leu Val Pro Gln Ser Ile Ser Glu Glu Met
        515                 520                 525

ttt tat caa ctt agt aac atg ctt ccc cag att ttc cga gta tca tca 1632
Phe Tyr Gln Leu Ser Asn Met Leu Pro Gln Ile Phe Arg Val Ser Ser
    530                 535                 540

aca ctc act ctg aca tcc aag cac taa 1659
Thr Leu Thr Leu Thr Ser Lys His
545                 550

<210> 37
<211> 552
<212> PRT
<213> Macaca mulatta

<400> 37

Met Lys Lys Asn Arg Glu Arg Phe Cys Asn Arg Glu Arg Glu Phe Val
1               5                   10                  15

Tyr Lys Phe Lys Val Gly Ser Gln Cys Leu Glu Leu Arg Val Pro Leu
            20                  25                  30

Arg Phe Pro Val Gln Glu Asn Ala Ser His Leu His Gly Arg Leu Met
        35                  40                  45

Leu Leu His Ser Leu Pro Cys Phe Ile Glu Lys Asp Leu Lys Glu Ala
    50                  55                  60

Leu Thr Gln Phe Ile Glu Glu Glu Ser Leu Arg Asp Tyr Asp Arg Asp
65                  70                  75                  80

Ala Glu Ala Ser Leu Glu Ala Val Lys Ser Gly Glu Val Asp Leu His
                85                  90                  95

Gln Leu Ala Ser Thr Trp Ala Lys Ala Tyr Ala Glu Thr Thr Leu Glu
            100                 105                 110

His Ala Arg Pro Glu Glu Pro Ser Trp Asp Glu Asp Phe Ala Asp Val
        115                 120                 125

Tyr His Asp Leu Ile His Ser Pro Ala Ser Glu Thr Leu Leu Asn Leu
    130                 135                 140

Glu His Asn Tyr Phe Val Ser Ile Ser Glu Leu Ile Gly Glu Arg Asp
145                 150                 155                 160

Val Glu Leu Lys Lys Leu Arg Glu Arg Gln Gly Ile Glu Met Glu Lys
                165                 170                 175

Val Met Gln Glu Leu Gly Lys Ser Leu Thr Asp Gln Asp Val Asn Ser
            180                 185                 190

Leu Ala Ala Gln His Phe Glu Ser Gln Gln Asp Leu Glu Asn Lys Trp
        195                 200                 205

Ser Asn Glu Leu Lys Gln Ser Thr Ala Ile Gln Lys Gln Glu Tyr Gln
    210                 215                 220

Glu Trp Val Ile Lys Leu His Gln Asp Leu Lys Asn Pro Asn Asn Ser
225                 230                 235                 240

Ser Leu Ser Glu Glu Ile Lys Val Gln Pro Ser Gln Phe Arg Glu Ser
                245                 250                 255

Val Glu Ala Ile Gly Arg Ile Tyr Glu Glu Gln Arg Lys Leu Glu Glu
            260                 265                 270

Ser Phe Thr Ile His Leu Gly Ala Gln Leu Lys Thr Met His Asn Leu
        275                 280                 285

Arg Leu Leu Arg Ala Asp Met Leu Asp Phe Cys Lys His Lys Arg Asn
    290                 295                 300

His Arg Ser Gly Val Lys Leu His Arg Leu Gln Thr Ala Leu Ser Leu
305                 310                 315                 320

Tyr Ser Thr Ser Leu Cys Gly Leu Val Leu Leu Val Asp Asn Arg Ile
                325                 330                 335

Asn Ser Tyr Ser Gly Ile Lys Arg Asp Phe Ala Thr Val Cys Gln Glu
            340                 345                 350

Cys Thr Asp Phe His Phe Pro Arg Ile Glu Glu Gln Leu Glu Val Val
        355                 360                 365

Gln Gln Val Val Leu Tyr Ala Arg Thr Gln Arg Arg Ser Lys Leu Lys
    370                 375                 380

Glu Ser Leu Asp Ser Gly Asn Gln Asn Gly Gly Asn Asp Asp Lys Thr
385                 390                 395                 400

Lys Asn Ala Glu Arg Asn Tyr Leu Asn Val Leu Pro Gly Glu Phe Tyr
                405                 410                 415

Ile Thr Arg His Ser Asn Leu Ser Glu Ile His Val Ala Phe His Leu
            420                 425                 430

Cys Val Asp Asp His Val Lys Ser Gly Asn Ile Thr Ala Arg Asp Pro
        435                 440                 445

Ala Ile Met Gly Leu Arg Asn Ile Leu Lys Val Cys Cys Thr His Asp
    450                 455                 460

Ile Thr Thr Ile Ser Ile Pro Leu Leu Leu Val His Asp Met Ser Glu
465                 470                 475                 480

Glu Met Thr Ile Pro Trp Cys Leu Arg Arg Ala Glu Leu Val Phe Lys
                485                 490                 495

Cys Val Lys Gly Phe Met Met Glu Met Ala Ser Trp Asp Gly Gly Ile
            500                 505                 510

Ser Arg Thr Val Gln Phe Leu Val Pro Gln Ser Ile Ser Glu Glu Met
        515                 520                 525

Phe Tyr Gln Leu Ser Asn Met Leu Pro Gln Ile Phe Arg Val Ser Ser
    530                 535                 540

Thr Leu Thr Leu Thr Ser Lys His
545                 550

<210>  38
<211>  1659
<212>  DNA
<213>  Mus musculus

<220>
<221>  CDS

<222>  (1)..(1659)
<223>  Protein LOC28040

<400>  38
atg aag aaa aac cga gaa agg ttc tgc aac aag gaa agg gaa ttt gtg      48
Met Lys Lys Asn Arg Glu Arg Phe Cys Asn Lys Glu Arg Glu Phe Val
1               5                   10                  15

tac aag ttc cag gta gga cgt gag cgc tta gag ctg aga gtg ccc ctc      96
Tyr Lys Phe Gln Val Gly Arg Glu Arg Leu Glu Leu Arg Val Pro Leu
            20                  25                  30

agg ttt cct gtg gag gag aat gcc agt cat ctg cat gga cgt ctg atg      144
Arg Phe Pro Val Glu Glu Asn Ala Ser His Leu His Gly Arg Leu Met
        35                  40                  45

ctg ctg cac agc ttg cct tgc ttc ata gag agt gac tta aaa gat gct      192
Leu Leu His Ser Leu Pro Cys Phe Ile Glu Ser Asp Leu Lys Asp Ala
    50                  55                  60

ctg agc cgg ttt ata gaa gaa gaa tcc ctc aga gac cat gac agc gat      240
Leu Ser Arg Phe Ile Glu Glu Glu Ser Leu Arg Asp His Asp Ser Asp
65                  70                  75                  80

gca gaa gca tgc ttg gag gcc gtt aaa tcc ggt gaa gta gat ctg cac      288
Ala Glu Ala Cys Leu Glu Ala Val Lys Ser Gly Glu Val Asp Leu His
                85                  90                  95

cag ctg gca agt gcg tgg gcc aaa gct tac gct gag acc acc tta gag      336
Gln Leu Ala Ser Ala Trp Ala Lys Ala Tyr Ala Glu Thr Thr Leu Glu
            100                 105                 110

cat gcg aga cct gag gag cct gac tgg gac gaa gac ttt gca gat gtg      384
His Ala Arg Pro Glu Glu Pro Asp Trp Asp Glu Asp Phe Ala Asp Val
        115                 120                 125

tac cat gac cta atc cac tcc ccg gcc tct gaa act ctc tta aat ctg      432
Tyr His Asp Leu Ile His Ser Pro Ala Ser Glu Thr Leu Leu Asn Leu
    130                 135                 140

gaa cac aac tac ttc gtt agc atc tca gag ctg att ggt gaa aga gac      480
Glu His Asn Tyr Phe Val Ser Ile Ser Glu Leu Ile Gly Glu Arg Asp
145                 150                 155                 160

gtg gag ctg aaa aag tta cga gag aga caa ggt att gaa atg gaa aag      528
Val Glu Leu Lys Lys Leu Arg Glu Arg Gln Gly Ile Glu Met Glu Lys
                165                 170                 175

gtg atg cag gag ctg ggg aaa tcg ctc aca gat caa gat gtg aat tca      576
Val Met Gln Glu Leu Gly Lys Ser Leu Thr Asp Gln Asp Val Asn Ser
            180                 185                 190

ctg gct gcc cag cac ttt gaa tcc cag caa gac tta gag aat aaa tgg      624
Leu Ala Ala Gln His Phe Glu Ser Gln Gln Asp Leu Glu Asn Lys Trp
        195                 200                 205

tca aat gaa ctg aaa cag tct aca gct atc cag aag caa gaa tat cag      672
Ser Asn Glu Leu Lys Gln Ser Thr Ala Ile Gln Lys Gln Glu Tyr Gln
    210                 215                 220

gaa tgg gtg ata aag ctt cat cag gac cta aaa aac ccc aac aac agc      720
Glu Trp Val Ile Lys Leu His Gln Asp Leu Lys Asn Pro Asn Asn Ser
225                 230                 235                 240

tcc ctc agt gaa gag att aaa gtg cag ccg agt cag ttc aga gag tct      768

Ser Leu Ser Glu Glu Ile Lys Val Gln Pro Ser Gln Phe Arg Glu Ser
                245                 250                 255

gcg gac gct gct ggg agg atc tac gag gaa cag agg aag ctg gag gag      816
Ala Asp Ala Ala Gly Arg Ile Tyr Glu Glu Gln Arg Lys Leu Glu Glu
            260                 265                 270

agc ttc acc att cac ctg gga gcc cag ttg aag acc atg cac aac ctg      864
Ser Phe Thr Ile His Leu Gly Ala Gln Leu Lys Thr Met His Asn Leu
        275                 280                 285

cgg ctg ctg agg gca gac atg ctg gac ttc tgt aag cat aag aga act      912
Arg Leu Leu Arg Ala Asp Met Leu Asp Phe Cys Lys His Lys Arg Thr
    290                 295                 300

cac ggg agc ggc gtg aag ctc cac cgt ctg cag aca gca ctg tcc ctc      960
His Gly Ser Gly Val Lys Leu His Arg Leu Gln Thr Ala Leu Ser Leu
305                 310                 315                 320

tac tcc acg tct ctc tgt ggc ctg gtt ttg cta gta gat aat cga att     1008
Tyr Ser Thr Ser Leu Cys Gly Leu Val Leu Leu Val Asp Asn Arg Ile
                325                 330                 335

aat tca tat agc ggc atc aaa aga gat ttt gcc aca gtt tgc caa gaa     1056
Asn Ser Tyr Ser Gly Ile Lys Arg Asp Phe Ala Thr Val Cys Gln Glu
            340                 345                 350

tgt acg gac ttc cac ttc cca cgg att gag gaa caa ctg gaa gtt gtc     1104
Cys Thr Asp Phe His Phe Pro Arg Ile Glu Glu Gln Leu Glu Val Val
        355                 360                 365

cag cag gtg gcg ctg tac gcg aga acc caa cgc aag agc aag tgc aaa     1152
Gln Gln Val Ala Leu Tyr Ala Arg Thr Gln Arg Lys Ser Lys Cys Lys
    370                 375                 380

gag gcc cgc gat tct gga aac caa aat gga gga agt gat gat aag tct     1200
Glu Ala Arg Asp Ser Gly Asn Gln Asn Gly Gly Ser Asp Asp Lys Ser
385                 390                 395                 400

aag aat gct gag aga aac tat tta aat att tta cct gga gaa ttt tac     1248
Lys Asn Ala Glu Arg Asn Tyr Leu Asn Ile Leu Pro Gly Glu Phe Tyr
                405                 410                 415

att aca cgt cat tct aat ctc tcg gaa atc cat gtt gct ttc cac ctc     1296
Ile Thr Arg His Ser Asn Leu Ser Glu Ile His Val Ala Phe His Leu
            420                 425                 430

tgt gtg gat gat aac gtg aag tcg gga aat atc act gct cgg gat ccc     1344
Cys Val Asp Asp Asn Val Lys Ser Gly Asn Ile Thr Ala Arg Asp Pro
        435                 440                 445

gct att atg gga ctc cga aac att ctc aaa gtc tgc tgt acc cat gat     1392
Ala Ile Met Gly Leu Arg Asn Ile Leu Lys Val Cys Cys Thr His Asp
    450                 455                 460

atc aca acg ata agc atc cct ctc ttg ctg gtg cat gat atg tca gag     1440
Ile Thr Thr Ile Ser Ile Pro Leu Leu Leu Val His Asp Met Ser Glu
465                 470                 475                 480

gaa atg act ata cct tgg tgt tta agg aga gca gaa ctg gta ttt aag     1488
Glu Met Thr Ile Pro Trp Cys Leu Arg Arg Ala Glu Leu Val Phe Lys
                485                 490                 495

tgt gtc aaa ggt ttc atg atg gag atg gct tca tgg gac gga gga att     1536
Cys Val Lys Gly Phe Met Met Glu Met Ala Ser Trp Asp Gly Gly Ile

500                 505                 510

tct cgg aca gtg caa ttt ctg gta cca cag agc att tct gaa gaa atg       1584
Ser Arg Thr Val Gln Phe Leu Val Pro Gln Ser Ile Ser Glu Glu Met
        515                 520                 525

ttt tat cag ctg agc aac atg ctt ccc cag atc ttc cgg gtc tcc tca       1632
Phe Tyr Gln Leu Ser Asn Met Leu Pro Gln Ile Phe Arg Val Ser Ser
    530                 535                 540

aca ctc acg ctg aca tcc aag cac taa                                   1659
Thr Leu Thr Leu Thr Ser Lys His
545                 550


<210>  39
<211>  552
<212>  PRT
<213>  Mus musculus

<400>  39

Met Lys Lys Asn Arg Glu Arg Phe Cys Asn Lys Glu Arg Glu Phe Val
1               5                   10                  15


Tyr Lys Phe Gln Val Gly Arg Glu Arg Leu Glu Leu Arg Val Pro Leu
            20                  25                  30


Arg Phe Pro Val Glu Glu Asn Ala Ser His Leu His Gly Arg Leu Met
        35                  40                  45


Leu Leu His Ser Leu Pro Cys Phe Ile Glu Ser Asp Leu Lys Asp Ala
    50                  55                  60


Leu Ser Arg Phe Ile Glu Glu Glu Ser Leu Arg Asp His Asp Ser Asp
65                  70                  75                  80


Ala Glu Ala Cys Leu Glu Ala Val Lys Ser Gly Glu Val Asp Leu His
                85                  90                  95


Gln Leu Ala Ser Ala Trp Ala Lys Ala Tyr Ala Glu Thr Thr Leu Glu
            100                 105                 110


His Ala Arg Pro Glu Glu Pro Asp Trp Asp Glu Asp Phe Ala Asp Val
        115                 120                 125


Tyr His Asp Leu Ile His Ser Pro Ala Ser Glu Thr Leu Leu Asn Leu
    130                 135                 140


Glu His Asn Tyr Phe Val Ser Ile Ser Glu Leu Ile Gly Glu Arg Asp
145                 150                 155                 160


Val Glu Leu Lys Lys Leu Arg Glu Arg Gln Gly Ile Glu Met Glu Lys
                165                 170                 175

Val Met Gln Glu Leu Gly Lys Ser Leu Thr Asp Gln Asp Val Asn Ser
            180                 185                 190

Leu Ala Ala Gln His Phe Glu Ser Gln Gln Asp Leu Glu Asn Lys Trp
        195                 200                 205

Ser Asn Glu Leu Lys Gln Ser Thr Ala Ile Gln Lys Gln Glu Tyr Gln
    210                 215                 220

Glu Trp Val Ile Lys Leu His Gln Asp Leu Lys Asn Pro Asn Asn Ser
225                 230                 235                 240

Ser Leu Ser Glu Glu Ile Lys Val Gln Pro Ser Gln Phe Arg Glu Ser
                245                 250                 255

Ala Asp Ala Ala Gly Arg Ile Tyr Glu Glu Gln Arg Lys Leu Glu Glu
            260                 265                 270

Ser Phe Thr Ile His Leu Gly Ala Gln Leu Lys Thr Met His Asn Leu
        275                 280                 285

Arg Leu Leu Arg Ala Asp Met Leu Asp Phe Cys Lys His Lys Arg Thr
    290                 295                 300

His Gly Ser Gly Val Lys Leu His Arg Leu Gln Thr Ala Leu Ser Leu
305                 310                 315                 320

Tyr Ser Thr Ser Leu Cys Gly Leu Val Leu Leu Val Asp Asn Arg Ile
                325                 330                 335

Asn Ser Tyr Ser Gly Ile Lys Arg Asp Phe Ala Thr Val Cys Gln Glu
            340                 345                 350

Cys Thr Asp Phe His Phe Pro Arg Ile Glu Glu Gln Leu Glu Val Val
        355                 360                 365

Gln Gln Val Ala Leu Tyr Ala Arg Thr Gln Arg Lys Ser Lys Cys Lys
    370                 375                 380

Glu Ala Arg Asp Ser Gly Asn Gln Asn Gly Gly Ser Asp Asp Lys Ser
385                 390                 395                 400

Lys Asn Ala Glu Arg Asn Tyr Leu Asn Ile Leu Pro Gly Glu Phe Tyr
                405                 410                 415

Ile Thr Arg His Ser Asn Leu Ser Glu Ile His Val Ala Phe His Leu
            420                 425                 430

Cys Val Asp Asp Asn Val Lys Ser Gly Asn Ile Thr Ala Arg Asp Pro
        435                 440                 445

Ala Ile Met Gly Leu Arg Asn Ile Leu Lys Val Cys Cys Thr His Asp
    450                 455                 460

Ile Thr Thr Ile Ser Ile Pro Leu Leu Leu Val His Asp Met Ser Glu
465                 470                 475                 480

Glu Met Thr Ile Pro Trp Cys Leu Arg Arg Ala Glu Leu Val Phe Lys
                485                 490                 495

Cys Val Lys Gly Phe Met Met Glu Met Ala Ser Trp Asp Gly Gly Ile
            500                 505                 510

Ser Arg Thr Val Gln Phe Leu Val Pro Gln Ser Ile Ser Glu Glu Met
        515                 520                 525

Phe Tyr Gln Leu Ser Asn Met Leu Pro Gln Ile Phe Arg Val Ser Ser
    530                 535                 540

Thr Leu Thr Leu Thr Ser Lys His
545                 550

<210> 40
<211> 2538
<212> DNA
<213> Homo sapiens

<220>
<221> CDS
<222> (1)..(2538)
<223> ABCF1 Protein

<400> 40
atg ccg aag gcg ccc aag cag cag ccg ccg gag ccc gag tgg atc ggg 48
Met Pro Lys Ala Pro Lys Gln Gln Pro Pro Glu Pro Glu Trp Ile Gly
1               5                   10                  15

gac gga gag agc acg agc cca tca gac aaa gtg gtg aag aaa ggg aag 96
Asp Gly Glu Ser Thr Ser Pro Ser Asp Lys Val Val Lys Lys Gly Lys
            20                  25                  30

aag gac aag aag atc aaa aaa acg ttc ttt gaa gag ctg gca gta gaa 144
Lys Asp Lys Lys Ile Lys Lys Thr Phe Phe Glu Glu Leu Ala Val Glu
        35                  40                  45

gat aaa cag gct ggg gaa gaa gag aaa gtg ctc aag gag aag gag cag 192
Asp Lys Gln Ala Gly Glu Glu Glu Lys Val Leu Lys Glu Lys Glu Gln
    50                  55                  60

cag cag cag caa cag caa cag cag caa aaa aaa aag cga gat acc cga 240
Gln Gln Gln Gln Gln Gln Gln Gln Gln Lys Lys Lys Arg Asp Thr Arg
65                  70                  75                  80

aaa ggc agg cgg aag aag gat gtg gat gat gat gga gaa gag aaa gag 288

Lys Gly Arg Arg Lys Lys Asp Val Asp Asp Asp Gly Glu Glu Lys Glu
                85                  90                  95

ctc atg gag cgt ctt aag aag ctc tca gtg cca acc agt gat gag gag      336
Leu Met Glu Arg Leu Lys Lys Leu Ser Val Pro Thr Ser Asp Glu Glu
            100                 105                 110

gat gaa gta ccc gcc cca aaa ccc cgc gga ggg aag aaa acc aag ggt      384
Asp Glu Val Pro Ala Pro Lys Pro Arg Gly Gly Lys Lys Thr Lys Gly
        115                 120                 125

ggt aat gtt ttt gca gcc ctg att cag gat cag agt gag gaa gag gag      432
Gly Asn Val Phe Ala Ala Leu Ile Gln Asp Gln Ser Glu Glu Glu Glu
    130                 135                 140

gag gaa gaa aaa cat cct cct aag cct gcc aag ccg gag aag aat cgg      480
Glu Glu Glu Lys His Pro Pro Lys Pro Ala Lys Pro Glu Lys Asn Arg
145                 150                 155                 160

atc aat aag gcc gta tct gag gaa cag cag cct gca ctc aag ggc aaa      528
Ile Asn Lys Ala Val Ser Glu Glu Gln Gln Pro Ala Leu Lys Gly Lys
                165                 170                 175

aag gga aag gaa gag aag tca aaa ggg aag gct aag cct caa aat aaa      576
Lys Gly Lys Glu Glu Lys Ser Lys Gly Lys Ala Lys Pro Gln Asn Lys
            180                 185                 190

ttc gct gct ctg gac aat gaa gag gag gat aaa gaa gaa gaa att ata      624
Phe Ala Ala Leu Asp Asn Glu Glu Glu Asp Lys Glu Glu Glu Ile Ile
        195                 200                 205

aag gaa aag gag cct ccc aaa caa ggg aag gag aag gcc aag aag gca      672
Lys Glu Lys Glu Pro Pro Lys Gln Gly Lys Glu Lys Ala Lys Lys Ala
    210                 215                 220

gag cag ggt tca gag gaa gaa gga gaa ggg gaa gaa gag gag gag gaa      720
Glu Gln Gly Ser Glu Glu Glu Gly Glu Gly Glu Glu Glu Glu Glu Glu
225                 230                 235                 240

gga gga gag tct aag gca gat gat ccc tat gct cat ctt agc aaa aag      768
Gly Gly Glu Ser Lys Ala Asp Asp Pro Tyr Ala His Leu Ser Lys Lys
                245                 250                 255

gag aag aaa aag ctg aaa aaa cag atg gag tat gag cgc caa gtg gct      816
Glu Lys Lys Lys Leu Lys Lys Gln Met Glu Tyr Glu Arg Gln Val Ala
            260                 265                 270

tca tta aaa gca gcc aat gca gct gaa aat gac ttc tcc gtg tcc cag      864
Ser Leu Lys Ala Ala Asn Ala Ala Glu Asn Asp Phe Ser Val Ser Gln
        275                 280                 285

gcg gag atg tcc tcc cgc caa gcc atg tta gaa aat gca tct gac atc      912
Ala Glu Met Ser Ser Arg Gln Ala Met Leu Glu Asn Ala Ser Asp Ile
    290                 295                 300

aag ctg gag aag ttc agc atc tcc gct cat ggc aag gag ctg ttc gtc      960
Lys Leu Glu Lys Phe Ser Ile Ser Ala His Gly Lys Glu Leu Phe Val
305                 310                 315                 320

aat gca gac ctg tac att gta gcc ggc cgc cgc tac ggg ctg gta gga     1008
Asn Ala Asp Leu Tyr Ile Val Ala Gly Arg Arg Tyr Gly Leu Val Gly
                325                 330                 335

ccc aat ggc aag ggc aag acc aca ctc ctc aag cac att gcc aac cga     1056
Pro Asn Gly Lys Gly Lys Thr Thr Leu Leu Lys His Ile Ala Asn Arg

340                 345                 350

gcc ctg agc atc cct ccc aac att gat gtg ttg ctg tgt gag cag gag        1104
Ala Leu Ser Ile Pro Pro Asn Ile Asp Val Leu Leu Cys Glu Gln Glu
        355                 360                 365

gtg gta gca gat gag aca cca gca gtc cag gct gtt ctt cga gct gac        1152
Val Val Ala Asp Glu Thr Pro Ala Val Gln Ala Val Leu Arg Ala Asp
    370                 375                 380

acc aag cga ttg aag ctg ctg gaa gag gag cgg cgg ctt cag gga cag        1200
Thr Lys Arg Leu Lys Leu Leu Glu Glu Glu Arg Arg Leu Gln Gly Gln
385                 390                 395                 400

ctg gaa caa ggg gat gac aca gct gct gag agg cta gag aag gtg tat        1248
Leu Glu Gln Gly Asp Asp Thr Ala Ala Glu Arg Leu Glu Lys Val Tyr
                405                 410                 415

gag gaa ttg cgg gcc act ggg gcg gca gct gca gag gcc aaa gca cgg        1296
Glu Glu Leu Arg Ala Thr Gly Ala Ala Ala Ala Glu Ala Lys Ala Arg
            420                 425                 430

cgg atc ctg gct ggc ctg ggc ttt gac cct gaa atg cag aat cga ccc        1344
Arg Ile Leu Ala Gly Leu Gly Phe Asp Pro Glu Met Gln Asn Arg Pro
        435                 440                 445

aca cag aag ttc tca ggg ggc tgg cgc atg cgt gtc tcc ctg gcc agg        1392
Thr Gln Lys Phe Ser Gly Gly Trp Arg Met Arg Val Ser Leu Ala Arg
    450                 455                 460

gca ctg ttc atg gag ccc aca ctg ctg atg ctg gat gag ccc acc aac        1440
Ala Leu Phe Met Glu Pro Thr Leu Leu Met Leu Asp Glu Pro Thr Asn
465                 470                 475                 480

cac ctg gac ctc aac gct gtc atc tgg ctt aat aac tac ctc cag ggc        1488
His Leu Asp Leu Asn Ala Val Ile Trp Leu Asn Asn Tyr Leu Gln Gly
                485                 490                 495

tgg cgg aag acc ttg ctg atc gtc tcc cat gac cag ggc ttc ttg gat        1536
Trp Arg Lys Thr Leu Leu Ile Val Ser His Asp Gln Gly Phe Leu Asp
            500                 505                 510

gat gtc tgc act gat atc atc cac ctc gat gcc cag cgg ctc cac tac        1584
Asp Val Cys Thr Asp Ile Ile His Leu Asp Ala Gln Arg Leu His Tyr
        515                 520                 525

tat agg ggc aat tac atg acc ttc aaa aag atg tac cag cag aag cag        1632
Tyr Arg Gly Asn Tyr Met Thr Phe Lys Lys Met Tyr Gln Gln Lys Gln
    530                 535                 540

aaa gaa ctg ctg aaa cag tat gag aag caa gag aaa aag ctg aag gag        1680
Lys Glu Leu Leu Lys Gln Tyr Glu Lys Gln Glu Lys Lys Leu Lys Glu
545                 550                 555                 560

ctg aag gca ggc ggg aag tcc acc aag cag gcg gaa aaa caa acg aag        1728
Leu Lys Ala Gly Gly Lys Ser Thr Lys Gln Ala Glu Lys Gln Thr Lys
                565                 570                 575

gaa gcc ctg act cgg aag cag cag aaa tgc cga cgg aaa aac caa gat        1776
Glu Ala Leu Thr Arg Lys Gln Gln Lys Cys Arg Arg Lys Asn Gln Asp
            580                 585                 590

gag gaa tcc cag gag gcc cct gag ctc ctg aag cgc cct aag gag tac        1824
Glu Glu Ser Gln Glu Ala Pro Glu Leu Leu Lys Arg Pro Lys Glu Tyr
        595                 600                 605

act gtg cgc ttc act ttt cca gac ccc cca cca ctc agc cct cca gtg 1872
Thr Val Arg Phe Thr Phe Pro Asp Pro Pro Pro Leu Ser Pro Pro Val
    610                 615                 620

ctg ggt ctg cat ggt gtg aca ttc ggc tac cag gga cag aaa cca ctc 1920
Leu Gly Leu His Gly Val Thr Phe Gly Tyr Gln Gly Gln Lys Pro Leu
625                 630                 635                 640

ttt aag aac ttg gat ttt ggc atc gac atg gat tca agg att tgc att 1968
Phe Lys Asn Leu Asp Phe Gly Ile Asp Met Asp Ser Arg Ile Cys Ile
                645                 650                 655

gtg ggc cct aat ggt gtg ggg aag agt acg cta ctc ctg ctg ctg act 2016
Val Gly Pro Asn Gly Val Gly Lys Ser Thr Leu Leu Leu Leu Leu Thr
            660                 665                 670

ggc aag ctg aca ccg acc cat ggg gaa atg aga aag aac cac cgg ctg 2064
Gly Lys Leu Thr Pro Thr His Gly Glu Met Arg Lys Asn His Arg Leu
        675                 680                 685

aaa att ggc ttc ttc aac cag cag tat gca gag cag ctg cgc atg gag 2112
Lys Ile Gly Phe Phe Asn Gln Gln Tyr Ala Glu Gln Leu Arg Met Glu
    690                 695                 700

gag acg ccc act gag tac ctg cag cgg ggc ttc aac ctg ccc tac cag 2160
Glu Thr Pro Thr Glu Tyr Leu Gln Arg Gly Phe Asn Leu Pro Tyr Gln
705                 710                 715                 720

gat gcc cgc aag tgc ctg ggc cgc ttc ggc ctg gag agt cac gcc cac 2208
Asp Ala Arg Lys Cys Leu Gly Arg Phe Gly Leu Glu Ser His Ala His
                725                 730                 735

acc atc cag atc tgc aaa ctc tct ggt ggt cag aag gcg cga gtt gtg 2256
Thr Ile Gln Ile Cys Lys Leu Ser Gly Gly Gln Lys Ala Arg Val Val
            740                 745                 750

ttt gct gag ctg gcc tgt cgg gaa cct gat gtc ctc atc ttg gac gag 2304
Phe Ala Glu Leu Ala Cys Arg Glu Pro Asp Val Leu Ile Leu Asp Glu
        755                 760                 765

cca acc aat aac ctg gac ata gag tct att gat gct cta ggg gag gcc 2352
Pro Thr Asn Asn Leu Asp Ile Glu Ser Ile Asp Ala Leu Gly Glu Ala
    770                 775                 780

atc aat gaa tac aag ggt gct gtg atc gtt gtc agc cat gat gcc cga 2400
Ile Asn Glu Tyr Lys Gly Ala Val Ile Val Val Ser His Asp Ala Arg
785                 790                 795                 800

ctc atc aca gaa acc aat tgc cag ctg tgg gtg gtg gag gag cag agt 2448
Leu Ile Thr Glu Thr Asn Cys Gln Leu Trp Val Val Glu Glu Gln Ser
                805                 810                 815

gtt agc caa atc gat ggt gac ttt gaa gac tac aag cgg gag gtg ttg 2496
Val Ser Gln Ile Asp Gly Asp Phe Glu Asp Tyr Lys Arg Glu Val Leu
            820                 825                 830

gag gcc ctg ggt gaa gtc atg gtc agc cgg ccc cga gag tga 2538
Glu Ala Leu Gly Glu Val Met Val Ser Arg Pro Arg Glu
        835                 840                 845

<210> 41
<211> 845
<212> PRT

<213>  Homo sapiens

<400>  41

Met Pro Lys Ala Pro Lys Gln Gln Pro Pro Glu Pro Glu Trp Ile Gly
1               5                   10                  15

Asp Gly Glu Ser Thr Ser Pro Ser Asp Lys Val Val Lys Lys Gly Lys
            20                  25                  30

Lys Asp Lys Lys Ile Lys Lys Thr Phe Phe Glu Glu Leu Ala Val Glu
        35                  40                  45

Asp Lys Gln Ala Gly Glu Glu Glu Lys Val Leu Lys Glu Lys Glu Gln
    50                  55                  60

Gln Gln Gln Gln Gln Gln Gln Gln Gln Lys Lys Lys Arg Asp Thr Arg
65                  70                  75                  80

Lys Gly Arg Arg Lys Lys Asp Val Asp Asp Asp Gly Glu Glu Lys Glu
                85                  90                  95

Leu Met Glu Arg Leu Lys Lys Leu Ser Val Pro Thr Ser Asp Glu Glu
            100                 105                 110

Asp Glu Val Pro Ala Pro Lys Pro Arg Gly Gly Lys Lys Thr Lys Gly
        115                 120                 125

Gly Asn Val Phe Ala Ala Leu Ile Gln Asp Gln Ser Glu Glu Glu Glu
    130                 135                 140

Glu Glu Glu Lys His Pro Pro Lys Pro Ala Lys Pro Glu Lys Asn Arg
145                 150                 155                 160

Ile Asn Lys Ala Val Ser Glu Glu Gln Gln Pro Ala Leu Lys Gly Lys
                165                 170                 175

Lys Gly Lys Glu Glu Lys Ser Lys Gly Lys Ala Lys Pro Gln Asn Lys
            180                 185                 190

Phe Ala Ala Leu Asp Asn Glu Glu Glu Asp Lys Glu Glu Glu Ile Ile
        195                 200                 205

Lys Glu Lys Glu Pro Pro Lys Gln Gly Lys Glu Lys Ala Lys Lys Ala
    210                 215                 220

Glu Gln Gly Ser Glu Glu Glu Gly Glu Gly Glu Glu Glu Glu Glu Glu
225                 230                 235                 240

Gly Gly Glu Ser Lys Ala Asp Asp Pro Tyr Ala His Leu Ser Lys Lys

245                 250                 255

Glu Lys Lys Lys Leu Lys Lys Gln Met Glu Tyr Glu Arg Gln Val Ala
            260                 265                 270

Ser Leu Lys Ala Ala Asn Ala Ala Glu Asn Asp Phe Ser Val Ser Gln
        275                 280                 285

Ala Glu Met Ser Ser Arg Gln Ala Met Leu Glu Asn Ala Ser Asp Ile
    290                 295                 300

Lys Leu Glu Lys Phe Ser Ile Ser Ala His Gly Lys Glu Leu Phe Val
305                 310                 315                 320

Asn Ala Asp Leu Tyr Ile Val Ala Gly Arg Arg Tyr Gly Leu Val Gly
                325                 330                 335

Pro Asn Gly Lys Gly Lys Thr Thr Leu Leu Lys His Ile Ala Asn Arg
            340                 345                 350

Ala Leu Ser Ile Pro Pro Asn Ile Asp Val Leu Leu Cys Glu Gln Glu
        355                 360                 365

Val Val Ala Asp Glu Thr Pro Ala Val Gln Ala Val Leu Arg Ala Asp
    370                 375                 380

Thr Lys Arg Leu Lys Leu Leu Glu Glu Glu Arg Arg Leu Gln Gly Gln
385                 390                 395                 400

Leu Glu Gln Gly Asp Asp Thr Ala Ala Glu Arg Leu Glu Lys Val Tyr
                405                 410                 415

Glu Glu Leu Arg Ala Thr Gly Ala Ala Ala Ala Glu Ala Lys Ala Arg
            420                 425                 430

Arg Ile Leu Ala Gly Leu Gly Phe Asp Pro Glu Met Gln Asn Arg Pro
        435                 440                 445

Thr Gln Lys Phe Ser Gly Gly Trp Arg Met Arg Val Ser Leu Ala Arg
    450                 455                 460

Ala Leu Phe Met Glu Pro Thr Leu Leu Met Leu Asp Glu Pro Thr Asn
465                 470                 475                 480

His Leu Asp Leu Asn Ala Val Ile Trp Leu Asn Asn Tyr Leu Gln Gly
                485                 490                 495

Trp Arg Lys Thr Leu Leu Ile Val Ser His Asp Gln Gly Phe Leu Asp
            500                 505                 510

Asp Val Cys Thr Asp Ile Ile His Leu Asp Ala Gln Arg Leu His Tyr
        515                 520                 525

Tyr Arg Gly Asn Tyr Met Thr Phe Lys Lys Met Tyr Gln Gln Lys Gln
    530                 535                 540

Lys Glu Leu Leu Lys Gln Tyr Glu Lys Gln Glu Lys Lys Leu Lys Glu
545                 550                 555                 560

Leu Lys Ala Gly Gly Lys Ser Thr Lys Gln Ala Glu Lys Gln Thr Lys
                565                 570                 575

Glu Ala Leu Thr Arg Lys Gln Gln Lys Cys Arg Arg Lys Asn Gln Asp
            580                 585                 590

Glu Glu Ser Gln Glu Ala Pro Glu Leu Leu Lys Arg Pro Lys Glu Tyr
        595                 600                 605

Thr Val Arg Phe Thr Phe Pro Asp Pro Pro Pro Leu Ser Pro Pro Val
    610                 615                 620

Leu Gly Leu His Gly Val Thr Phe Gly Tyr Gln Gly Gln Lys Pro Leu
625                 630                 635                 640

Phe Lys Asn Leu Asp Phe Gly Ile Asp Met Asp Ser Arg Ile Cys Ile
                645                 650                 655

Val Gly Pro Asn Gly Val Gly Lys Ser Thr Leu Leu Leu Leu Leu Thr
            660                 665                 670

Gly Lys Leu Thr Pro Thr His Gly Glu Met Arg Lys Asn His Arg Leu
        675                 680                 685

Lys Ile Gly Phe Phe Asn Gln Gln Tyr Ala Glu Gln Leu Arg Met Glu
    690                 695                 700

Glu Thr Pro Thr Glu Tyr Leu Gln Arg Gly Phe Asn Leu Pro Tyr Gln
705                 710                 715                 720

Asp Ala Arg Lys Cys Leu Gly Arg Phe Gly Leu Glu Ser His Ala His
                725                 730                 735

Thr Ile Gln Ile Cys Lys Leu Ser Gly Gly Gln Lys Ala Arg Val Val
            740                 745                 750

Phe Ala Glu Leu Ala Cys Arg Glu Pro Asp Val Leu Ile Leu Asp Glu
        755                 760                 765

Pro Thr Asn Asn Leu Asp Ile Glu Ser Ile Asp Ala Leu Gly Glu Ala
    770                 775                 780

Ile Asn Glu Tyr Lys Gly Ala Val Ile Val Val Ser His Asp Ala Arg
785                 790                 795                 800

Leu Ile Thr Glu Thr Asn Cys Gln Leu Trp Val Val Glu Glu Gln Ser
                805                 810                 815

Val Ser Gln Ile Asp Gly Asp Phe Glu Asp Tyr Lys Arg Glu Val Leu
            820                 825                 830

Glu Ala Leu Gly Glu Val Met Val Ser Arg Pro Arg Glu
        835                 840                 845

<210> 42
<211> 7
<212> PRT
<213> Artificial Sequence

<220>
<223> CDR3 sequence of the VH domain of intrabody 3H2-1

<400> 42

Pro Ile Ala Val Ser Asp Tyr
1               5

<210> 43
<211> 10
<212> PRT
<213> Artificial Sequence

<220>
<223> CDR3 sequence of the VH chain of intrabody 3H2-VH

<400> 43

Gly Val Arg Gly Gly Tyr Gly Leu Asp Phe
1               5                   10

<210> 44
<211> 10
<212> PRT
<213> Artificial Sequence

<220>
<223> CDR3 sequence of the VH chain of intrabody 5H4

<400> 44

Asp Gly Gly Leu Arg Glu Gly Phe Asp Cys
1               5                   10

<210> 45
<211> 66

<212>  DNA
<213>  Artificial Sequence

<220>
<223>  antisense strand of sh1 shRNA to LOC297607

<400>  45
gcgtaagatt agagagcctt taagttctct aaaggctctc taatcttaca aaaaaagatc      60

tcttaa                                                                  66


<210>  46
<211>  66
<212>  DNA
<213>  Artificial Sequence

<220>
<223>  antisense strand of sh2 shRNA to LOC297607

<400>  46
gctcttaact aaccgctttc taagttctct agaaagcggt tagttaagaa aaaaaagatc      60

tcttaa                                                                  66